(19) Europäisches Patentamt
European Patent Office
Office européen des brevets

(11) Veröffentlichungsnummer: **0 368 175**
**A1**

(12) # EUROPÄISCHE PATENTANMELDUNG

(21) Anmeldenummer: **89120420.8**

(22) Anmeldetag: **04.11.89**

(51) Int. Cl.[5]: **C07D 495/14, A61K 31/55, //(C07D495/14,233:00,333:00, 243:00)**

(30) Priorität: **06.11.88 DE 3837693**

(43) Veröffentlichungstag der Anmeldung:
**16.05.90 Patentblatt 90/20**

(84) Benannte Vertragsstaaten:
**AT BE CH DE ES FR GB GR IT LI LU NL SE**

(71) Anmelder: **BOEHRINGER INGELHEIM KG**

**D-6507 Ingelheim am Rhein(DE)**
(84) **BE CH DE ES FR GR IT LI LU NL SE AT**

Anmelder: **BOEHRINGER INGELHEIM INTERNATIONAL G.M.B.H.**

**D-6507 Ingelheim am Rhein(DE)**
(84) **GB**

(72) Erfinder: **Weber, Karl-Heinz, Dr.**
**Kaiser-Karl-Strasse 11**
**D-6535 Gau-Algesheim(DE)**
Erfinder: **Walther, Gerhard, Dr.**
**Pfarrer-Heberer-Strasse 37**
**D-6530 Bingen(DE)**
Erfinder: **Stransky, Werner, Dr.**
**Im Hippel 24**
**D-6535 Gau-Algesheim(DE)**
Erfinder: **Birke, Franz, Dr.**
**Albrecht-Dürer-Strasse 23**
**D-6507 Ingelheim am Rhein(DE)**
Erfinder: **Muacevic, Gojko, Dr.**
**In der Dörrwiese 13**
**D-6507 Ingelheim(DE)**
Erfinder: **Heuer, Hubert, Dr.**
**Am Alten Weg 29**
**D-6500 Mainz 32(DE)**
Erfinder: **Bechtel, Wolf-Dietrich, Dr.**
**Mühlstrasse 3**
**D-6531 Appenheim(DE)**

(54) **Neue Hetrazepine.**

(57) Die Erfindung betrifft neue Hetrazepine, Verfahren zu deren Herstellung sowie ihre Verwendung als Arzneimittel mit PAF-antagonistischer Wirkung.

EP 0 368 175 A1

## Neue Hetrazepine

Die Erfindung betrifft neue Hetrazepine, deren Herstellung und ihre Verwendung als Arzneimittel insbesondere als PAF-Antagonisten.

Die neuen Hetrazepine entsprechen der allgemeinen Formel I

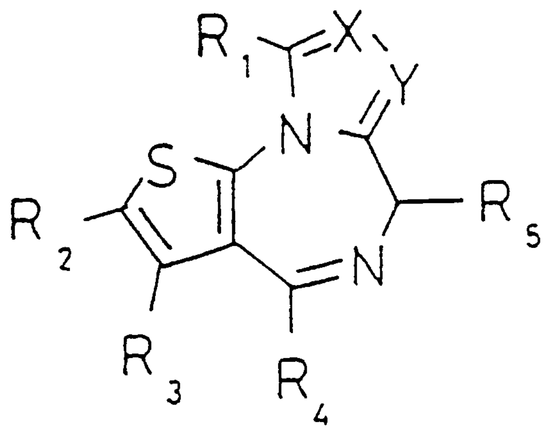

Ia

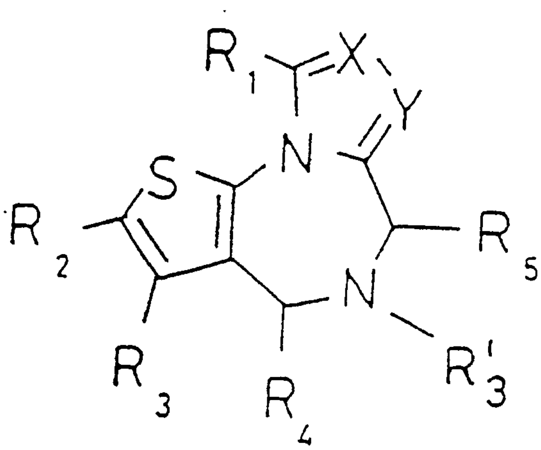

Ib

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, eine Cyclopropyl-, eine Cyclobutyl-, Cyclopentylgruppe, eine verzweigte oder unverzweigte Alkoxygruppe mit 1 bis 4 Kohlenstoffatomen, bevorzugt Methoxy, Halogen, bevorzugt Chlor oder Brom;

$R_2$ den Rest $R_a$-$Z_n$-

worin

für n > O

$R_a$ Halogen, Hydroxy, Alkenyl,

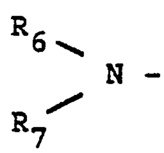

,

wobei $R_6$ und $R_7$, die gleich oder verschieden sein können, Wasserstoff, eine verzweigte oder unverzweigte Alkylgruppe mit 1 -18 Kohlenstoffatomen, bevorzugt 1 -6 Kohlenstoffatomen, eine verzweigte oder unverzweigte Alkenylgruppe mit 3 bis 18 Kohlenstoffatomen, bevorzugt 3 - 6 Kohlenstoffatomen, eine verzweigte oder unverzweigte Alkinylgruppe mit 3 bis 18 Kohlenstoffatomen, bevorzugt 3 bis 6 Kohlenstoffatomen, die gegebenenfalls durch Halogen, Hydroxy, Phenyl, substituiertes Phenyl, oder durch einen C-verknüpften Heterocyclus substituiert sein können, wobei die Kohlenstoffketten durch Stickstoff (auch NH), Sauerstoff oder Schwefel unterbrochen sein können, eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine Aminogruppe, die gegebenenfalls ein- oder zweifach durch eine verzweigte oder unverzweigte Alkylgruppe mit 1 bis 6 Kohlenstoffatomen substituiert sein kann, wobei die Alkylgruppe wiederum

durch Halogen oder Hydroxy substituiert sein kann,
eine gegebenenfalls substituierte Arylcarbonylgruppe, bevorzugt Phenylcarbonyl oder Tolylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, einen gegebenenfalls substituierten $C_3$-$C_7$-Cycloalkylrest, einen gegebenenfalls substituierten $C_5$-$C_7$-Cycloalkenylrest,
$R_6$ oder $R_7$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,
oder
$R_6$ und $R_7$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;
$R_a$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatomen substituiert;
eine verzweigte oder unverzweigte Alkylsulfonyloxygruppe mit 1 bis 4 Kohlenstoffatomen;
eine Arylcarbonyloxygruppe, bevorzugt Phenylcarbonyloxy oder Tolylcarbonyloxy -gegebenenfalls ein- oder mehrfach durch verzweigte oder unverzweigte Alkyl- und/oder Alkoxygruppen mit 1 bis 4 Kohlenstoffatomen substituiert;
$R_a$ eine verzweigte oder unverzweigte Alkylcarbonyloxygruppe mit 1 bis 12, bevorzugt 1 bis 8, Kohlenstoffatomen, wobei die Alkylkette durch Stickstoff, Sauerstoff oder Schwefel unterbrochen sein kann;

Ra 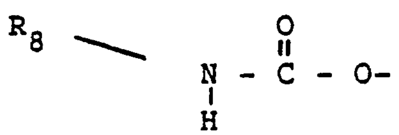 , 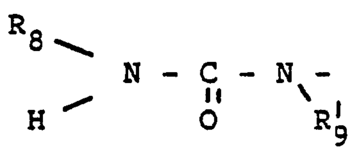

wobei $R_8$ eine verzweigte oder unverzweigte $C_1$-$C_{10}$-, bevorzugt $C_1$-$C_4$-Alkyl-, $C_3$-$C_{10}$-, bevorzugt $C_3$-$C_4$-Alkenyl- oder $C_3$-$C_{10}$-, bevorzugt $C_3$-$C_4$-Alkinylgruppe, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Arylgruppe, bevorzugt Phenyl, und $R_9'$ Wasserstoff oder eine verzweigte oder unverzweigte Alkylgruppe mit 1 bis 4 Kohlenstoffatomen;

Ra 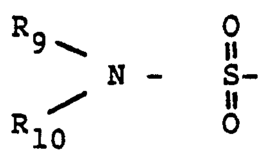

wobei $R_9$ und $R_{10}$, die gleich oder verschieden sein können, eine verzweigte oder unverzweigte Alkylgruppe mit 1 bis 18 Kohlenstoffatomen, bevorzugt 1 bis 6, einen gegebenenfalls substituierten $C_3$-$C_7$-Cycloalkylrest, einen gegebenenfalls substituierten $C_5$ - $C_7$-Cycloalkenylrest,
$R_9$ oder $R_{10}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,
oder $R_9$ und $R_{10}$ zusammen mit dem Stickstoffatom einen gegebenenfalls ein- oder mehrfach durch verzweigte oder unverzweigte Alkylgruppen mit 1 bis 4 Kohlenstoffatomen substituierten gesättigten oder ungesättigten 5-, 6- oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatomen, bevorzugt Methyl, substituiert sein kann;
$R_a$ eine verzweigte oder unverzweigte Alkoxygruppe mit 1 bis 10, bevorzugt 1 bis 4 Kohlenstoffatomen;
eine Aryloxygruppe, bevorzugt Phenyloxy oder substituiertes Phenyloxy;
$R_a$ einen C-verknüpften 5 bis 7-gliedrigen nicht aromatischen Heterocyclus;

$R_a$ einen Imidorest, einen Benzimidazoylrest, ein Imid;
für n größer gleich O
Ra -CH=0, COOH, Cyano, Wasserstoff,
einen Rest der allgemeinen Formel

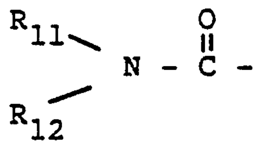

,

worin $R_{11}$ und $R_{12}$, die gleich oder verschieden sein können, Wasserstoff, Phenyl, substituiertes Phenyl, eine verzweigte oder unverzweigte Alkylgruppe mit 1 bis 18 Kohlenstoffatomen, bevorzugt 1 bis 6 Kohlenstoffatomen, eine verzweigte oder unverzweigte Alkenylgruppe mit 3 bis 18, bevorzugt 3 bis 6 Kohlenstoffatomen oder eine verzweigte oder unverzweigte Alkinylgruppe mit 3 bis 18, bevorzugt 3 bis 6 Kohlenstoffatomen, wobei die Alkyl-, Alkenyl- oder Alkinylgruppe gegebenenfalls durch Halogen, Hydroxy, Nitro, Amino, substituiertes Amino oder
im Fall von $R_{11}$ = Wasserstoff oder Alkyl und Y = C-$R_1$ oder Y Stickstoff und X C-Alkyl, $R_{12}$ durch eine Esterfunktion oder ein Säureamid der allgemeinen Formel

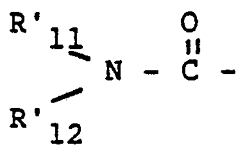

worin $R'_{11}$ und $R'_{12}$ dieselbe Bedeutung wie $R_{11}$ und $R_{12}$ - jedoch mit Ausnahme eines Säureamids - haben, substituiert sein kann,
$R_{11}$ oder $R_{12}$ einen gegebenenfalls substituierten $C_3$ bis $C_7$-Cycloalkylrest, einen gegebenenfalls substituierten $C_5$ bis $C_7$-Cycloalkenylrest,
oder
$R_{11}$ oder $R_{12}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,
$R_{11}$ und $R_{12}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann,
$R_a$ einen Rest der allgemeinen Formel

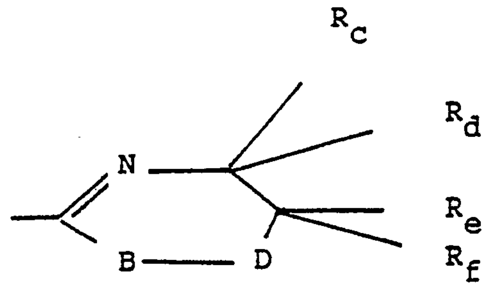

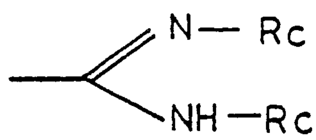

worin
B Sauerstoff, NH oder $NC_1$-$C_6$-Alkyl,
D den Rest $(CR_gR_h)_n$, wobei n 0 bis 3 sein kann,
$R_c$ Wasserstoff, gegebenenfalls durch eine Hydroxy- oder Aminogruppe substituiertes Alkyl mit 1 bis 6 Kohlenstoffatomen, $C_1$ bis $C_4$ Alkoxycarbonyl, Dialkylaminocarbonyl, $R_d$, $R_e$, $R_f$, $R_g$, $R_h$ Wasserstoff, gegebenenfalls durch eine Hydroxy- oder Aminogruppe substituiertes Alkyl mit 1 bis 6 Kohlenstoffatomen, oder Phenyl;
$R_a$ Aryl, bevorzugt Phenyl oder substituiertes Phenyl, Naphthyl, substituiertes Naphthyl;
$R_3$ Wasserstoff, Phenyl, substituiertes Phenyl oder eine verzweigte oder unverzweigte Alkylgruppe mit 1 bis 4 Kohlenstoffatomen, bevorzugt Methyl;
Z eine verzweigte oder unverzweigte Alkyl-, Alkenyl-, oder Alkinylgruppe mit n Kohlenstoffatomen, wobei Z gegebenenfalls zusätzlich durch Aryl oder zusätzlich durch $R_2$, bevorzugt Ra, substituiert sein kann;
n eine der Zahlen 0, 1, 2, 3, 4, 5, 6, 7, 8, 9 oder 10;
oder
$R_2$ und $R_3$ zuammen einen Rest der allgemeinen Formel

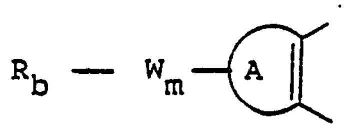

worin
A einen ankondensierten einfach ungesättigten 5-, 6-oder 7- gliedriger Ring, wobei im Fall von m = 0 und $R_b$ = Wasserstoff ein Kohlenstoffatom durch C=0 ersetzt werden kann,
oder A einen ankondensierten Ring der Formel

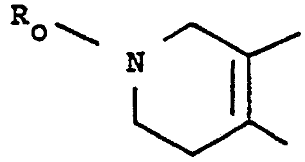

bedeutet,
wobei $R_o$ eine verzweigte oder unverzweigte Alkylgruppe mit 1 bis 18, bevorzugt mit 1 bis 4, Kohlenstoffatomen, eine Alkylcarbonyl- oder Alkylthiocarbonylgruppe mit 1 bis 18 bevorzugt 1 bis 4, Kohlenstoffatomen in der Alkylkette oder eine Arylcarbonyl- oder Arylthiocarbonylgruppe eine Alkoxycarbonylalkylgruppe mit bis zu 18, bevorzugt bis zu 8 Kohlenstoffatomen, besonders bevorzugt bis zu 4, eine Alkylcarbonylalkylgruppe mit bis zu 18, bevorzugt bis zu 8 Kohlenstoffatomen, besonders bevorzugt bis zu 4, eine Alkylcarbonylaminoalkylcarbonylgruppe mit bis zu 18, bevorzugt bis 10 Kohlenstoffatomen oder eine Aminocarbonylalkylgruppe mit bis zu 18, bevorzugt bis 4 Kohlenstoffatomen in der Alkylkette oder Wasserstoff

$R_o$ einen Rest der allgemeinen Formel

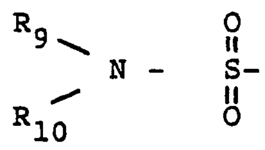

wobei $R_9$ und $R_{10}$, die gleich oder verschieden sein können, eine verzweigte oder unverzweigte Alkylgruppe mit 1 bis 18 Kohlenstoffatomen, bevorzugt 1 bis 6, einen gegebenenfalls substituierten $C_3$-$C_7$-Cycloalkylrest, einen gegebenenfalls substituierten $C_5$ - $C_7$-Cycloalkenylrest,

$R_9$ oder $R_{10}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,

oder $R_9$ und $R_{10}$ zusammen mit dem Stickstoffatom einen gegebenenfalls ein- oder mehrfach durch verzweigte oder unverzweigte Alkylgruppen mit 1 bis 4 Kohlenstoffatomen substituierten gesättigten oder ungesättigten 5-, 6- oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatomen, bevorzugt Methyl, substituiert sein kann;

W eine verzweigte oder unverzweigte Alkyl-, Alkenyl-, Alkinylgruppe mit m Kohlenstoffatomen;

m 0, 1, 2, 3, 4, 5 oder 6;

$R_b$ Wasserstoff, Hydroxy, Amino, Formyl, Carboxy, Cyano, verzweigtes oder unverzweigtes Alkyloxycarbonyl mit 1 bis 18, bevorzugt 1 bis 8, Kohlenstoffatomen, wobei die Alkylkette gegebenenfalls durch Hydroxy, Amino, Nitro oder Halogen substituiert sein kann, eine gegebenenfalls substituierte Aryloxycarbonylgruppe bevorzugt Phenyloxycarbonyl;

$R_b$ einen Rest der allgemeinen Formel

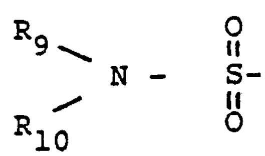

worin $R_9$ und $R_{10}$ wie zuvor definiert sind;

$R_b$ einen Rest der allgemeinen Formel

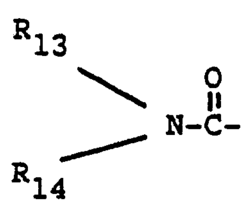

worin $R_{13}$ und $R_{14}$, die gleich oder verschieden sein können, Wasserstoff, eine verzweigte oder unverzweigte Alkylgruppe mit 1 bis 18 Kohlenstoffatomen, bevorzugt mit 1 bis 6, besonders bevorzugt 1 bis 4, unverzweigte Alkenyl- mit 3 bis 18 Kohlenstoffatomen, bevorzugt 3 - 6 Kohlenstoffatomen, eine verzweigte oder unverzweigte Alkinylgruppe mit 3 bis 18 Kohlenstoffatomen, bevorzugt 3 bis 6 Kohlenstoffatomen, wobei die Alkyl-, Alkenyl- oder Alkinylgruppe gegebenenalls durch Halogen, Hydroxy, Nitro, Amino, substituiertes Amino, $C_1$ bis $C_6$ Alkoxy, bevorzugt Methoxy oder im Fall von $R_{13}$ = Wasserstoff oder Alkyl, durch eine Esterfunktion oder durch ein Säureamid der allgemeinen Formel

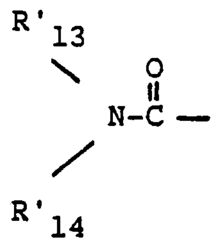

worin $R'_{13}$ und $R'_{14}$ dieselbe Bedeutung wie $R_{13}$ und $R_{14}$ jedoch mit Ausnahme eines Säureamids haben können, substituiert sein kann,
Phenyl, substituiertes Phenyl,
$R_{13}$ oder $R_{14}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring oder ein gegebenenfalls substituierter $C_3$ bis $C_7$ Cycloalkylrest, ein gegebenenfalls substituierter $C_5$ bis $C_7$ Cycloalkenylrest
oder
$R_{13}$ und $R_{14}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann;
$R_b$ einen Rest der allgemeinen Formel

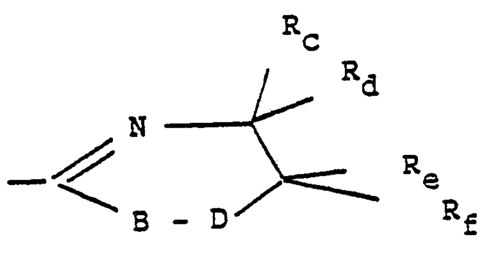

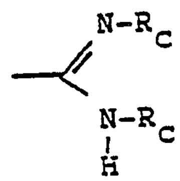

worin
B Sauerstoff, Schwefel, NH oder N-$C_1$-$C_6$-Alkyl
D den Rest $(CR_gR_h)_n$, wobei n 0 bis 3 sein kann,
$R_c$ Wasserstoff, gegebenenfalls durch eine Hydroxy- oder Aminogruppe substituiertes Alkyl mit 1 bis 6 Kohlenstoffatomen, $C_1$ bis $C_4$ Alkoxycarbonyl, $C_1$ bis $C_4$ Dialkylaminocarbonyl,
$R_d$, $R_e$, $R_f$, $R_g$, $R_h$ Wasserstoff, gegebenenfalls durch eine Hydroxy oder Aminogruppe substituiertes Alkyl mit 1 bis 6 Kohlenstoffatomen oder Phenyl;

$R_b$ 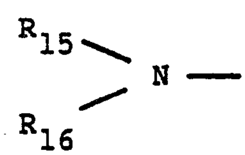 ,

$R_{15}$ = Wasserstoff, $R_{16}$ = Wasserstoff, Alkylcarbonyl oder Alkoxycarbonyl mit 1 bis 18, bevorzugt 1 bis 6 Kohlenstoffatomen, Aminocarbonyl, Alkylaminocarbonyl, Dialkylaminocarbonyl mit 1 bis 18 bevorzugt 1 bis 6 Kohlenstoffatomen in der Alkylkette;
$R_b$ Wasserstoff, Aryl, bevorzugt Phenyl, substituiertes Phenyl, Naphthyl, substituiertes Naphthyl;
$R_b$ Halogen,

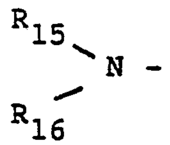 ,

wobei $R_{15}$ und $R_{16}$, die gleich oder verschieden sein können, Wasserstoff, eine verzweigte oder unverzweigte Alkylgruppe mit 1 - 18 Kohlenstoffatomen, bevorzugt 1 bis 6 Kohlenstoffatomen, eine verzweigte oder unverzweigte Alkenylgruppe mit 3 bis 18, bevorzugt 3 bis 6 Kohlenstoffatomen, eine verzweigte oder unverzweigte Alkinylgruppe mit 3 bis 18, bevorzugt 3 bis 6 Kohlenstoffatomen, wobei die Alkyl-, Alkenyl- oder Alkinylgruppe gegebenenfalls durch Halogen, Hydroxy oder einen C-verknüpften Heterocyclus substituiert sein kann, wobei die Kohlenstoffkette der Alkyl-, Alkenyl- oder Alkinylgruppe durch Stickstoff (auch NH), Sauerstoff oder Schwefel unterbrochen sein kann,
eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann, eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen,
$R_{15}$ oder $R_{16}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring, ein gegebenenfalls substituierter $C_3$ bis $C_7$-Cycloalkylrest, ein gegebenenfalls substituierter $C_5$ bis $C_7$ Cycloalkenylrest,
oder
$R_{15}$ und $R_{16}$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;
$R_b$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatomen substituiert;
$R_b$ eine verzweigte oder unverzweigte Alkylsulfonyloxygruppe mit 1 bis 4 Kohlenstoffatomen;
$R_b$ eine Arylcarbonyloxygruppe, bevorzugt Tolylcarbonyloxy oder Phenylcarbonyloxy, gegebenenfalls ein- oder mehrfach durch verzweigte oder unverzweigte Alkyl- und/oder Alkoxygruppen mit 1 bis 4 Kohlenstoffatomen substituiert;
$R_b$ eine verzweigte oder unverzweigte Alkylcarbonyloxygruppe mit 1 bis 18, bevorzugt 1 bis 8, Kohlenstoffatomen, wobei die Alkylkette durch Stickstoff, Sauerstoff oder Schwefel unterbrochen sein kann;

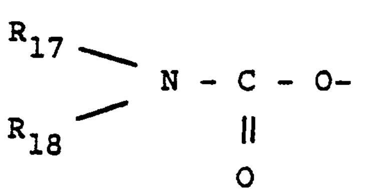 , 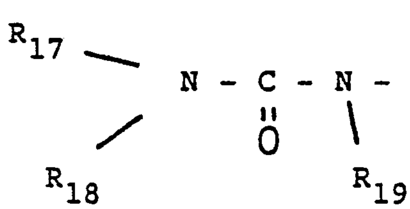

wobei $R_{17}$ Wasserstoff und $R_{18}$ eine Alkyl-, Alkenyl- oder Alkinylgruppe mit 1 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Arylgruppe,
$R_{19}$ Wasserstoff oder eine verzweigte oder unverzweigte Alkylgruppe mit 1 bis 4 Kohlenstoffatomen;
$R_b$ einen Imidorest; einen Benzimidazolylrest, ein Imid;
$R_b$ einen verzweigten oder unverzweigten Alkyloxy-bzw. Alkylthiorest mit 1 bis 18, bevorzugt 1 bis 4 Kohlenstoffatomen, Aryloxy, bevorzugt einen gegebenenfalls substituierten Phenyloxy- oder Phenylthiorest, einen über Sauerstoff oder Schwefel verknüpften, gesättigten oder ungestättigten 5- oder 6-gliedrigen heterocyclischen Ring;

$R_3'$ Wasserstoff, $C_1$ bis $C_4$-Alkyl, $C_1$ bis $C_4$-Acyl;

$R_4$ Phenyl, wobei der Phenylring ein oder mehrfach, bevorzugt in 2-Stellung, durch Methyl, bevorzugt Halogen, bevorzugt Brom, besonders bevorzugt Chlor, Nitro und/oder Trifluormethyl substituiert sein kann, oder Pyridyl;

$R_5$ Hydroxy, verzweigtes oder unverzweigtes Alkyl mit 1 bis 6, bevorzugt 1 bis 4 Kohlenstoffatomen, besonders bevorzugt Methyl, gegebenenfalls ist die Alkylgruppe durch Hydroxy, Halogen, bevorzugt Fluor, Alkylsulfonyloxy, bevorzugt Methylsulfonyloxy,

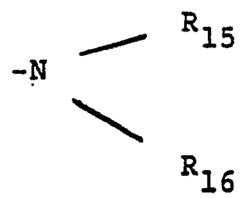

worin $R_{15}$ und $R_{16}$ die zuvor genannte Bedeutung aufweisen können, oder

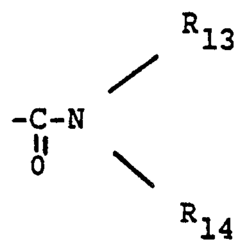

worin $R_{13}$ und $R_{14}$ die zuvor genannte Bedeutng aufweisen können, substituiert;

$R_5$ Alkylcarbonyloxy mit 1 bis 4 Kohlenstoffatomen in den verzweigten oder unverzweigten Alkylkette, Alkoxycarbonyl mit 1 bis 4 Kohlenstoffatomen in der verzweigten oder unverzweigten Alkylkette, Carboxyalkyl mit 1 bis 4 Kohlenstoffatomen in der verzweigten oder unverzweigten Alkylkette, Alkoxycarbonylalkyl mit 1 bis 4 Kohlenstoffatomen in den verzweigten oder unverzweigten Alkylresten, X/Y unabhängig voneinander C-$R_1$, oder N, aber nicht beide C-$R_1$, mit $R_1$ bevorzugt Wasserstoff oder Methyl, oder Y die Gruppe C-COOR′, wobei R′ Alkyl oder Wasserstoff bedeutet und X Stickstoff bedeutet;

gegebenenfalls in Form ihrer Racemate, ihrer Enantiomere, ihrer Diastereomere und ihrer Gemische sowie gegebenenfalls ihre physiologisch unbedenklichen Säureadditionssalze.

Bevorzugte Verbindungen der allgemeinen Formel Ia, in denen $R_2$, $R_aZ_n$, $R_3$ Wasserstoff, Z eine unverzweigte Alkylgruppe,

$R_1$ Ethyl, Methoxy, Ethoxy oder Halogen, besonders bevorzugt Chlor oder Brom; besonders bevorzugt Methyl;

$R_a$ Chlor, Brom, Jod, Hydroxy, Phenyl, P-Isobutylphenyl,

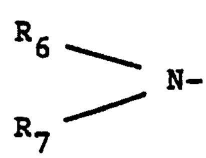

worin $R_6$ und $R_7$, die gleich oder verschieden sein können, Wasserstoff, eine verzweigte oder unverzweigte Alkylgruppe mit 1 bis 6, besonders bevorzugt 1 bis 4, Kohlenstoffatomen, wobei die Kohlenstoffkette durch Stickstoff unterbrochen sein kann, eine verzweigte oder unverzweigte Alkylcarbonylgruppe mit 1 bis 4 Kohlenstoffatomen, gegebenenfalls substituiert durch eine Dimethylaminogruppe, eine Phenylcarbonylgruppe, im Fall von $R_6$ = Wasserstoff eine gegebenenfalls durch Acylamino, besonders bevorzugt Acetylamino, Amino, Alkylamino- oder Dialkylamino substituierte Phenylsulfonylgruppe,

oder

$R_6$ und $R_7$ bilden zusammen mit dem Stickstoffatom einen Piperidin-, Pyrrolidin-, N′-Methylpiperazin-, einen gegebenenfalls dimethylsubstituierten Morpholinring, einen Pyrrol-, Pyrazol-, Imidazol-oder Triazolring,

$R_a$ -CH=O,

$R_a$ ein gegebenenfalls durch Methyl ein- oder mehrfach substituiertes $\Delta^2$-Imidazolin, -Oxazolin, -Thiazolin, eine Tolylsulfonyloxygruppe, eine Methylsulfonyloxygruppe,

$R_a$ eine Phenylcarbonyloxygruppe, eine Alkylcarbonyloxygruppe mit 1 bis 5 Kohlenstoffatomen

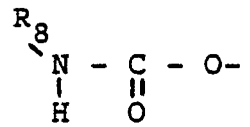
,
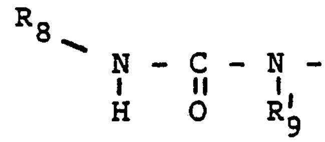

wobei $R_8$ eine verzweigte oder unverzweigte Alkylgruppe mit 1 bis 4 Kohlenstoffatomen,
$R_9'$ Wasserstoff oder eine verzweigte oder unverzweigte Alkylgruppe mit 1 bis 4 Kohlenstoffatomen;

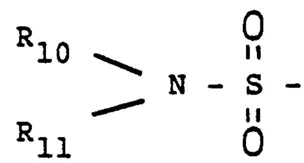

wobei $R_{10}$ und $R_{11}$ die gleich oder verschieden sein können, eine Methyl-, Ethyl-, Propyl- oder Isopropylgruppe oder $R_{10}$ und $R_{11}$ zusammen mit dem Stickstoffatom einen N′-Methylpiperazin- oder Morpholinring;

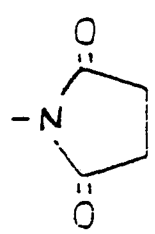
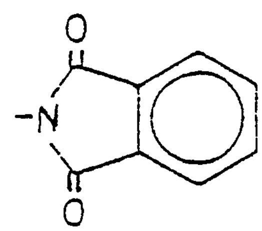
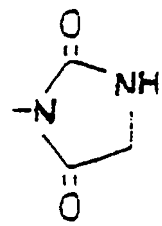

$R_a$
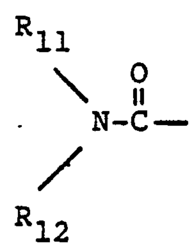

worin $R_{11}$ und $R_{12}$, die gleich oder verschieden sein können, Wasserstoff, eine verzweigte oder unverzweigte Alkylgruppe oder Alkenylgruppe mit 1 bis 6, 8 oder 16 Kohlenstoffatomen, die gegebenenfalls durch Halogen, Hydroxy, Methoxy, Nitro, Amino, Alkylamino oder Dialkylamino mit 1 bis 4 Kohlenstoffatomen in der Alkylkette, oder im Fall von $R_{12}$ = Wasserstoff oder Alkyl durch Morpholinylcarbonyl oder Diethylaminocarbonyl substituiert sein kann,
im Fall von $R_{11}$ = Wasserstoff oder Methyl, $R_{12}$ ein Thiazolin- oder Thiazolrest, der gegebenenfalls durch eine verzweigte oder unverzweigte Alkylgruppe mit 1 bis 4 C-Atomen substituiert sein kann,
oder
$R_{11}$ und $R_{12}$ zusammen mit dem Stickstoffatomen einen Morpholino- oder Piperazinorest bilden, der gegebenenfalls ein- oder mehrfach durch Methyl substituiert sein kann;

$R_4$ Phenyl, wobei der Phenylring, bevorzugt in 2-Stellung durch Halogen, bevorzugt Chlor, substituiert sein kann;

$R_5$ Hydroxy, Methyl oder Hydroxymethyl

X,Y unabhängig voneinander C-$R_1$ oder N, aber nicht gleichzeitig beide C-$R_1$, ($R_1$ auch Wasserstoff),

oder Y die Gruppe C-COOR*, mit R* = Alkyl oder Wasserstoff und X = Stickstoff;

n eine der Zahlen 1, 2, 3, 4, 5, 6, 7, 8, 9 oder 10 bedeuten können sowie gegebenenfalls ihre physiologisch unbedenklichen Säureadditionssalze und ihre optisch aktiven Verbindungen.

Bevorzugte Verbindungen der allgemeinen Formel Ia sind solche, in denen $R_2$ und $R_3$ einen Rest der allgemeinen Formel

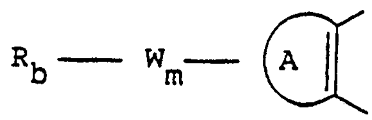

bilden, sind solche worin A ein ankondensierter einfach ungesättigter 5- oder 6-gliedriger Ring bedeutet, wobei im Fall von m = o und $R_2$ = Wasserstoff in einem 6-gliedrigen Ring ein Kohlenstoffatom durch CO in 2-, 3-oder 4-Stellung des Hetrazepins, ersetzt werden kann, oder A einen ankondensierten Ring der Formel

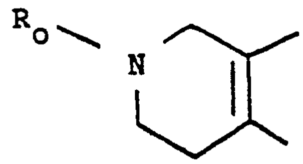

worin $R_0$ Acetylaminoacetyl, Acetyl, Thioacetyl, Ethoxycarbonylmethyl, Methoxycarbonylmethyl, Morpholinylcarbonylmethyl, Diethylaminocarbonylmethyl;

W eine unverzweigte Alkylgruppe mit m Kohlenstoffatomen

m 0, 1, 2, 3 oder 4;

X/Y unabhängig voneinander C-$R_1$ oder N, bevorzugt beide N oder X C-$R_1$ und Y N, aber nicht beide C-$R_1$,

oder

Y C-COOR′, wobei R′ Wasserstoff oder Niederalkyl und X Stickstoff bedeutet;

$R_1$ Wasserstoff, Hydroxymethyl, Chlormethyl, Cyclopropyl, Ethyl, Methoxy, Ethoxy, Chlor oder Brom, bevorzugt Methyl;

$R_b$ Hydroxy, Amino, Carboxy, Cyano, Brom, Phenyl, p-Isobutylphenyl, Alkyloxycarbonyl mit 1 bis 6 Kohlenstoffatomen, bevorzugt 1 bis 2 Kohlenstoffatomen,

einen Rest der allgemeinen Formel

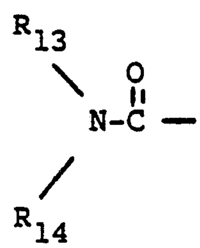

worin $R_{13}$ und $R_{14}$, die gleich oder verschieden sein können, Wasserstoff, eine verzweigte oder unverzweigte Alkylgruppe oder Alkenylgruppe mit 1 bis 6, 8 oder 16 Kohlenstoffatomen, die gegebenenfalls durch Halogen, Hydroxy, Methoxy, Nitro, Amino, Alkylamino oder Dialkylamino mit 1 bis 4 Kohlenstoffatomen in der Alkylkette, oder im Fall von $R_{14}$ = Wasserstoff oder Alkyl durch Morpholinylcarbonyl oder Diethylaminocarbonyl substituiert sein kann,

im Fall von $R_{13}$ = Wasserstoff oder Methyl,

$R_{14}$ ein Thiazolin- oder Thiazolrest, der gegebenenfalls durch eine verzweigte oder unverzweigte Alkylgruppe mit 1 bis 4 C-Atomen substituiert sein kann,

oder

$R_{13}$ und $R_{14}$ zusammen mit dem Stickstoffatomen einen Morpholino- oder Piperazinorest bilden, der gegebenenfalls ein- oder mehrfach durch Methyl substituiert sein kann;

$R_b$ ein C-verknüpfter $\Delta^2$-Imidazolin-, -Thiazolin-, -Oxazolin-, oder Tetrahydropyrimidin-Rest, der gegebenenfalls ein- oder mehrfach durch Alkyl mit 1 bis 4 Kohlenstoffatomen substituiert sein kann;
$R_b$ im Fall von m = O Wasserstoff, wenn A eine Carbonylfunktion oder Stickstoff als Ringglied enthält;

$R_b$ 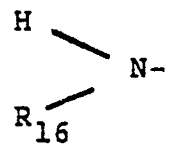

$R_{16}$ eine Alkyloxycarbonylgruppe mit 1 bis 4 Kohlenstoffatomen;
im Fall von m > O
$R_b$ eine Alkylcarbonyloxygruppe mit 1 bis 3 Kohlenstoffatomen; $R_b$ eine Alkylsulfonyloxygruppe mit 1 bis 2 Kohlenstoffatomen;

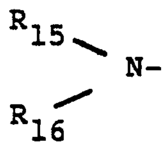

wobei $R_{15}$ und $R_{16}$, die gleich oder verschieden sein können, Wasserstoff, eine verzweigte oder unverzweigte Alkylgruppe mit 1 bis 6 Kohlenstoffatomen, gegebenenfalls durch Dialkylamino mit 1 bis 4 Kohlenstoffatomen, bevorzugt Methyl oder Ethyl, Morpholino oder N-Alkylpiperazino oder ein Indolrest substituiert, eine Alkylcarbonylgruppe mit 1 bis 4 Kohlenstoffatomen,
oder
$R_{15}$ und $R_{16}$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Pyrazolorest, Pyrrolorest, ein Imidorest,
$R_b$ eine verzweigte oder unverzweigte Alkylsulfonyloxygruppe mit 1 bis 4 Kohlenstoffatomen, bevorzugt Methylsulfonyloxy, eine verzweigte oder unverzweigte Alkylcarbonyloxygruppe mit 1 bis 8 Kohlenstoffatomen, bevorzugt 1 bis 4 Kohlenstoffatomen;
$R_b$ Phenyloxy, 3,4-Methylendioxyphenoxy einen Pyridinyloxyrest, einen Alkyloxy- oder Alkylthiorest mit 1 bis 4 Kohlenstoffatomen;
$R_4$ Phenyl oder o-Chlorphenyl;
$R_5$ Hydroxy, Methyl, Hydroxymethyl oder Trifluormethyl bedeuten können, sowie gegebenenfalls ihre physiologisch unbedenklichen Säureadditionssalze und ihre optisch aktiven Verbindungen.

Ganz besonders bevorzugt sind Verbindungen der allgemeinen Formel Ia, worin A ein ankondensierter, bevorzugt in 3-oder 4-Stellung des Hetrazepins substituierter, einfach ungesättigter 5- oder 6-gliedriger Ring,
W eine unverzweigte Alkylgruppe mit m Kohlenstoffatomen m 0, 1 oder 2;
X/Y beide N, oder X C-H oder $C-CH_3$ und Y N,
$R_1$ Wasserstoff, Cyclopropyl, Methoxy, Brom, bevorzugt Methyl;
$R_b$ Hydroxy, Amino, Carboxy, Cyano, Methoxycarbonyl, Ethoxycarbonyl, Isobutylphenyl,
einen Rest der allgemeinen Formel

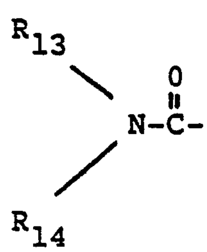

worin $R_{13}$ und $R_{14}$, die gleich oder verschieden sein können, Wasserstoff, eine verzweigte oder unverzweigte Alkylgruppe mit 1 bis 6, 8 oder 16 Kohlenstoffatomen, die gegebenenfalls durch Halogen, Hydroxy, Nitro, Amino, Ethylamino oder Diethylamino, Methoxy, oder im Fall von $R_{14}$ = Wasserstoff oder Alkyl durch Morpholinylcarbonyl oder Diethylaminocarbonyl substituiert sein kann,

Propenyl, Phenyl,

im Fall von $R_{13}$ = Wasserstoff oder Methyl,

$R_{14}$ ein Thiazolin- oder Thiazolrest, der gegebenenfalls durch Methyl substituiert sein kann,

oder

$R_{13}$ und $R_{14}$ zusammen mit dem Stickstoffatom einen Morpholino- oder Piperazinorest bilden, der gegebenenfalls ein- oder mehrfach durch Methyl substituiert sein kann;

$R_b$ ein C-verknüpfter $\Delta^2$-Imidazolin-, -Thiazolin-Oxazolin-Rest, der gegebenenfalls ein- oder mehrfach durch Methyl, Ethyl und/oder iso-Propyl substituiert sein kann, ein Tetrahydro-Pyrimidinring, gegebenenfalls ein- oder mehrfach durch Methyl substituiert, ein Benzimidazolrest, ein Indolrest,

$R_2$ im Fall von m = 0 Wasserstoff, wenn A eine Carbonylfunktion oder Stickstoff als Ringglied enthält, oder Methoxycarbonylamino;

im Fall von m > 0

$R_b$ eine Acetoxygruppe, eine Methansulfonyloxygruppe,

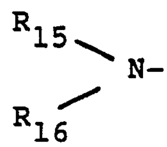

wobei $R_{15}$ und $R_{16}$, die gleich oder verschieden sein können, Wasserstoff, eine verzweigte oder unverzweigte Alkylgruppe mit 1 bis 4 Kohlenstoffatomen, die durch Diethylamino oder Morpholino, substituiert sein kann, eine Acetylgruppe oder

$R_{15}$ und $R_{16}$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Phthalimid,

$R_b$ eine verzweigte oder unverzweigte Alkylsulfonyloxygruppe mit 1 bis 4 Kohlenstoffatomen, bevorzugt Methylsulfonyloxy, eine verzweigte oder unverzweigte Alkylcarbonyloxygruppe mit 1 bis 8 Kohlenstoffatomen, bevorzugt 1 bis 4 Kohlenstoffatomen;

$R_b$ ein Phenyloxyrest, ein Pyridyloxyrest, 3,4-Methylendioxyphenoxy, eine 1,2,4-Triazol-3-yl-thiogruppe, Methoxy;

$R_4$ Phenyl, bevorzugt o-Chlorphenyl;

$R_5$ Hydroxy, Hydroxymethyl oder Methyl bedeuten können, sowie gegebenenfalls ihre physiologisch unbedenklichen Säureadditionssalze, gegebenenfalls ihre optisch aktiven Verbindungen.

Bevorzugte Imide sind:

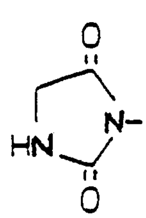

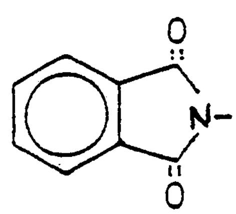

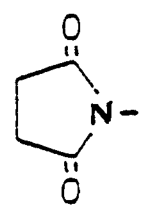

Als Alkylgruppen (auch soweit sie Bestandteile anderer Reste sind) werden bevorzugt, soweit nichts anderes angegeben ist, Methyl, Ethyl, Propyl, iso-Propyl, n-Propyl, n-Butyl, iso-Butyl, tert.-Butyl, n-Pentyl, iso-Pentyl, sec.-Pentyl, tert. Pentyl, n-Hexyl, iso-Hexyl, sec. Hexyl und tert. Hexyl. Von den langkettigen Alkylresten sind n-Hexadecanyl und n-Octadecanyl bevorzugt. Alkylgruppen können beispielsweise durch Amino, Halogen oder Hydroxy substituiert sein.

Die Angabe der Zahl der Kohlenstoffatome bezieht sich, sofern nichts anderes angegeben, auf die Länge der Alkyl-, Alkenyl- oder Alkinylkette.

Besonders bevorzugt sind anellierte 6-gliedrige Ringe A, worin die Seitenkette $W_m$-$R_b$ in der 3- oder 4-Position des Hetrazepins oder anellierte 5-gliedrige Ringe A, worin die Seitenkette $W_m$-$R_b$ in der 3-Position des Hetrazepins substituiert ist.

Als Arylreste - auch soweit sie Bestandteil anderer Reste sind, werden gegebenenfalls substituierte aromatische Ringsysteme mit 6 bis 12 Kohlenstofftomen verstanden, wie z.B. Naphthyl, Phenyl.

Als substituiertes Phenyl werden - soweit nicht anders bezeichnet - Phenylreste verstanden, die ein-, zwei- oder dreifach durch Halogen, Hydroxy und/oder verzweigtes oder unverzweigtes Alkyl mit 1 bis 5 Kohlenstoffatomen, Amino, mono- oder disubstituiertes $C_1$ - $C_4$-Alkylamino substituiert sind.

Als Beispiele werden genannt:

2-Chlorphenyl, 2-Bromphenyl, 3-Fluorphenyl, 2,3-Dichlorphenyl, 4-Hydroxyphenyl, 2-Methylphenyl, 4-Methylphenyl, 3-Ethylphenyl, 4-Propylphenyl, 4-Isopropylphenyl, 4-Butylphenyl, 4-tert.-Butylphenyl, 4-Pentylphenyl, 2,4-Dimethylphenyl, 2-Trifluoromethylphenyl, 3-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl,

Als Heterocyclus im Rahmen der oben angegebenen Definition steht unter anderem für einen 5- bis 6-gliedrigen Ring, der als Heteroatome Sauerstoff, Schwefel und/oder Stickstoff enthalten kann und an den ein weiterer aromatischer Ring bevorzugt ein Phenylring ankondensiert sein kann.

Als Beispiele für gesättigte heterocyclische 6-gliedrige Ringe werden beispielsweise genannt: 1,4 und 1,3-Dioxan, Morpholin, Thiomorpholin, Piperidin, Piperazin, 4- $C_1$ bis $C_4$-Alkylpiperazin, N-Hydroxy -$C_1$ bis $C_4$ - Alkylpiperazin, 2,5-Diketopiperazin. Als Beispiele für gesättigte heterocyclische 5-gliedrige Ringe werden beispielsweise genannt: Tetrahydropyrrol, Tetrahydrofuran, Prolin, Imidazolidin, Hydroxyprolin, Pyrrolidon, Thiolan, Butyrolacton, 1,2-Oxathiolan.

Als Beispiel für 5-, 6- und 7-gliedrige ein- und mehrfach ungesättigte heterocyclische Ringe werden beispielsweise genannt:

Pyrrol, Imidazol, Imidazolin, 1,2,4- und 1,2,3-Triazol, Tetrazol, Isothiazol, Furan, Dihydrofuran, Thiophen, Pyridin, Pyrimidin, Pyran, 2,5-Dihydropyrrol, Thiazol, Thiadiazin, Azepin, 1,2-Oxathiepan, Dimethylpyrrol, 2-Acylfuran, Dihydrothiophen. Als weitere Heteroarylreste seien beispielsweise Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Als über ein Stickstoffatom gebundener 5, 6 oder 7-gliedriger heterocyclischer Ring werden außerdem beispielhaft genannt:

Phenantridin-6-on, Chinolin-2-on, Isochinolin-1,3-dion, Benz[c,d]indol, 1,4-Benzoxazin-3-on, Indol-2,3-dion, Indol-2-on, 1,2,4-Triazolo[4,3-a]pyridin-3-on, 1,2-Benzisothiazol-3-on, 1H-Indazol, 1H-Benzimidazol, Isoindol-1,3-dion, 1H-Benztriazol, Benzothiazin-3-on, Isoindole-1,3-dion, Benz[d,e]isochinolin-1,3-dion, 4-Chinazolinon, Isoindol-1-on, 1,2,4-Triazolo[4,3-a]pyridin-3-on, Pyrrolo[1,2-c]imidazol-1,3-dion, 1,3-Dihydro-2H-indol-2-on, Tetrahydro-1H-isoindol-1,3-dion, 3,7-Dihydro-1H-Purin-2,6-dion, Indol, Indazol, Benzimidazol, Benzimidazol-2-on, 1,4-Benzothiazin-3-on-, 1H-Isoindol-1,3-dion.

Der Heterocyclus kann ein- oder mehrfach durch Halogen, Hydroxy, verzweigtes oder unverzweigtes $C_1$ bis $C_4$ Alkyl, $C_1$ bis $C_4$ Hydroxyalkyl und/oder $C_1$ bis $C_4$ Alkoxy substituiert sein.

Die erfindungsgemäßen Verbindungen der allgemeinen Formel Ia in denen $R_2$ den Rest Ra-Zn-bedeutet weisen, an C-7 ($R_5$ ≠ Wasserstoff) ein Asymmetriezentrum auf. Die bei der Synthese im allgemeinen erhaltenen Racemate können gegebenenfalls in ihre Enantiomere aufgetrennt werden; in den Beispielen ist exemplarisch eine präparative Methode zur Trennung der Enantiomeren beschrieben.

Überraschenderweise wurde gefunden, daß die (-)-Enantiomeren der allgemeinen Formel Ia - insbesondere wenn $R_5$ = Methyl bedeutet - gegenüber den (+)-Enantiomeren eine wesentlich stärkere pharmakologische Wirksamkeit aufweisen. Die (-)-Enantiomeren der allgemeinen Formel Ia mit $R_2$ = $R_a$-$Z_n$- gelten daher als bevorzugte Verbindungen und werden als solche beansprucht. Diese Aussage bezieht sich auf solche Enantiomere deren $[\alpha]^{D}_{20}$ Wert [c = 1, Methanol] einen negativen Wert annimmt.

Die erfindungsgemäßen Verbindungen der allgemeinen Formel I worin $R_2$ und $R_3$ eine anellierten Ring bilden weisen zwei Asymmetriezentren auf, eins am durch $R_5$ substituierten Kohlenstoffatom des Diazepinringes und ein zweites im anellierten Ring.

Die bei der Synthese im allgemeinen erhaltenen Diastereomeren - Gemische können durch an sich bekannte Verfahren, wie z. B. Kristallisation, chromotographische Trennverfahren, in ihre Diastereomere getrennt werden. Diese lassen sich wiederum durch präparative Verfahren -z.B. Säulenchromatographie mit optisch aktiven Trägermaterial - in ihre reinen, optisch aktiven Stereoisomere auftrennen.

Verbindungen der allgemeinen Formel I, worin $R_2$ und $R_3$ einen Rest der allgemeinen Formel I

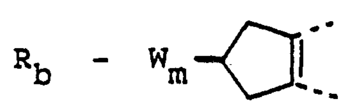

bilden,weisen demnach ebenfalls zwei Asymmetriezentren auf, eins im carbocyclischen 5-Ring und ein zweites am durch $R_5$ substituierten Kohlenstoffatom des Diazepinringes. Sofern der Substituent $R_b$-$W_m$ kein weiteres Asymmetriezentrum enthält, bilden Verbindungen der oben genannten Struktur ein Diastereomerengemisch, das aus 4 optisch reinen Isomeren besteht. Das Diastereomerengemisch läßt sich in die Diastereomere auftrennen; diese können wiederum in die reinen Enantiomere aufgetrennt werden. Präparative Methoden zur Trennung sind exemplarisch in Beispiel 9d beschrieben. Überraschenderweise wurde gefunden, daß die optisch reinen Enantiomere - erhalten aus den aufgetrennten Diastereoisomeren - deren $[\alpha]_D^{20}$ Wert - gemessen in Methanol - einen negativen Wert annimmt eine wesentlich stärkere pharmakologische Wirkung aufweisen als die entsprechende positiv drehenden Verbindungen. Diese Verbindungen werden erfindungsgemäß als (-)-Enantiomere bezeichnet und als solche beansprucht.

Die erfindungsgemäßen Verbindungen der allgemeinen Formel I können -in Abhängigkeit der Art des Restes $R_5$- nach verschiedenen Verfahren synthetisiert werden.

In der Regel erfolgt die Synthese der erfindungsgemäßen Verbindungen ausgehend von Verbindungen der allgemeinen Formel I worin $R_5$ die Bedeutung von Wasserstoff aufweist. Die Herstellung dieser Hetrazepine ($R_5$ = H) ist unter anderem in den Europäischen Patentanmeldungen 194 416, 230 942 und 254 245 beschrieben, auf die diesbezüglich inhaltlich Bezug genommen wird.

Die Einführung des Substituenten $R_5$ erfolgt nach an sich bekannten Verfahren, wie sie in der Diazepinchemie üblich sind.

Verbindungen der allgemeinen Formel I, in denen $R_5$ eine Hydroxy oder eine Acyloxygruppe bedeuten, können in Anlehnung an in der Diazepinchemie übliche Verfahren aus dem N-Oxid von Verbindungen der allgemeinen Formel I mit $R_5$ = Wasserstoff erhalten werden. Die entsprechenden N-Oxide sind durch Umsetzungen mit Persäuren zugänglich. Das so erhaltene N-Oxid wird der sogenannten Polonowsky Reaktion unterworfen und ergibt die gewünschte Acyloxygruppe. Verbindungen mit $R_5$ = Hydroxy oder Hydroxyalkyl erhält man ausgehend von der entsprechenden Acetoxyverbindung bzw. Acyloxyalkylverbindung durch Abspaltung der Acetylgruppe bzw. Acylgruppe (vergl. K.H. Weber et al., Arzneimittelforschung 36, 518 (1986)).

Verbindungen der allgemeinen Formel I, worin $R_5$ eine Alkyloxycarbonylalkylgruppe bedeutet erhält man durch Umsetzung von Verbindungen der allgemeinen Formel I mit $R_5$ = Wasserstoff mit einem Deprotonierungsmittel, wie z.B. Natriumhydrid oder einem Natriumamid in einem inerten Lösungsmittel und anschließender Zugabe eines Alkenylcarbonsäurealkyleester mit $\gamma$-ständiger Doppelbindung - wie z.B. eines Acrylsäureesters. Die so erhaltenen Verbindungen können nach an sich bekannten Verfahren weiter derivatisiert werden.

Zu dieser Derivatisierung gehören Amidbildung über die entsprechende Carbonsäure; Reduktion des Esters zum Alkohol; Überführung dieses Alkohols in entsprechende Amine.

Ebenfalls ausgehend von Verbindungen der allgemeinen Formel I, $R_5$ = H erhält man mit Dialkylcarbonaten in Gegenwart von NaH die Carbonsäureester.

Die freien Carbonsäuren hiervon sind jedoch instabil, so daß sie sich weder isolieren noch zu Carbonsäurederivaten umsetzen lassen. Der Ester kann jedoch zum Alkohol reduziert werden.

a) Die Herstellung von Verbindungen der allgemeinen Formel I worin $R_5$ einen Alkylgruppe bedeutet, erfolgt nach folgendem Synthesekonzept:

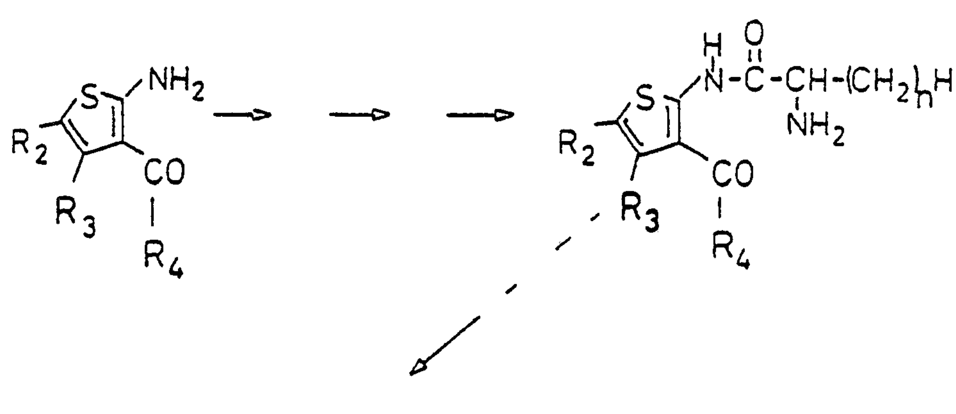

I a

n=1-6

Die Herstellung der entsprechenden 2-Aminothiophene ist aus der Literatur, insbesondere aus den oben zitierten Europäischen Patentanmeldungen, bekannt. Zur Synthese der erfindungsgemäßen Verbindung erfolgt die Umsetzung der 2-Amino-thiophene anstelle eines Halogenacetyhalogenids mit den entsprechenden homologen α-Halogenacylhalogeniden - wie z.B. Chlorpropionylchlorid - wobei bei dem zunächst erhaltenen 2-Acylamino-thiophen das verbleibende Halogenatom gegen Jod ausgetauscht wird bevor mit Ammoniak die Ringschlußreaktion zu dem Thienodiazepinon erfolgen kann. Der weitere Aufbau des erfindungsgemäßen Hetrazepinsystems erfolgt nach an sich bekannten Verfahren, wie sie in den oben zitierten Patentanmeldungen offenbart sind.

Die erfindungsgemäßen Verbindungen besitzen PAF-antagonistische Wirkung.
Bekanntlich handelt es sich bei PAF (Plättchen Aktivierender Faktor) um das Phospholipid Acetyl-glycerylether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.
Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), der Gelenke (rheumatische Erkrankungen), anaphylaktische Zustände, Allergien und Entzündungen im Bereich der Schleimhäute und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni.

Weiterhin eignen sich die erfindungsgemäßen Verbindungen zur Behandlung bei den folgenden Indikationsstellungen:
Obstruktive Lungenerkrankungen, wie z.B. bronchiale Hyperreaktivität, entzündliche Lungenwegserkrankungen, wie z.B. chronische Bronchitis;
Herz- Kreislauferkrankungen, wie z.B. Polytrauma, Anaphylaxe, Arteriosklerose, entzündliche Darmerkrankungen, EPH-Gestose (ederma-proteinuria Hypertension), Erkrankungen des extrakorporalen Kreislauf, ischämische Erkrankungen, entzündliche und immunologische Erkrankungen, Immunmodulation bei Transplantationen von Fremdgeweben, Immunmodulation bei Leukämie.
Metastasenausbreitung z.B. bei bronchialer Neoplasie, Erkrankungen des ZNS, wie z.B. Migräne, Agarophobie (panic disorder), weiterhin erweisen sich die erfindungsgemäßen Verbindungen als Cyto- und Organoprotektiv, z.B. zur Neuroprotektion, z.B. bei Leberzirrhose, DIC (disiminierte intravasale Gerinnung);

Nebenwirkungen einer Arzneimitteltherapie, z.B. anaphylaktoide Kreislaufreaktionen, Kontrastmittelzwischenfälle, Nebenwirkungen bei der Tumortherapie;
Unverträglichkeiten bei Bluttransfusionen; fulminantes Leberversagen ($CCl_4$-Intoxikation) Amanitaphalloides-Intoxikation (Knollenblätterpilzvergiftung);
Symptome von parasitären Erkrankungen (z.B. Wurmerkrankungen); Immunfunktion bei Aids, Kaposi-Syndrom, Diabetes, juvenile Diabetes, diabetische Retinopathie, polytraumatischer Schock, hämorrhagischer Schock, CNS: Ischämie, Multiple Sklerose.
PAF assoziierte Interaktion mit Gewebshormon (autocoid hormones), Lymphokine und anderen Mediatoren;
Autoimmunerkrankungen, so z.B. bei autoimmunhaemolytischen Anaemien, autoimmunologisch bedingte Glomerulonephritiden, Thyreoidis Hashimoto, primäres Myxoedem, periziöse Anaemie, autoimmune atrophische Gastritis, Morbus Addison, iuveniler Diabetes, Goodpasture-Syndrom, idiopathische Leukopenie, primär biliäre Zirrhose, aktive bzw. chronisch aggressive Hepatitis (HBsAg-neg.), Colitis ulcerosa, chronische Polyathritis und systemischer Lupus erythematodes (SLE).

PAF-Antagonisten eignen sich auch zur Behandlung des durch verminderte ß-Rezeptorstimulation am Herzen bzw. durch Down Regulation der ß-Rezeptoren am Herzen verursachten Krankheiten, z.B. zur Behandlung der zu geringen Pumpleistung des Herzens bei akutem Herversagen, beispielsweise nach Myocardinfarkt und cardiogenem Schock, oder bei chronischen cardivasculären Erkrankungen wie kongestive Herzinsuffizienz, Herzinsuffizienz nach Myocardinfarkt oder bei ischämischen Cardiomyopathien.

Sie können auch in Kombination eingesetzt werden, vor allem bei solchen Indikationen, für die PAF-Antagonisten geeignet sind. Dementsprechend können die PAF-Antagonisten z.B. mit ß-Adrenergika, Parasympatholytika, Corticosteroiden, Antiallergika, Sekretolytika, Antibiotika kombiniert werden. Bei der Kombination mit TNF (Tumor-Nekrose-Faktor) wird eine bessere Verträglichkeit (Ausschaltung störender Nebenwirkungen) des TNF erreicht; TNF läßt sich daher gewünschtenfalls auch in höheren Dosen einsetzen als bei seiner alleinigen Anwendung.

(Unter "Kombination" ist hier auch die Anwendung der beiden Wirkstoffe in getrennten Zubereitungen und in einem gewissen zeitlichen Abstand zu verstehen). Bei der gemeinsamen Anwendung der erfindungsgemäßen Verbindungen mit ß-Adrenergika kann ein synergistischer Effekt erzielt werden, z.B. in der Broncholyse. Sehr vorteilhaft ist auch die Kombination der PAF-Antagonisten mit Immunsuppressiva, z.B. den verschiedenen Cyclosporinen.

Die neuen Verbindungen können topisch, oral, parenteral oder durch Inhalation verbreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Inhalationsaerosole, Salben, Emulsionen, Siruppe, Suppositorien.

Die therapeutischen und prophylaktische Dosis ist abhängig von der Beschaffenheit und Ernsthaftigkeit des Krankheitszustandes.

Eine wirksame Dosis der erfindungsgemäßen Verbindungen liegt bei oraler Anwendung zwischen 1 und 100, vorzugsweise zwischen 10 und 80 mg/Dosis, bei intravenöser oder intramuskulärer Anwendung zwischen 0,001 und 50, vorzugsweise zwischen 0,1 und 30 mg/Dosis. Für die Inhalation sollen Lösungen, die 0,01 bis 1,0, vorzugsweise 0,1 bis 0,5 % Wirkstoff enthalen, eingesetzt werden, ferner Pulver und Suspensionen in verflüssigten Treibgasen. Die PAF-antagonistische Wirkung einzelner Triazolobenzodiazepine ist bekannt, vgl. E. Kornecki et al., Science 226, 1454 - 1456 (1984). Für Alprazolam z. B. wurde nach der unten beschriebenen Methode ein $IK_{50}$ (Konzentration bei einer 50%igen Aggregationshemmung) von 14 µM, für Triazolam ein $IK_{50}$ von 9 µM gemessen. Diese, als Tranquilizer beziehungsweise Hypnotika ausgewiesenen und im Handel befindlichen Verbindungen sind jedoch wegen ihrer ausgeprägten sedierenden Wirkung trotz ihrer PAF-antagonistischen Wirkung zum Einsatz in der Therapie in vielen Fällen ungeeignet.

Bei den erfindungsgemäßen Verbindungen hingegen fehlt die sedierenden Wirksamkeit, während die PAF-antagonistische Wirkung im Vergleich zu derjenigen der bekannten Benzodiazepine zumindest gleichwertig ist.

Die PAF-antagonistische Wirksamkeit einiger Verbindungen der Formeln Ia und Ib wurde anhand der Hemmung der Blutplättchen-Aggregation in vitro geprüft.

1. In vitro Untersuchungen: Hemmung der Blutplättchen-Aggregation

Zur Bestimmung der PAF-antagonistischen Wirkung von Substanzen wurde die durch PAF induzierte Aggregation von Humanthrombozyten in vitro verwendet. Zur Gewinnung von thrombozytenreichem Plasma (TRP) erfolgt die Blutentnahme aus einer nicht gestauten Vene mit Hilfe einer Plastikspritze, in der sich 3,8 %ige Natriumcitratlösung befindet. Das Verhältnis zwischen Natriumcitratlösung und Blut beträgt 1:9. Nach vorsichtiger Durchmischung wird das Citratblut bei 150 x g (1200 U/min) 20 Minuten lang zentrifugiert. Die Messung der Thrombozytenaggregation erfolgt nach dem von Born und Cross ausgearbeiteten Verfahren (G. V. R. Born und M.J. Cross, J. Physiol. 168, 178 (1963)), wobei dem TRP unter ständigem Rühren PAF als Auslöser der Aggregation zugesetzt wird.

Die Prüfsubstanz wird jeweils 2 - 3 Minuten vor Auslösung der Aggregation in einem Volumen von 10 µl zugesetzt. Als Lösungsmittel dienen entweder Aqua.dest., Ethanol und/oder Dimethylsulfoxyd. Kontrollansätze erhalten entsprechende Volumina dieser Lösungsmittel. Nach Registrierung der Ausgangsabsorption (2-3 Minuten) wird die Aggregation mit PAF ($5 \times 10^{-8}$ M) induziert.

Zur Beurteilung von Substanzeffekten wird das Maximum der ersten Aggregationswelle verwendet. Die durch PAF induzierte maximale Absorptionsrate (= maximale Aggregation x 100 %) wird jeweils gleichzeitig in einem Parallelansatz (= Kontrollansatz in einem der Kanäle des 2 Kanal-Aggregometers) zu jedem Testansatz (zweiter Kanal) mitgeprüft und als 100 %-Wert verwendet. Der unter dem Einfluß der Testsubstanz erreichte Aggregationswert wird als 100 % angegeben.

Jede Prüfsubstanz wird bei Konzentrationen von $10^{-3}$ bis $10^{-8}$ M mit einem Stichprobenumfang von jeweils n = 4 hinsichtlich einer hemmenden Wirkung auf die durch PAF induzierte Thrombozytenaggregation untersucht. Danach wird eine Konzentrations-Wirkungskurve anhand von 3 Konzentrationen erstellt und

die $IK_{50}$ (Konzentration bei einer 50%igen Aggregationshemmung) ermittelt. Bei einigen Verbindungen werden lediglich orientierende Versuche durchgeführt.

Die Ergebnisse sind in Tabelle A dargestellt.

Tabelle A

| Verbindung | Hemmung der PAF-induzierten Plättchenaggregation $IK_{50}$ [μMol] |
|---|---|
| Verbindung nach Beispiel 1 | 0.67 |
| Verbindung nach Beispiel 4 | 0.17 |
| Verbindung nach Beispiel 1a | 0.29 |
| Verbindung nach Beispiel 9a | 0.16 |
| Verbindung nach Beispiel 9 | 0.09 |

[$^3$H]PAF-Rezeptor-Bindung an vitalen humanen Blutplättchen

Bestimmt wird die konkurrierende Wechselwirkung von Prüfsubstanzen (hier PAF-Antagonisten) mit der bekannten Wechselwirkung des Radioliganden [$^3$H]PAF zum gleichen Rezeptor.

Die Bindungsstudien wurden an vitalem menschlichen Thrombozyten vorgenommen. Blutproben von gesunden Spendern wurden mit ACD-Puffer verdünnt und zentrifugiert (15 Min., 160 x g). Das blättchenreiche Plasma wird durch Chromatographie an Sepharose CL-2B [Zum Eluieren: HEPES-Puffer, pH 7,4 20° C] gereinigt.

Definierte Mengen [z.B. 800 μl] der Blättchensuspension wurden 90 Min. bei Raumtemperatur inkubiert, d.h. vermischt mit:

a) einer 30 mmolarem [$^3$H]PAF-Lösung, verdünnt mit Puffer.

b) mit einer 30 p molaren [$^3$H] PAF-Lösung, die gleichzeitig eine μ-molare (nicht markierte) PAF-Lösung enthält,

c) mit der 30 pmolaren [$^3$H]PAF-Lösung und den Lösungen der Prüfsubstanzen (unterschiedliche Konzentrationen)

a) Dient zur Ermittlung der totalen Bindung,

b) Dient zur Ermittlung der unspezifischen Bindung.

Die Reaktion wurde durch Vakuumfiltration abgestoppt. Die Filter mit den Blutplättchen werden mit Scintilationsflüssigkeit versetzt und die verbliebene Radioaktivität in einem Counter gemessen. Die spezifische Bindung ergibt sich aus totaler Bindung minus unspezifischer Bindung. Man bestimmt entweder die $IC_{50}$-Werte (d.h. jene Konzentration der Prüfsubstanz, die 50 % des Radioliganden - hier [$^3$H] PAF - vom Rezeptor verdrängt), und gibt diese an. Oder errechnet hieraus die Ki-Werte. Dies kann computerunterstützt durch ein Iterationsverfahren der Bindungskurven geschehen. $IC_{50}$- bzw. Ki-Werte sind ein Maß für die Rezeptoraffinität der Testsubstanz. Kleinere Werte zeigen die höhere Affinität an.

Beispiel 1

2-(Morpholinylcarbonylethyl)-4-(2-chlorphenyl)-6,9-dimethyl-6H-thieno[3,2-f][1,2,4]triazolo[4,3-a][1,4]diazepin

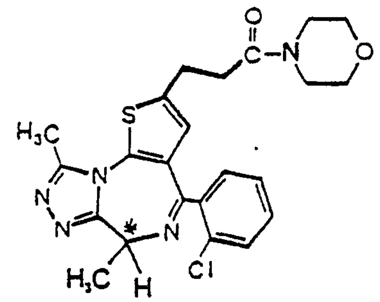

2,4 g (6 mmol) des 2(Carboxyethyl)-4-(2-chlorphenyl)-6,9-dimethyl-6H-thieno[3,2-f][1,2,4]triazolo[4,3-a]-[1,4]diazepin werden in 50 ml Dichlormethan gelöst und mit 1,06 g 1,1-Carbonyldiimidazol versetzt. Nach 45 Minuten werden 0,5 ml (6 mmol) Morpholin hinzugegeben. Man läßt 12 Stunden reagieren. Danach wird das Reaktionsgemisch 1x mit gesättigter Natriumhydrogen- carbonatlösung und 1x mit Wasser extrahiert, dann wird die getrocknete organische Phase über einer Schicht aus Kiselgur/Kohle filtriert. Nach üblicher chromatographischer Aufarbeitung ($SiO_2$) und Kristallisation aus Ether erhält man 1,83 g weiße Kristalle vom Schmelzpunkt 164 °C.

$^1$H-NMR($CDCl_3$) $\delta$ : 7.24-7.57 (4H, m, Aryl-H); 6.47 (1H, s, Thiophen-H); 4.34 (1H, qu, J=7Hz, CH-$CH_3$); 3.39-3.80 (8H, m, Morpholin-H); 3.15 (2H, t, J=7Hz, Thiophen-$CH_2$); 2.68 (3H, s, $CH_3$-Triazol); 2.63 (2H, t, J=7Hz, $CH_2C=O$); 2.09 (3H, d, J=7Hz, CH-$CH_3$).

Hemmung der Blutplättchen-Aggregation 0,67 µM Affinität zum PAF-Rezeptor 16 nM

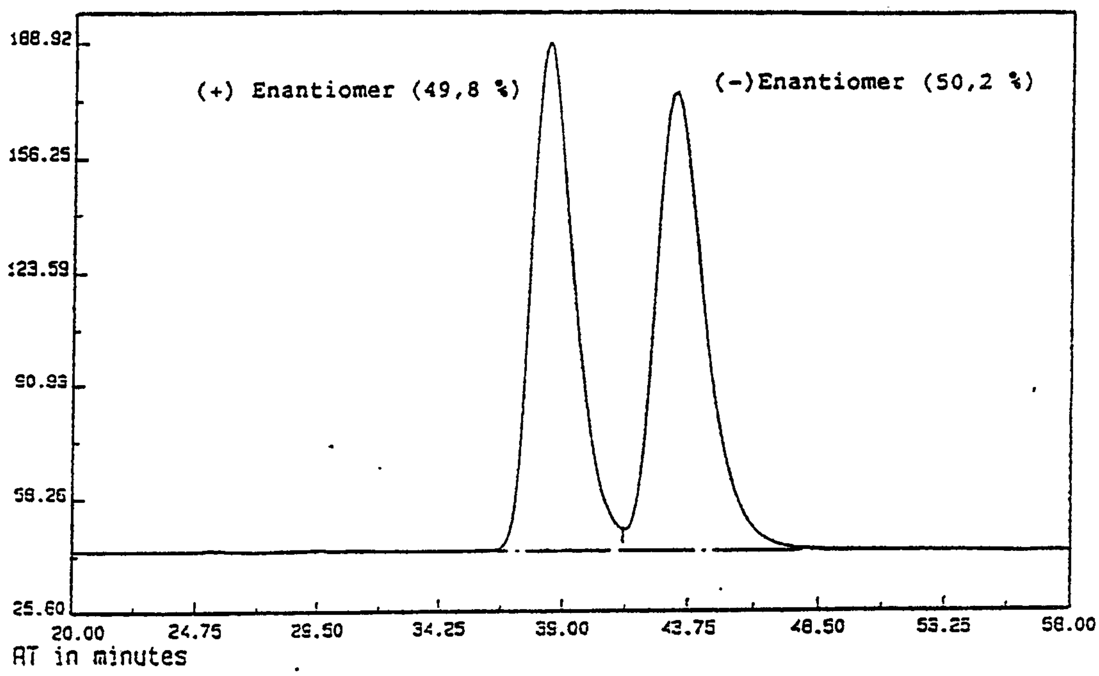


Chromatogramm des Racemates von Beispiel 1 (Web 2315)

Stationäre Phase:
Poly-N-aryloyl)-L-phenylalaninethylester
Säulendimension: 250-4 mm
Eluens: n-Hexan/Dioxan 55 : 45

100 mg des Racemats werden auf einer semipräparativen Säule (250 mm x 10 mm i.d.), welche mit Poly-N-acryloyl-L-phenylalaninethylester gefüllt ist, in die Enantiomeren getrennt.

Als mobile Phase wird ein Gemisch von n-Hexan und Dioxan (60/40 v/v) benutzt. Der Durchfluß beträgt 5 ml/min, die Meßwellenlänge 254nm, die Temperatur 20 °C. Es werden pro Injektion 10 mg des Racemats,

gelöst in mobiler Phase, auf die Säule aufgegeben und die getrennten Enantiomere einzeln isoliert. Die optische Reinheit der isolierten Enantiomeren wird anschließend auf einer analytischen Säule, gefüllt mit der gleichen Trennphase, kontrolliert (Durchlaß: 1 ml/min). Die getrennten Enantiomere werden nochmals auf der semipräparativen Säule nachgereinigt und erneut auf der analytischen Säule ihre optische Reinheit (ee) bestimmt. Es wurden von beiden Antipoden jeweils 30 mg mit einer optischen Reinheit von > 99 % erhalten. Die spezifische Drehung beider Komponenten wurde gemessen:
Verbindung 1A (+)-Form:$[\alpha]_D^{20}$ = +64,9 (Methanol)
Verbindung 1B (-)-Form:$[\alpha]_D^{20}$ = -63,6 (Methanol)

Es wurde gefunden, daß die (-)-Form (IB) 35 mal stärker an den PAF-Rezeptor bindet als die (+)-Form (1A).

Herstellung der Ausgangsverbindungen:

a) 2-(2-Chlorpropionylamino)-3-(2-chlorbenzoyl)-4-(methoxycarbonylethyl)thiophen

36 g (0.11 Mol) 3-(2-Amino-3-(2-chlorbenzoyl)thiophen-4-yl)propionsäuremethylester werden in 371 ml Dioxan gelöst, anschließend werden 8.9 ml Pyridin und 15,5 g (0.12 Mol) 2-Chlorpropionsäurechlorid zugegeben. Man läßt das Reaktionsgemisch zwei Stunden bei Raumtemperatur reagieren, saugt die ausgefallenen Kristalle ab und engt das Filtrat ein. Der Rückstand wird mit $CH_2Cl_2$ und Wasser geschüttelt. Anschließend wird die getrocknete organische Phase über eine Schicht aus Kieselgur/Aktivkohle filtriert und eingeengt.. Der Rückstand wird aus Ether umkristallisiert. Man erhält 38 g der Titelverbindung als hellgelbe Kristalle vom Schmelzpunkt 106-108° C.

b) 2-(2-Jodpropionylamino)-3-(2-chlorbenzoyl)-4-(methoxycarbonylethyl)thiophen

40.0 g (96,5 mmol) der nach a erhaltenen Chlorverbindung werden in 350 ml wasserfreiem Aceton gelöst und mit 29 g (193 mmol) Natriumjodid versetzt. Nach 3 Stunden wird über Kieselgur filtriert und eingeengt. Den in Dichlormethan aufgenommenen Rückstand extrahiert man mehrmals mit Wasser und isoliert aus der organischen Phase nach Umkristallisieren aus Petrolether 46.7 g der Titelverbindungen vom Schmelzpunkt 116-118° C.

c) 2-(2-Aminopropionylamino)-3-(2-chlorphenyl)-5-(methoxycarbonylethyl)thiophen

46,0 g (91 mmol) der Jodverbindung hergestellt nach b - werden in 850 ml Essigester gelöst. In diese Lösung wird 4 Stunden lang $NH_3$-Gas eingeleitet. Man läßt 24 h nachreagieren, extrahiert mit Wasser und engt die getrocknete organische Phase nach einer Filtration über Kieselgur/Kohle ein. Man erhält 44,17 g eines hellbraunen Öls.

d) 3-Methyl-5-(2-chlorphenyl)-7-(methoxycarbonylethyl)-3H-thieno[3,2-f][1,4]diazepin-2-on

35,2 g (102 mmol) der nach c hergestellten Verbindung und 193 g Kieselgel werden in 850 ml Toluol und 3,5 Stunden am Wasserabscheider unter Rückfluß erhitzt. Anschließend wird das Toluol dekantiert und das Kieselgel 4 mal mit je 600 ml Methanol ausgekocht. Die vereinigten Methanol-Phasen werden eingeengt und der Rückstand aus Ether kristallisiert. Man erhält 16 g der Titelverbindung als hellgelbe Kristalle vom Schmelzpunkt 200-202° C.

e) 3-Methyl-5-(2-chlorphenyl)-7-(methoxycarbonylethyl)-3H-thieno[3,2-f][1,4]diazepin-2-thion

Man erhält die Titelverbindung ausgehend von dem entsprechenden Diazepin-2-on durch Umsetzung mit Phosphorpentasulfid in Diglyme bei ca. 65° C als gelbe Kristalle vom Schmelzpunkt 193-196° C.

f) 2-Hydrazono-3-methyl-5-(2-chlorphenyl)-7-(methoxycarbonylethyl)-3H-thieno[3,2-f][1,4]diazepin

Man erhält die Titelverbindung aus dem entsprechenden Diazepinthion - hergestellt nach e - nach der Umsetzung mit Hydrazinhydrat als rotbraunes Öl.

g) 2-(Methoxycarbonylethyl)-4-(2-chlorphenyl)-6,9-dimethyl-6H-thieno[3,2-f][1,2,4]triazolo[4,3-a][1,4]diazepin

8.42 g (23 mmol) des aus der zuvor beschriebenen Reaktionsstufe erhaltenen Öls werden in 67 ml wasserfreien Ethanol gelöst und mit 12,7 ml Orthoessigsäureethylester versetzt. Nach einstündiger Reaktionszeit bei Rückflußtemperatur wird die Reaktionslösung eingeengt und der Rückstand chromatographisch aufgearbeitet ($SiO_2$) ($CH_2Cl_2/CH_3OH$ 96/4). Das erhaltene Eluat wird eingeengt und der Rückstand aus Ether umkristallisiert. Man erhält 7,2 g der Titelverbindung als weiße Kristalle vom Schmelzpunkt 119-120 °C.

h) 2-(Carboxyethyl)-4-(2-chlorphenyl)-6,9-dimethyl-6H-thieno-[3,2-f][1,2,4]triazolo[4,3-a][1,4]diazepin

Die Titelverbindung erhält man durch alkalische Verseifung mit KOH in Ethanol des entsprechenden Methylesters als weißbeige Kristalle vom Schmelzpunkt 200-203 °C.

Beispiel 1a

2-(N,N-Diethylaminocarbonylethyl)-4-(2-chlorphenyl)-6,9-dimethyl-6H-thieno[3,2-f][1,2,4]triazolo[4,3-a][1,4]-diazepin

Die Titelverbindung erhält man in Analogie zu Beispiel 1 durch Umsetzung der Säure mit Diethylamin. $^1$H-NMR($CDCl_3$) δ : 7.23-7.63 (4H, m, Aryl-H); 6.46 (1H, s, Thiophen-H); 4.35 (1H, qu, J=7Hz, CH-$CH_3$); 3.38 (4H, 2qu, J=7Hz, 2N-$CH_2CH_3$); 3.15 (2H, t, J=7Hz, Thiophen-$CH_2$); 2.69 (3H, s, $CH_3$-Triazol); 2.63 (2H, t, J=7Hz, $CH_2$-C=O); 2.11 (3H, d, J=7Hz, CH-$CH_3$); 1.11 (6H, 2t, J=7Hz, 2N-$CH_2CH_3$).

Beispiel 2

2-(Morpholinylcarbonylethyl)-4-(2-chlorphenyl)-6-hydroxy-9-methyl-6H-thieno[3,2-f][1,2,4]triazolo[4,3-a][1,4]-diazepin

1,2g (2,3 mmol) 2-(Morpholinylcarbonylethyl)-4- (2-chlorphenyl)-6-acetoxy-9-methyl-6H-thieno[3,2-f]-[1,2,4]triazolo[4,3-a][1,4]diazepin Beispiel 9c und 2,4 g (2.3 mmol) Imidazol werden in 24 ml Ethanol und 12 ml Wasser suspendiert und drei Stunden unter Rückfluß erhitzt. Das Reaktionsgemisch wird im Vakuum teilweise eingeengt, der Rückstand mit 2N HCl neutralisiert und mit Dichlormethan extrahiert. Aus der organischen Phase erhält man nach der Aufarbeitung und Filtration über eine mit $SiO_2$ gefüllte Säule (Lsgm: $CH_2Cl_2/CH_3OH$ 58:2) 0,6 g der Titelverbindung vom Schmelzpunkt 175-182 °C (aus Ether). $^1$H-NMR($CDCl_3$) δ : 7.24-7.75 (4H, m, Aryl-H); 6.48 (1H, s, Thiophen-H); 5.75 (1H, s, CH-OH); 3.98 (1H, s, breit, OH); 3.31-3.79 (8H, m, Morpholin-H); 3.15 (2H, t, J=7Hz, $CH_2$-Thiophen); 2.71 (3H, s, $CH_3$-Triazol); 2.64 (2H, t, J=7Hz, $CH_2$-C=O).

Beispiel 2a

2-(Morpholinylcarbonylethyl)-4-(2-chlorphenyl)-6-(3-hydroxypropyl)-9-methyl-6H-thieno[3,2-f][1,2,4]triazolo-[4,3-a][1,4]diazepin

Man erhält die Titelverbindung aus dem Ester von Beispiel 6 durch $NaBH_4$-Reduktion analog Beispiel 4

-aus Essigester kristallisiert- vom Schmelzpunkt 110 °C (Substanz sintert).
$^1$H-NMR($CDCl_3$) δ : 7.32-7.49 (4H, m, Aryl-H); 6.47 (1H, s, Thiophen-H); 4.18 (1H, m, CH-Siebenring); 3.78 (2H, t, J=6Hz, $OCH_2$); 3.37-3.71 (8H, m, Morpholin-H); 3.16 (2H, m, Thiophen-$CH_2$); 3.09 (1H, t, J=7Hz, OH); 2.70 (3H, s, $CH_3$-Triazol); 2.67 (4H, m, $CH_2$-C=O u. $OCH_2\underline{CH_2}$; 1.95 (2H, m, $OCH_2CH_2\underline{CH_2}$).

Beispiel 3

2-(Morpholinylcarbonylethyl)-4-(2-chlorphenyl)-6-carboxyethyl-9-methyl-6H-thieno[3,2-f][1,2,4]triazolo[4,3-a]-[1,4]diazepin

19,9 g (36,7 mmol) des 2-(Morpholinyl)-4-(2-chlorphenyl)-6-(methoxycarbonylethyl)-9-methyl-6H-thieno-[3,2-f][1,2,4]triazolo[4,3-a][1,4]diazepin (Beispiel 6) werden mit 190 ml 2N Kalilauge bei Raumtemperatur verseift. Es folgt die übliche extraktive Aufarbeitung mit Dichlormethan, Filtrierung über eine Kieselgelsäule, Lösen des eingeengten Rückstandes in Natriumhydrogencarbonatlösung, Filtration über Kieselgur/ Kohle und anschließendes Fällen der Säure mit 2N HCl bei pH 4. Der Niederschlag wird mit Dichlormethan extrahiert und die organische Phase aufgearbeitet. Der so erhaltene Rückstand wird mit Ether verrieben und abgesaugt. Man erhält 4,2 g der Titelverbindung.
$^1$H-NMR($CDCl_3$) δ : 7.35-7.52 (4H, m, Aryl-H); 6.48 (1H, s, Thiophen-H); 4.40 (1H, t, J=8Hz, CH-Siebenring); 3.36-3.80 (8H, m, Morpholin-H); 3.16 (2H, m, Thiophen-$CH_2$); 2.86 (4H, m, $CH_2CH_2$-COO); 2.71 (3H, s, $CH_3$-Triazol); 2.58 (2H, m, $CH_2C=O$).

Beispiel 3a

5-(2-Chlorphenyl)-7-(carboxyethyl)-10-methyl-3,4-dihydro-2H, 7H-cyclopenta[4,5]thieno[3,2-f][1,2,4,]triazolo-[4,3-a]diazepin

In Analogie zu Beispiel 3 erhält man aus dem entsprechenden Methylester (Öl) durch alkalische Hydrolyse die Titelverbindung als Kristalle aus Essigester vom Schmelzpunkt 244-246 °C.

Beispiel 4

2-(Morpholinylcarbonylethyl)-4-(2-chlorphenyl)-6-hydroxymethyl-9-methyl-6H-thieno[3,2-f][1,2,4]triazolo[4,3-a][1,4]diazepin

5,28 g (10 mmol) des entsprechenden Diazepin-6-yl-carbonsäureethylesters (Beispiel 5), gelöst in 25 ml Methanol werden bei 0-10 °C mit einem Überschuß Natriumborhydrid ($NaBH_4$) - gelöst in $H_2O$ - versetzt. Nachdem in einem Dünnschichtchromatogramm kein Ausgangsprodukt mehr feststellbar ist, wird das Reaktionsgemisch vorsichtig mit Eisessig neutralisiert, das Methanol abdestilliert und der Rückstand zwischen Wasser/Dichlormethan verteilt. Aus der organischen Phase erhält man nach der Aufarbeitung 4,6 g der Titelverbindung als Schaum.

Die Substanz wird über eine Kieselgelsäule filtriert (Elutionsmittel: Essigester/Methanol 70/30) und kristallisiert nach der Aufarbeitung aus Essigester. Schmelzpunkt: 221-222 °C
$^1$H-NMR($CDCl_3$) δ : 7.35-7.54 (4H, m, Aryl-H); 6.50 (1H, s, Thiophen-H); 4.63 (2H, m, $OCH_2$); 4.40 (1H, t, J=8Hz, CH-Siebenring); 3.33-3.72 (8H, m, Morpholin-H); 3.40 (1H, t, J=6Hz), OH); 2.77 (2H, m, $CH_2$-Thiophen); 2.73 (3H, s, $CH_3$-Triazol); 2.65 (2H, m, $CH_2C=0$).

Beispiel 4 a

2-(4-Isobutylphenylethyl)-4-(2-chlorphenyl)-6-hydroxymethyl-9-methyl-6H-thieno[3,2-f][1,2,4]triazolo-[4,3-a]-[1,4]diazepin

Analog Beispiel 4 erhält man aus dem Diazepin-6-yl-carbon-säureester (Beispiel 5 b) die Titelverbindung als gelbes Öl nach Flash-Chromatographie an Kieselgel mit Methylenchlorid/Methanol (9 : 1) als Eluens in einer Ausbeute von 36 %.

$^1$H-NMR ($CDCl_3$) δ:
7,47 - 7,28 (4H, m, Aryl-H)
7,08 - 6,99 (4H, m, p-Aryl-H)
6,36 (1H, s, Thiophen-H)
4,70 - 4,49 (2H, m, $-CH_2-OH$)
4,36 (1H, t, J=6Hz-CH-7-Ring)
3,05 (2H, t, J=7Hz, Thiophen-$CH_2$)
2,91 (2H, t, J=7Hz, Aryl-$CH_2$)
2,64 (3H, s, $CH_3$-Triazol)
2,44 (2H, d, J=7Hz, Aryl-$CH_2$-CH)
1,82 (1H, m, Isopropyl-CH-)
0,89 (6H, d, J=7Hz, 2 x $CH_3$)

Beispiel 5

2-(Morpholinylcarbonylethyl)-4-(2-chlorphenyl)-6-(ethoxycarbonyl)-9-methyl-6H-thieno[3,2-f][1,2,4]triazolo - [4,3-a][1,4]diazepin

6,0 g (13,2 mmol) 2-(Morpholinylcarbonylethyl)-4-(2-chlorphenyl)-9-methyl-6H-thieno-[3,2-f][1,2,4] triazolo[4,3-a][1,4]diazepin werden in 66 ml Diethylcarbonat vorgelegt und mit jeweils 0,64 g (13,2 mmol) 50%ige Natriumhydrid-Dispersion in Öl und 0,14 ml Ethanol versetzt und 1,5 Stunden auf Rückflußtemperatur erhitzt. Man gibt noch einmal 0,3 g Natriumhydrid zu, erhitzt 2 Stunden und läßt das Reaktionsgemisch 12 Stunden stehen. Anschließend wird mit 70 ml Eiswasser, das 1,3 g konz. Salzsäure enthält, versetzt. Man extrahiert mit Dichlormethan und engt die erhaltene organische Phase ein. Der Rückstand wird über eine mit Kieselgel gefüllte Säule filtriert ($CH_2Cl_2$/2-5% Methanol). Aus den vereinigten einheitlichen Fraktionen erhält man 6.3 g der Titelverbindung als Öl.
$^1$H-NMR($CDCl_3$) δ : 7.20-7.70 (4H, m, Aryl-H); 6.40 (1H, s, Thiophen H); 5.80 (1H, s, breit; CH-C=O); 4.26 (2H, qu, H=7Hz, $OCH_2$); 3.28-3.84 (8H, m, Morpholin-H); 3.13 (2H, t, J=7Hz, Thiophen-$CH_2$); 2.70 (3H, s, $CH_3$-Triazol); 2.61 (2H, t, J=7Hz, $CH_2$-C=O); 1.20 (3H, t, J=7Hz, $OCH_2$-$CH_3$).

Beispiel 5a

3-(Morpholinylcarbonyl)-5-(2-chlorphenyl)-7-(ethoxycarbonyl)-10-methyl-3,4-dihydro-2H,7H-cyclopenta[4,5]-thieno[3,2-f][1,2,4]triazolo[4,3-a][1,4]diazepin

Ausgehend von 3-(Morpholinylcarbonyl)-5-(2-chlorphenyl)-10-methyl-3,4-dihydro-2H,7H-cyclopenta[4,5]-thieno[3,2-f][1,2,4]triazolo[4,3-a][1,4]diazepin erhält man in Analogie zu Beispiel 5 die Titelverbindung als nichtkristallisierenden Schaum.
$^1$H-NMR($CDCl_3$) δ : 7.25-7.68 (5H, m, Aryl-H; Thiophen-H); 5.31 (1H, s, CH-C=O); 4.26 (2H, m, breit, $OCH_2$); 3.01-3.91 (8H, m, Morpholin-H); 2.69 (3H, s, $CH_3$-Triazol); 1.03 (3H, t, J=7Hz, $OCH_2$-$CH_3$).

Beispiel 5 b

2-(4-Isobutylphenylethyl)-4-(2-chlorphenyl)-6-(ethoxycarbonyl)-9-methyl-6H-thieno[3,2-f][1,2,4]triazolo[4,3-a]-[1,4]diazepin

Gemäß Beispiel 5 erhält man die 6-Ethoxycarbonylverbindung aus 2-(4-Isobutylphenylethyl)-4-(2-chlorphenyl)-9-methyl-6H-thieno[3,2-f][1,2,4]triazolo[4,3-a][1,4]diazepin, die nach Reinigung durch Flash-Chromatographie (Kieselgel, Methylenchlorid/Methanol (19 : 1) als Eluens) als farbloses Öl in 72 % Ausbeute

anfällt.
$^1$H-NMR ($CDCl_3$) δ:
7,61 - 7,49 (1H, m, Aryl-H)
7,44 - 7,31 (3H, m, Aryl-H
7,09 - 6,98 (4H, m, p-Aryl-H)
6,32 (1H, s, Thiophen-H)
5,86 (1H, s, breit, CH-7-Ring)
4,38 - 4,05 (2H, m, breit, Ester-$CH_2$)
3,04 (2H, t, J = 7Hz, Thiophen-$CH_2$-)
2,89 (2H, t, J = 7Hz, Aryl-$CH_2$-)
2,65 (3H, s, $CH_3$-Triazol)
2,43 (2H, d, J = 7Hz, Aryl-$CH_2$-CH-)
1,82 (1H, m, Isopropyl-CH-)
1,19 (3H, t, breit, Ester-$CH_3$)
0,88 (6H, d, J = 7Hz, 2 x $CH_3$)

Beispiel 5 c

3-(Morpholinomethyl)-5-(2-chlorphenyl)-7-(ethoxycarbonyl)-10-methyl-3,4-dihydro-2H,7H-cyclopenta[4,5]-thieno[3,2-f][1,2,4]triazolo[4,3-a][1,4]diazepin

Aus Diethylcarbonat, Natriumhydrid und 3-(Morpholinomethyl)-5-(2-chlorphenyl)-10-methyl-3,4-dihydro-2H,7H-cyclopenta[4,5]thieno[3,2-f][1,2,4]triazolo[4,3-a][1,4]diazepin wird gemäß Beispiel 5 die 7-Ethoxycarbonylverbindung hergestellt, die nach Flash-Chromatographie an Kieselgel mit Methylenchlorid/Methanol (19:1) als Eluens in 67 % Ausbeute als amorphe Verbindung anfällt.
$^1$H-NMR (DMSO-$d_6$, 373k) δ
7,52 - 7,38 (4H, m, Aryl-H)
5,92; 5,77 (1H, 2 s, CH-7-Ring) Diastereomere
4,20 - 4,04 (2H, m, Ester-$CH_2$)
3,50 (4H, t, O-Morpholin-H)
3,08 - 1,98 (6H, m, $CH_2$-5-Ring, $CH_2$-N-Morpholin)
2,61 (3H, s, $CH_2$-Triazol)
1,78 - 1,63 (1H, m. CH-5-Ring)
1,20 - 1,04 (3H, 2t, Ester-$CH_3$) Diastereomere
2,33 - 2,25 (4H, t, N-Morpholin-H)

Beispiel 5 d

3-(Morpholinomethyl)-5-(2-chlorphenyl)-7-hydroxymethyl-10-methyl-3,4-dihydro-2H,7H-cyclopenta[4,5]-thieno[3,2-f][1,2,4]triazolo[4,3-a][1,4]diazepin

Man erhält die Titelverbindung analog Beispiel 4 aus Beispiel 5c. Das gelbe, amorphe Rohprodukt wird durch Flash-Chromatographie an Kieselgel mit Methylenchlorid/Methanol (9 : 1) als Fließmittel aufgereinigt und das erhaltene Öl durch Verreiben mit Essigester zur Kristallisation gebracht.
Ausbeute 78 %, Fp. 210 - 212° C
$^1$H-NMR ($CDCl_3$) δ:
7,52 - 7,29 (4H, m, Aryl-H)
4,72 - 4,53 (2H, m, -$CH_2$-OH)
4,43 - 4,33 (1H, m, CH-7-Ring)
3,74 - 3,58 (4H, m, O-Morpholin-H)
3,40 - 1,79 (10H, m, $CH_2$-5-Ring, $CH_2$-N-Morpholin, N-Morpholin-H)
2,71 (3H, s, $CH_3$-Triazol)
1,60 - 1,48 (1H, m, CH-5-Ring)

Beispiel 6

2-(Morpholinylcarbonylethyl)-4-(2-chlorphenyl)-6-(methoxycarbonylethyl)-9-methyl-6H-thieno[3,2-f][1,2,4]-triazolo[4,3-a][1,4]diazepin

6,0 g (13,2 mmol) 2-(Morpholinylcarbonylethyl)-4-(2-chlorphenyl)-9-methyl-6H-thieno[3,2-f][1,2,4]triazolo-[4,3-a][1,4]diazepin werden in 50 ml Methylacrylat vorgelegt und mit 0,64 g (13,2 mmol) NaH (50%ige Dispersion in Öl - gewaschen mit Toluol) und 0,14 ml Methanol versetzt, anschließend erhitzt man 4 Stunden unter Rückfluß und läßt weitere 12 Stunden bei Raumtemperatur reagieren. Das zähe Reaktionsgemisch wird dann in 70 ml Eiswasser (enthaltend 1,3 g konz. HCl) gegossen und mit Dichlormethan extrahiert. Aus der organischen Phase erhält man nach der Aufarbeitung 30,2 g eines zähen Öls, das über eine mit Kieselgel gefüllte Säule (Elutionsmittel Dichlormethan/Methanol 96/4) filtriert wird. Die vereinigten einheitlichen Fraktionen werden eingeengt, man erhält 1,8 g der Titelverbindung als Öl.
$^1$H-NMR($CDCl_3$) $\delta$ : 7.35-7.52 (4H, m, Aryl-H); 6.49 (1H, s, Thiophen-H); 4.34 (1H, m, CH-Siebenring); 3.69 (3H, s, $OCH_3$); 3.38-3.81 (8H, m, Morpholin-H); 3.16 (2H, m, $CH_2$-Thiophen); 2.87 (4H, m, $CH_2CH_2COO$); 2.66 (2H, m, $CH_2C=O$); 2.72 (3H, s, $CH_3$-Triazol).

Beispiel 7

2-(3-Morpholinylpropyl)-4-(2-chlorphenyl)-6,9-dimethyl-6H-thieno[3,2-f][1,2,4]triazolo[4,3-a][1,4]diazepin

7,6 g (16,4 mmol) des entsprechenden 2-(3-Methansulfonyloxypropyl)-Derivates werden in 200 ml wasserfreiem Dioxan gelöst, mit 14,3 g (16,4 mmol) Morpholin versetzt und 4 Stunden auf Rückflußtemperaturen erhitzt. Nach üblicher extraktiver Aufarbeitung mit Dichlormethan als Extraktionsmittel, Filtration über eine mit Kieselgel gefüllte Säule ($CH_2Cl_2$/$CH_3OH$ 96/4) erhält man nach der Umkristallisation aus Essigester 1,8 g der Titelverbindung als weißliche Kristalle vom Schmelzpunkt 175 °C.
$^1$H-NMR($CDCl_3$) $\delta$ : 7.24-7.59 (4H, m, Aryl-H); 6.44 (1H, s, Thiophen-H); 4.39 (1H, qu, J=7Hz, $\underline{CH}$-$CH_3$); 3.70; 2.59 (8H, m, Morpholin-H); 2.83 (2H, t, J=7Hz, Thiophen-$CH_2$); 2.70 (3H, s, $CH_3$-Triazol); 2.40 (2H, t, J=7Hz, $CH_2$-N); 2.11 (3H, d, J=7Hz, CH-$\underline{CH_3}$); 1.86 (2H, m, N-$CH_2\underline{CH_2}$-).

Herstellung der Ausgangsverbindung für 2-(3-Methansulfonyloxypropyl)-4-(2-chlorphenyl)-6,9-dimethyl-6H-thieno[3,2-f][1,2,4]triazolo[4,3-a][1,4]diazepin

5,9 g des entsprechenden Propylalkohols werden in 40 ml wasserfreiem Dichlormethan gelöst und mit 1,95 g (17 mmol) Methansulfonylchlorid versetzt. Bei 5 °C werden dann 1,75 g (17,3 mmol) Triethylamin zugetropft und 24 Stunden bei Raumtemperaturen gerührt. Man wäscht einmal mit stark verdünnter $NH_4OH$-Lösung und einmal mit Wasser. Aus der organischen Phase isoliert man nach Filtration über Kieselgur/Kohle 7,6 g der Titelverbindung als zähes braunes Öl, das in die nächste Reaktionsstufe eingesetzt wird.

Beispiel 8

5-(2-Chlorphenyl)-7-(morpholinocarbonylethyl)-10-methyl-3,4-dihydro-2H,7H-cyclopenta[4,5]thieno[3,2-f]-[1,2,4]triazolo[4,3-a][1,4]diazepin

1,3 g (3mmol) der entsprechenden 7-Propansäure werden in 20 ml wasserfreiem Dichlormethan suspendiert und mit 0,6 g (3,6 mmol) 1,1-Carbonyldiimidazol versetzt. Nach 30 Minuten werden bei Raumtemperaturen 0,29 g (3,3 mmol) Morpholin zugegeben. Nach 4 Stunden wird das Reaktionsgemisch mit $NaHCO_3$-Lsg. und Wasser gewaschen. Aus der organischen Phase isoliert man noch einer Filtration über Kieselgel und nach Umkristallisieren aus Essigester 0,6 g der Titelverbindung vom Schmelzpunkt 225-227 °C.
$^1$H-NMR($CDCl_3$) $\delta$ : 7.22-7.55 (4H, m, Aryl-H); 4.43 (1H, m, $\underline{CH}$-$CH_2$-); 3.42-3.84 (8H, m, Morpholin-H); 1.48-2.50 (10H, Cyclopentenyl-H; $CH_2CH_2$-C=O); 2.70 (3H, s, $CH_3$-Triazol).

Beispiel 8a

5-(2-Chlorphenyl)-7-(carboxyethyl)-10-methyl-3,4-dihydro-2H,7H-cyclopenta[4,5]thieno[3,2-f][1,2,4]triazolo-[4,3-a][1,4]diazepin

Die Titelverbindung erhält man aus dem entsprechenden Methylester durch alkalische Verseifung.
$^1$H-NMR($CDCl_3$) δ : 7.22-7.58 (4H, m, Aryl-H); 4.35 (1H, m, CH-$CH_2$); 1.46-3.07 (10H, m, Cyclopentenyl-H, -$CH_2CH_2$-C=O); 2.70 (3H, s, $CH_3$-Triazol).

Beispiel 9

2-(Morpholinylcarbonylethyl)-4-)2-chlorphenyl-6,9-dimethyl-6H-thieno[2,3-f]imidazo[1,2-a][1,4]diazepin.

5,6 g (14 mmol) der entsprechenden Propansäure werden mit 2.5 g (15,4 mmol) 1,1-Carbonyldiimidazol und 1,3 g (15,4 mmol) in 50 ml wasserfreiem Dichlormethan in Analogie zu beschriebenen Verfahren umgesetzt. Man erhält 0,3 g der Titelverbindung als gelbes Öl.
$^1$H-NMR($CDCl_3$) δ : 7.34-7.56 (4H, m, Aryl-H); 6.98 (1H, s, Imidazol-H); 6.50 (1H, s, Thiophen-H); 4.19 (1H, qu,(J=7Hz) CH-$CH_3$); 3.37-3.72 (8H, m, Morpholin-H); 3.16 (2H, t, J=7Hz, $CH_2$-Thiophen); 2.65 (2H, m, $CH_2$C=0); 2.46 (3H, s, $CH_3$-Imidazol); 2.04 (3H, d, J=7Hz, $CH_3$-CH).

Herstellung der Ausgangsverbindung: 2-(Prop-2-inylimino)-3-methyl-5-(2-chlorphenyl)-7-(methoxycarbonylethyl)-1H,3H-thieno[2,3-f][1,4]diazepin

8,3 g (21 mmol) des entsprechenden Diazepinthions werden in 100 ml wasserfreiem Dioxan suspendiert und mit 3,47 g (63 mmol) Propargylamin versetzt. Man rührt 1 Stunde bei 50° C und engt anschließend das Reaktionsgemisch ein.

Der Rückstand wird in $H_2O$/Dichlormethan aufgenommen; aus der organischen Phase erhält man nach der Filtration über eine mit Kieselgel gefüllte Säule (Dichlormethan/Methanol 98/2) 7,8 g der Titelverbindung als Öl.

2-(Carboxyethyl)-4-(2-chlorphenyl)-6,9-dimethyl-6H-thieno[3,2-f]imidazo[1,2-a][1,4]diazepin

7,8 g (18 mmol) des Thienodiazepins der Vorstufe werden in 36 ml konz. Schwefelsäure 20 Minuten auf 100° C erhitzt. Anschließend wird die dunkle Lösung auf 200 g Eis geschüttet und mit konz. wässeriger Natronlauge auf pH4-5 gestellt. Die wässerige Phase wird mehrmals mit Dichlormethan (Zusatz von 5% Methanol) extrahiert. Die vereinigten org. Phasen werden einmal mit gesättigten NaCl-Lösung ausgeschüttelt und aufgearbeitet. Man erhält 5,6 der Titelverbindung als Schaum.

Beispiel 9a

4-(Diethylaminocarbonyl)-6-(2-chlorphenyl)-8,11-dimethyl-2,3,4,5-tetrahydro-8H-[1]benzothieno[3,2-f][1,2,4]-triazolo[4,3-a][1,4]diazepin.

1,1 g (2,6 mmol) der entsprechenden Carbonsäure werden mit 0,35 g (2,6 mmol) N-Hydroxybenzotriazol in 15 ml Dimethylformamid versetzt. Unter Eiskühlung werden 0,64 g (3,1 mmol) Dicyclohexylcarbodiimid und 0,19 g (2,6 mmol) Diethylamin zugesetzt, zur Reaktion gebracht und in Analogie zu Beispiel 1 aufgearbeitet.
Das erhaltene Diastereomerengemisch wird durch chromatographische Trennung an Kieselgel

(Elutionsmittel) Dichlormethan/Methanol 98:2) aufgetrennt.
Aus der 1. Fraktion erhält man nach Kristallisation aus Ether 0,26 g der Titelverbindung vom Schmelzpunkt 205-207° C.
$^1$H-NMR($CDCl_3$) δ : 7.27-7.46 (4H, m, Aryl-H); 4.28 (1H, qu, J = 7Hz, CH-$CH_3$); 3.28 (4H, m, 2N-$CH_2$); 2.69 (3H, s, $CH_3$-Triazol); 1.79-2.98 (7H, m, Cyclohexenyl-H); 2.12 (3H, d, J = 7Hz, CH-$CH_3$); 1.15; 1.02 (6H, 2t, J = 7Hz; 2N-$CH_2CH_3$).

Aus der 2. Fraktion erhält man nach Kristallisation aus Ether das andere Diastereomer der Titelverbindung vom Schmelzpunkt 224-226° C.
$^1$H-NMR($CDCl_3$) δ : 7.23-7.59 (4H, m, Aryl-H); 4.30 (1H, qu, J = 7Hz, CH-$CH_3$); 2.99-3.40 (4H, m, 2N-$CH_2$); 2.67 (3H, s, $CH_3$ Triazol); 1.69-2.98 (7H, m, Cyclohexenyl-H); 2.11 (3H, d, J = 7Hz, CH$CH_3$); 1.03; 1.02 (6H, 2t, J = 7Hz, 2N-$CH_2CH_3$).

Herstellung der Ausgangsverbindungen.

Ausgehend von dem 2-Amino-3-(2-chlorbenzoyl)-5-ethoxycarbonyl-4,5,6,7,-tetrahydro-[1]benzothiophen erhält man durch Umsetzung mit α-Brompropionsäurechlorid das entsprechende N-acylierte Thiophenderivat vom Schmelzpunkt 114-116° C.
In der nächsten Reaktionsstufe erfolgt der Austausch von Brom durch $NaN_3$ zu dem entsprechenden Azid, das im darauffolgendem Reaktionsschnitt mit Raney-Nickel zu dem entsprechenden 2-(2-Aminopropionylamino)-benzothiophenderivat reduziert wird.

Die nachfolgenden Cyclisierung zu dem entsprechenden Diazepinon (Schmp. 225-227° C), Überführung in das Thion (Schmp. 175-177° C), die Synthese der Hydrazino-Verbindung (Schmp. 190-192° C) und Ringschluß mit Orthoessigsäuretriethylester zu der eingangs genannten Carbonsäure (Schmp. 175-177° C) erfolgen in Analogie zu bereits beschriebenen Verfahren.

Beispiel 9b

2-(Morpholinylcarbonylethyl)-4-(2-chlorphenyl)-6-morpholinylmethyl-9-methyl-6H-thieno[3,2-f][1,2,4]triazolo-[4,3-a][1,4]diazepin

2,0g (3,7 mmol) des entsprechenden 6-Methylsulfonyloxomethyl-Hetrazepins (hergestellt aus der entsprechenden 6-Hydroxymethyl-Verbindung durch Umsetzung mit Methansulfonylchlorid) wurden in 50 ml wasserfreiem Dioxan mit 3,22 g (3,7 mmol) Morpholin unter Rückflußbedingungen umgesetzt. Nach 4-stündiger Reaktionszeit engt man ein und nimmt den Rückstand in Wasser/Dichlormethan auf. Die org. Phase wird eingeengt und über eine mit Kieselgel gefüllte Säule (Dichlormethan/Methanol 96/4) filtriert. Nach üblicher Aufarbeitung erhält man die Titelverbindung als nichtkristallisierenden Schaum.
$^1$H-NMR($CDCl_3$) δ : 7.29-7.47 (4H, m, Aryl-H); 6.49(1H, s, Thiophen H); 4.38 (1H, t, J = 7Hz; CH-$CH_2$); 3.29-3.89; 2,48-2.93 (20H, m, Morpholin H; N-$CH_2$; $CH_2C=O$); 3.14 (2H, t, J = 7Hz, Thiophen-$CH_2$); 2.68 (3H, s, $CH_3$-Triazol).

Beispiel 9c

2-(Morpholinylcarbonylethyl)-4-(2-chlorphenyl)-6-(acetoxy)-9-methyl-6H-thieno[3,2-f][1,2,4]triazolo[4,3-a][1,4]-diazepin

1,5 g (3,2 mmol) 2-(Morpholinylcarbonylethyl)-4-(2-chlorphenyl)-9-methyl-6H-thieno[3,2-f][1,2,4]triazolo-[4,3-a][1,4]diazepin-N-oxid werden in 12 ml Acetanhydrid suspendiert und 3 Stunden auf 110° C erhitzt. Die eingeengte Lösung wird mit Eis versetzt und mit konz. Ammoniaklösung neutralisiert. Anschließend wird Dichlormethan zugefügt und mit Wasser gewaschen. Die organische Phase wird eingeengt und der Rückstand über Kieselgel filtriert ($CH_2Cl_2$/$CH_3OH$ 96/4). Aus den vereinigten Fraktionen erhält man 0,2 g der Titelverbindung als Schaum.

Die Ausgangsverbindung erhält man durch Oxidation von 2-(Morpholinylcarbonylethyl)-4-(2-chlorphenyl)-9-methyl-6H-thieno[3,2-f][1,2,4]triazolo[4,3-a][1,4]diazepin mit 30% igem $H_2O_2$ in Eisessig nach länge-

rer Reaktionszeit (Wochen) bei Raumtemperatur.
Bei der chromatographischen Aufarbeitung (Kieselgel: $CH_2Cl_2/CH_3OH$ 96/4) erhält man als 1. Fraktion ein ringgeöffnetes Produkt vom Schmp. 150-152°C und aus der 2. Fraktion nach Kristallisation aus Essigester das N-Oxid vom Schmelzpunkt 208-210°C.
$^1$H-NMR($CDCl_3$) $\delta$ : 7.22- 7.67 (4H, m, Aryl-H); 6.80 (1H, s, CHO); 6.48 (1H, s, Thiophen-H); 3.31-3.80 (8H, m, Morpholin-H): 3.17 (2H, t, J=7Hz, Thiophen-$CH_2$); 2.72 (3H, s. $CH_3$-Triazol); 2.62 (2H, t, J=7Hz, $CH_2C=O$); 2.39 (2H, s, $CH_3$-C=O).

Beispiel 9d

3-(Morpholinocarbonyl)-5-(2-chlorphenyl)-7,10-dimethyl-3,4-dihydro-2H,7H-cyclopenta[4,5,thieno-[3,2-f]-[1.2.4]triazolo[4.3-a] [1,4]diazepin

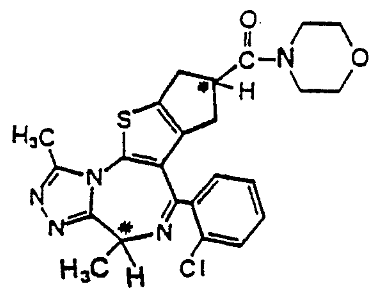

Die Titelverbindung wird aus von dem entsprechenden 2-Amino-3-(2-chlorbenzoyl)4,5-dihydro-6H-cyclopenta[4,5]thiophen wie folgt aufgebaut. Chloracylierung mit Chlorpropionsäurechlorid, Austausch von Chlor durch Jod in der Alkylseitenkette, Umsatz mit Ammoniak und Cyclisierung zum Diazepinring mit Toluol $SiO_2$ ergibt analog Beispiel 9a das entsprechende Diazepinon mit der Methylesterfunktion am Cyclopentanring. (Schmelzpunkt 208-210°C). Durch Umsetzung mit $P_2S_5$ in Diglyme/$NaHCO_3$ erhält man das Diazepinthion vom Schmelzpunkt 196 - 198°C. Umsetzung mit Hydrazin führt zur Hydrazinoverbindung (Schmp. 168 - 172°C), die mit Ortho-Essigsäuretriethylester das Diastereomerengemisch des 3-(Methylcarbonyl)-5-(2-chlorphenyl)-7,10-dimethyl-3,4-dihydro-2H,7H-cyclopenta[4,5]thieno-[3,2-f][1,2,4]-triazolo[4,3-a][1,4]diazepin vom Schmp. 169 - 171° ergibt. Verseifung des Esters zur Carbonsäure und Amidierung mit Morpholin in Gegenwart von Carbonyldiimidazol führt zu der Titelverbindung. Man erhält diese zunächst als 33 : 67- Diastereomerengemisch.

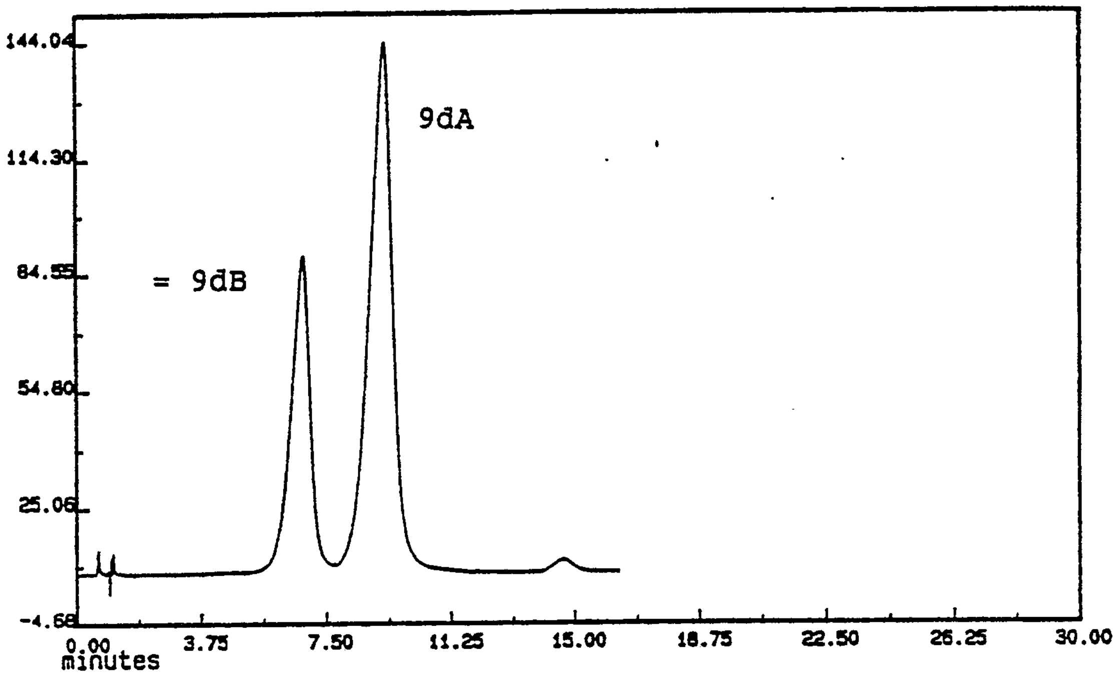


Chromatogramm des Diastereomerengemisches

Stationäre Phase: ODS-Hyperil (5 um)
Säulendimension: 200-6.35 mm
Eluens: Wasser/Methanol 6 : 4
$^1$H-NMR($CDCl_3$) : 7.27-7.61 (4H, m, Aryl-H); 4.38 (1H, qu, J=7Hz, CH-$CH_3$); 2.63-3.93 (13H, m, Cyclopentanyl- u. Morpholin-H); 2.89 (3H, s, Triazol-$CH_3$-; 2.11 (3H, d, J=7Hz, CH-$CH_3$).
[$^3$H]PAF-Bindung: $K_i$ = 83 nM

Durch präparative Säulenchromatographie lassen sich die DIASTEREMEREN trennen ($SiO_2$, Essigester/Methanol). Man erhält die Verbindung 9dA und 9dB mit folgenden NMR-Spekten:

$^1$H-NMR($CDCl_3$) für 9dA:
δ: 7.28 - 7.51 (4H, m, Aryl), 4.36 (1H, qu CH, J = 7Hz). 3.09 - 3.74 (11H, m, Morpholin, Cyclopentyl) 2.68 (3H, s, Triazol-$CH_3$) 2.65 (1H, m, Cyclopentyl) 2.11 (3H, d, $CH_3$, J = 7Hz) 1.92 (1H, m, pentyl)
$^1$H-NMR($CDcl_3$) für 9dB:
δ: 7.28 - 7.43 (4H, m, Aryl), 4.31 (1H, qu, CH, J = 6.7Hz) 3.03 (11H, m, Morpholin, Cyclopentyl) 2.70 (3H, s, $CH_3$) 2.49 (1H, m. Cyclopentyl) 2.11 (3H, d, $CH_3$, J = 6.7 Hz) 2.07 (1H, m. Cyclopentyl)

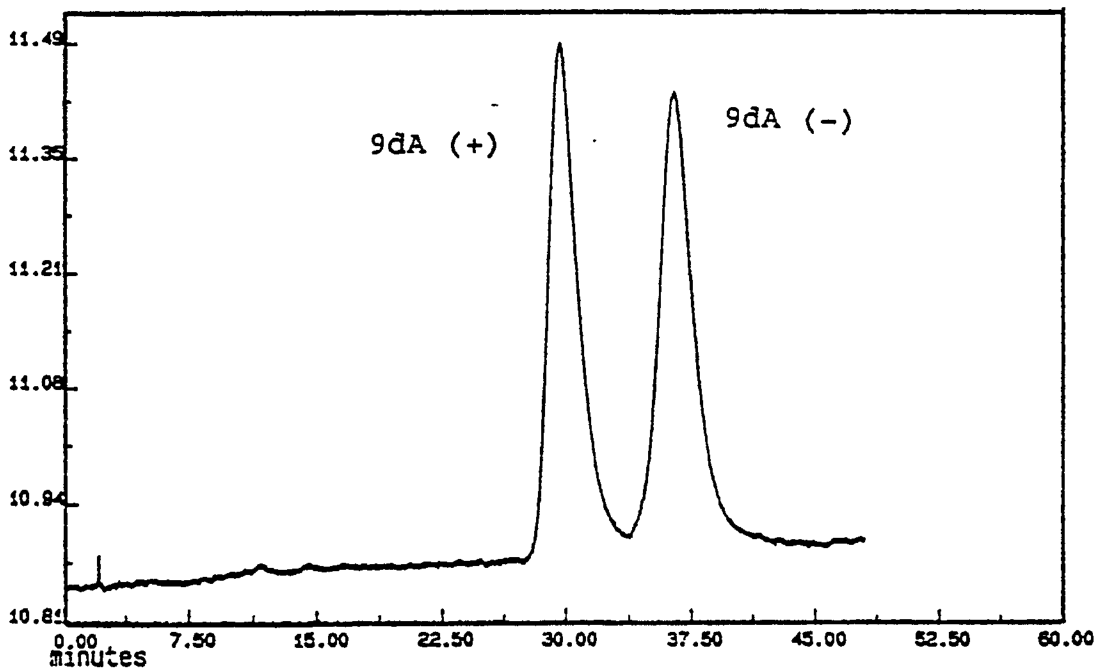


Chromatogramm des Diastereomeren 9dA

Stationäre Phase:
Poly-N-acryloyl-L-phenylalaninethylester
Säulendimension: 250-4 mm
Eluens: n-Hexan/Dioxan 55 : 45

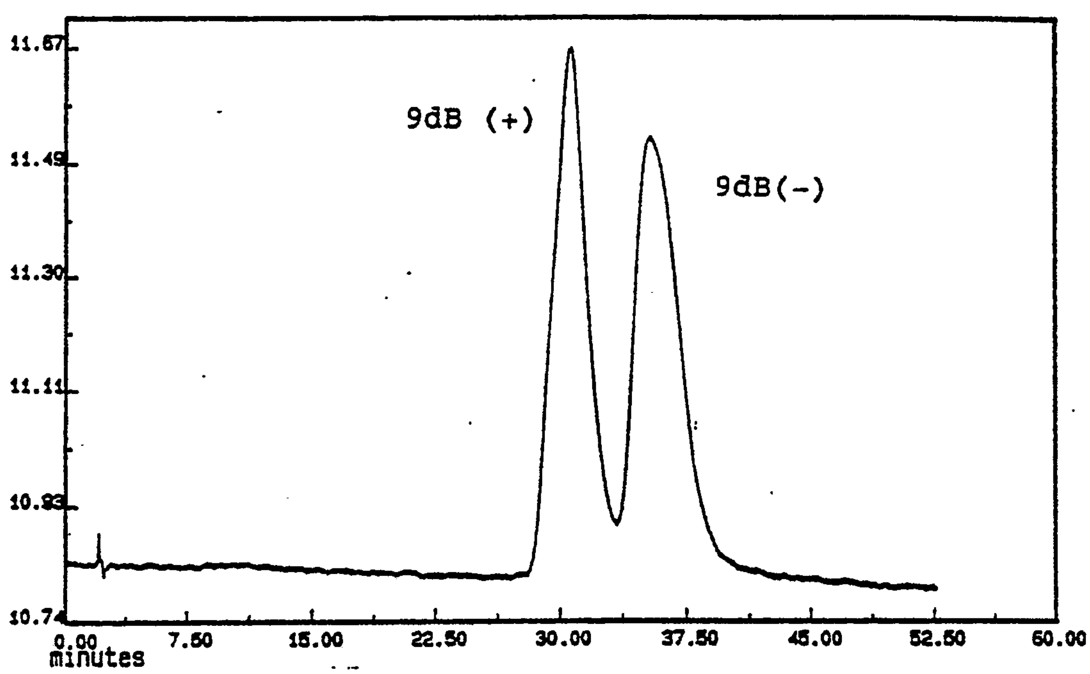


Chromatogramm des Diastereomeren 9dB

Bedingungen wie vorstehend

Je 100 mg obiger Diastereomere werden auf einer semipräparativen Säule (250 mm x 10 mm i.d.), welche mit Poly-N-acryloyl-L-phenylalaninethylester gefüllt ist, in die Enantiomeren getrennt.

Als mobile Phase wird ein Gemisch von n-Hexan und Dioxan (60/40 v/v) benutzt. Der Durchfluß beträgt 5 ml/min, die Meßwellenlänge 254nm, die Temperatur 20°C. Es werden pro Injektion 10 mg des Racemats, gelöst in mobiler Phase, auf die Säule aufgegeben und die getrennten Enantiomere einzeln isoliert. Die optische Reinheit der isolierten Enantiomeren wird anschließend auf einer analytischen Säule, gefüllt mit der gleichen Trennphase, kontrolliert (Durchlaß: 1 ml/min). Die getrennten Enantiomere werden nochmals auf der semipräparativen Säule nachgereinigt und erneut auf der analytischen Säule ihre optische Reinheit (ee) bestimmt. Es wurden von den Antipoden jeweils etwa 30 - 40 mg mit einer optischen Reinheit von > 99 % erhalten. Die spezifische Drehung der Komponenten wurde gemessen:

Die erhaltenen Enantiomere zeigen folgende Chromatogramme:

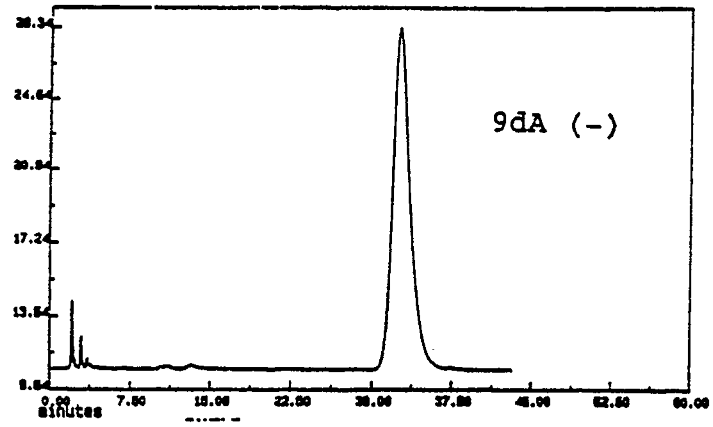
9dA (-)
minutes

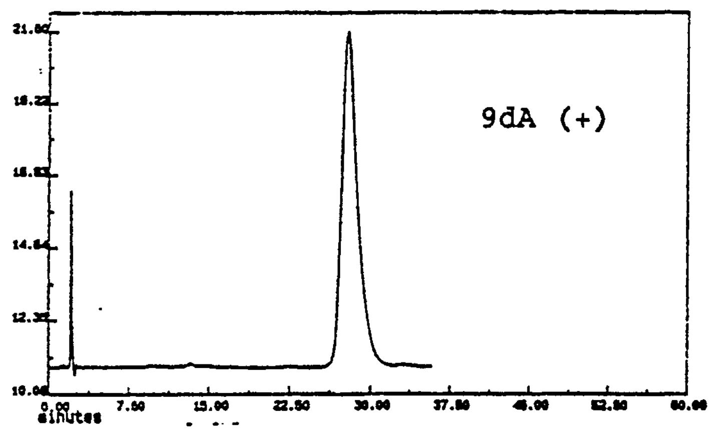
9dA (+)
minutes

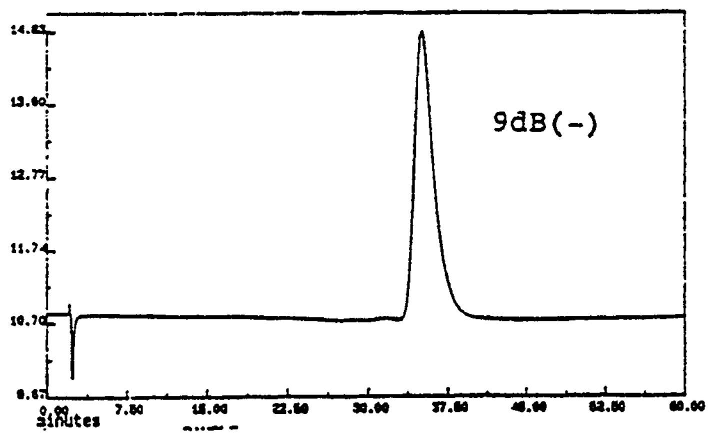
9dB(-)
minutes

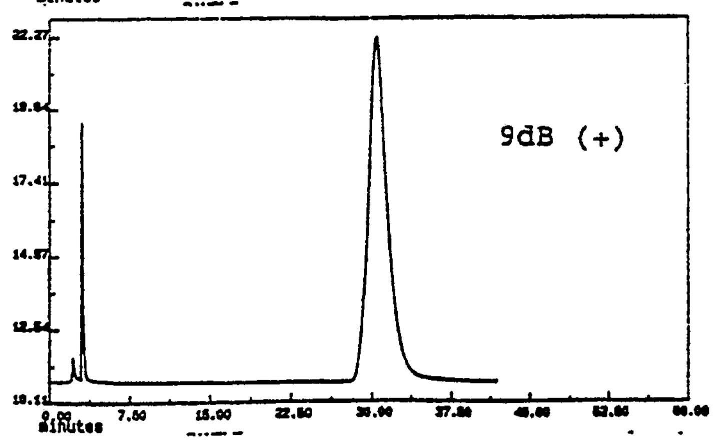
9dB (+)
minutes

Die vier isomeren Verbindungen haben die nachfolgenden Eigenschaften:

| | [$^3$H]PAF-Bindung ($K_i$) |
|---|---|
| (-) 9dA<br>$[\alpha]^D_{20}$ -9.0<br>(c = 1,45, Methanol) | 70 nM |
| (+) 9dA<br>$[\alpha]^D_{20}$ + 10.8<br>(c = 1,64, Methanol) | 400 nM |
| (-) 9dB<br>$[\alpha]^D_{20}$ - 31.6 | 8 nM |
| (+) 9dB<br>$[\alpha]^D_{20}$ + 32.5<br>(c = 1,21, Methanol) | 3000 nM |

Beispiel 9e

4-(Morpholinylcarbonyl)-6-(2-chlorphenyl-8,11-dimethyl-2,3,4,5-tetrahydro-8H-[1]benzothieno[3,2-f][1,2,4]-triazolo[4,3-a][1,4]diazepin

Die Titelverbindung wird ausgehend von den 2-Amino-3-(2-chlorbenzoyl)-5-ethoxycarbonyl-4,5,6,7-tetrahydro-[1] benzothienophen über das Acylderivat (Schmp. 114-116) nach bekannten Analogieverfahren in die Titelverbindung überführt.
Kristalle aus Ether, Schmp. 219-222 °C.
Zwischenverbindungen:
Diazepinon Schmp. 225-227 °C
Diazepinthion Schmp. 175-177 °C
Hydrazono-Verbindung Schmp. 190-192 °C
Triazolobenzothienodiazepin Schmp. 175-177 °C
$C_{23}H_{23}N_4O_2SCl$

Beispiel 9f

3-(Dipropylaminocarbonyl)-5-(2-chlorphenyl)-7,10-dimethyl 3,4-dihydro-2H,7H-cyclopenta[4,5]thieno-[3,2-f]-1,2,4]triazolo[4,3-a][1,4]diazepin

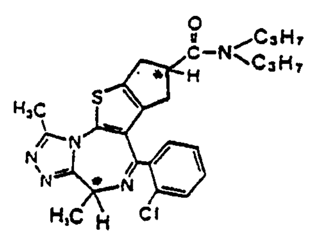

Ausgehend von 3-(Methylcarbonyl)-5-(2-chlorphenyl)-7,10-dimethyl- 3,4-dihydro-2H,7H-cyclopenta[4,5]-thieno[3,2-f][1,2,4]-triazolo[4,3-a][1,4]diazepin vom Schmp. 169 - 171 °, beschrieben in Beispiel 9d, wird durch Verseifung die entsprechende Carbonsäure hergestellt und diese mit Dipropylamin zur Titelverbindung amidiert. Das erhaltene Diasteriomerengemisch läßt sich auf analoge Weise wie in Beispiel 9d beschreiben in die entsprechenden Enantiomere auftrennen.
$^1$H-NMR($CDCl_3$) $\delta$ :
7.57 - 7.22 (4H, m, Aryl-H); 4.36 (1H, m, CH-7-Ring); 3.45 - 2.97 (8H, m, N-$(CH_2)_2$, $CH_2$-5-Ring); 3.70 (1H, m, CH-5-Ring); 2.69; 2.68 (3H, 2s, $CH_3$-triazole); 2.11 (3H, d, J = 6Hz, $CH_3$-CH); 1.50 (4H, m, N(-$CH_2$)$CH_2$)-$_2$); 0.86 (6H, t, J = 6.5 Hz, N-($CH_2$-$CH_2CH_3)_2$).

In Analogie zu den zuvor beschriebenen Beispielen können beispielsweise die Verbindungen der allgemeinen Formel Ia hergestellt werden:

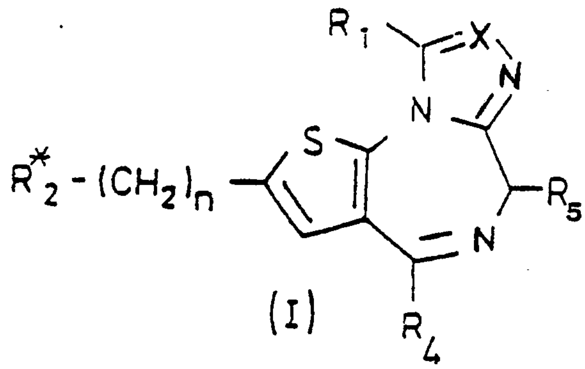

(I)

$R_5$ in der Bedeutung von Methyl-, Hydroxy wie auch Hydroxmethyl

| Nr. | R1 | R2* | R4 | X | n |
|---|---|---|---|---|---|
| 10 | $CH_3$ | OH | 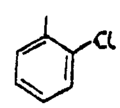 | N | 7 |
| 11 | $CH_3$ | -Cl | " | N | 3 |
| 12 | $CH_3$ | -Cl | " | N | 7 |
| 13 | $CH_3$ | J | " | N | 7 |

| Nr. | $R_1$ | $R_2$* | $R_4$ | X | n |
|---|---|---|---|---|---|
| 14 | $CH_3$ | $-NH_2$ | 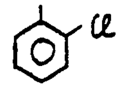 | N | 3 |
| 15 | $CH_3$ | $-SO_2-N$ 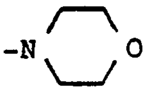 | " | N | 2 |
| 16 | $CH_3$ | $-O-SO_2-i-Pr$ | " | N | 3 |
| 17 | $CH_3$ | $-O-SO_2-$ 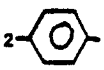 $-CH_3$ | " | N | 3 |
| 18 | $CH_3$ | $-O-SO_2-CH_3$ | " | N | 7 |
| 19 | $CH_3$ | $-O-SO_2-$ 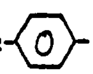 $-CH_3$ | " | N | 7 |
| 20 | $CH_3$ | -O-CO-t-Bu | " | N | 3 |
| 21 | $CH_3$ | -O-CO-i-Pr | " | N | 7 |
| 22 | $CH_3$ | -O-CO-t-Bu- | " | N | 7 |
| 23 | $CH_3$ | $-O-CO-NHCH_3$ | " | N | 7 |
| 24 | $CH_3$ | 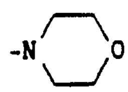 | " | N | 7 |
| 25 | $CH_3$ | 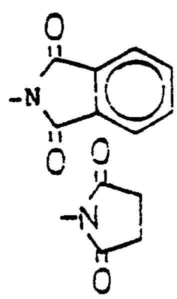 | " | N | 1 |
| 26 | $CH_3$ |  | " | N | 3 |

| Nr. | $R_1$ | $R_2$* | $R_4$ | X | n |
|---|---|---|---|---|---|
| 27 | $CH_3$ | 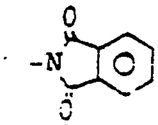 | 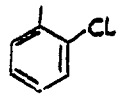 | N | 3 |
| 28 | $CH_3$ | 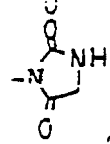 | " | N | 7 |
| 29 | $CH_3$ | 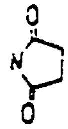 | " | N | 7 |
| 30 | $CH_3$ | $-NH-CO-CH_3$ | " | N | 1 |
| 31 | $CH_3$ | $-NH-CO-CH_3$ | " | N | 3 |
| 32 | $CH_3$ | -NH-CO-i-Pr | " | N | 3 |
| 33 | $CH_3$ | -NH-CO- 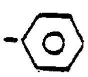 | " | N | 3 |
| 34 | $CH_3$ | $-NH-CO-CH_2$<br>\|<br>$N(CH_3)_2$ | " | N | 3 |
| 35 | $CH_3$ | $-NH-SO_2-$ 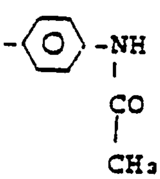 | " | N | 3 |
| 36 | $CH_3$ | -NH-CO-i-Pr | " | N | 7 |
| 37 | $CH_3$ | -NH-CO- 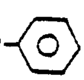 | " | N | 7 |
| 38 | $CH_3$ | $-NH-CO-CH_2$<br>\|<br>$N(CH_3)_2$ | " | N | 7 |

| Nr. | $R_1$ | $R_2^*$ | $R_4$ | X | n |
|---|---|---|---|---|---|
| 39 | $CH_3$ | 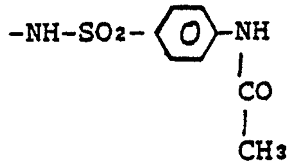 | " | N | 7 |
| 40 | $CH_3$ | 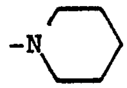 | " | N | 3 |
| 41 | $CH_3$ | 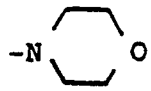 | " | N | 3 |
| 42 | $CH_3$ | 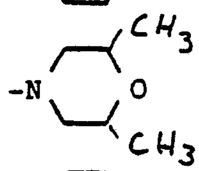 | " | N | 7 |
| 43 | $CH_3$ | 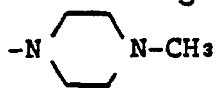 | " | N | 3 |
| 44 | $CH_3$ | 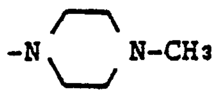 | " | N | 7 |
| 45 | H | $-O-CO-CH_3$ | " | N | 3 |
| 46 | $CH_3$ | $-O-CO-CH_3$ | 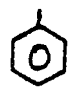 | N | 3 |
| 47 | $CH_3$ | $-OH$ | " | N | 3 |
| 48 | $CH_3$ | $-O-SO_2-CH_3$ | " | N | 3 |
| 49 | $CH_3$ | 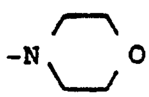 | " | N | 3 |
| 50 | $OCH_3$ | $-O-CO-CH_3$ | 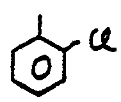 | N | 3 |
| 51 | $CH_3$ | $-O-CO-CH_3$ | " | CH | 3 |

| Nr. | $R_1$ | $R_2$* | $R_4$ | X | n | |
|---|---|---|---|---|---|---|
| 52 | $CH_3$ | -OH | 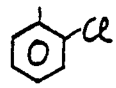 | CH | 7 | |
| 53 | $CH_3$ | $-O-CO-CH_3$ | " | CH | 7 | |
| 54 | $CH_3$ | $-N(CH_3)_2$ | " | N | 7 | |
| 55 | $CH_3$ | $-NH(CH_2)_2$ / $N(CH_{32}$ | " | N | 7 | |
| 56 | $CH_3$ | 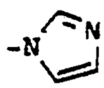 | " | N | 7 | |
| 57 | $CH_3$ | 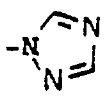 | " | N | 7 | (HCl) |
| 58 | $CH_3$ | 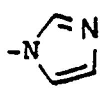 | " | N | 3 | |
| 59 | $CH_3$ | 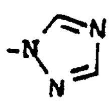 | " | N | 3 | |
| 60 | $CH_3$ | $-N(CH_3)_2$ | " | N | 3 | |
| 61 | $CH_3$ | $-NH(CH_2)_2$ / $N(CH_3)_2$ | | N | 3 | |

| Nr. | $R_1$ | $R_2$* | $R_4$ | X | n |
|---|---|---|---|---|---|
| 62 | $CH_3$ | $-NH-SO_2-$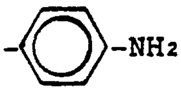 | 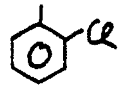 | N | 3 |
| 63 | $CH_3$ | $-NH-SO_2-$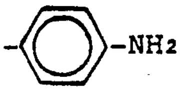 | | N | 7 |
| 64 | $CH_3$ | 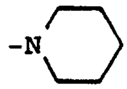 | " | N | 7 |
| 65 | $OCH_3$ | -OH | " | N | 3 |
| 66 | $CH_3$ | $N(C_2H_5)_2$ | " | N | 3 |
| 67 | $CH_3$ | 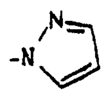 | " | N | 3 |
| 68 | $CH_3$ | 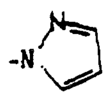 | " | N | 7 |
| 69 | $CH_3$ | 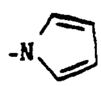 | " | N | 7 |
| 70 | $CH_3$ | $-NH(CH_2)_2-$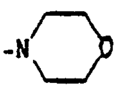 | " | N | 3 |
| 71 | $CH_3$ | $-NHCH_2-$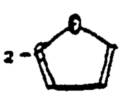 | " | N | 7 |
| 72 | $CH_3$ | $-NH(CH_2)_2$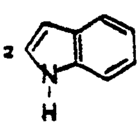 | " | N | 7 |
| 73 | $CH_3$ | 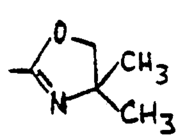 | " | N | 0 |

| Nr. | $R_1$ | $R_2$* | $R_4$ | X | n |
|---|---|---|---|---|---|
| 74 | $CH_3$ | 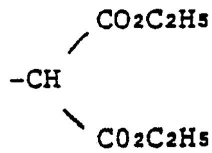 | 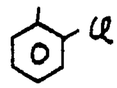 | N | 1 |
| 75 | $CH_3$ | 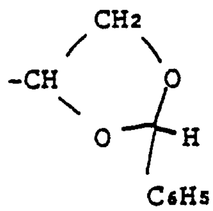 | " | N | 1 |
| 76 | $CH_3$ | 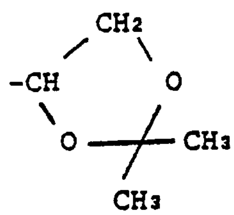 | " | N | 1 |
| 77 | $CH_3$ | 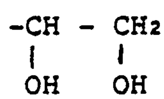 | " | N | 1 |
| 78 | $CH_3$ | 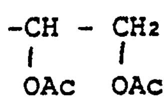 | " | N | 1 |

| Nr. | R1 | R2* | R4 | X | n |
|---|---|---|---|---|---|
| 79 | $CH_3$ | 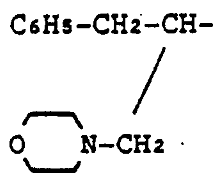 | 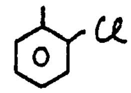 | N | 1 |
| 80 | $CH_3$ | 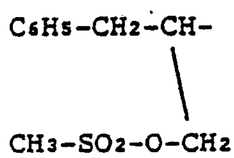 | " | N | 1 |
| 81 | $CH_3$ | 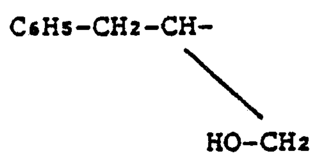 | " | N | 1 |
| 82 | $CH_3$ | $-OC_6H_5$ | " | N | 3 |
| 83 | $CH_3$ | $-OCH_3$ | " | N | 3 |
| 83 | $CH_3$ | $-NH-(CH_2)_2$ 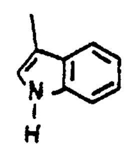 | " | N | 3 |
| 85 | $CH_3$ | -OAc | 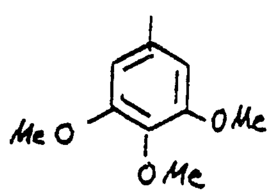 | N | 3 |

| Nr. | $R_1$ | $R_2$* | $R_4$ | X | n |
|---|---|---|---|---|---|
| 86 | $CH_3$ | 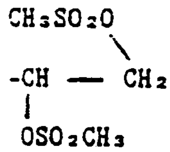 | 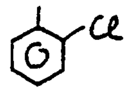 | N | 1 |
| 87 | $CH_3$ | 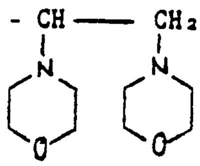 | " | N | 1 |
| 88 | $CH_3$ | 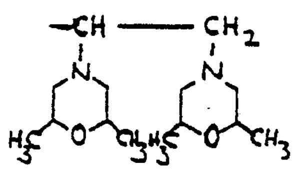 | " | N | 1 |
| 89 | $CH_3$ | 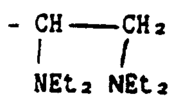 | " | N | 1 |
| 90 | $CH_3$ | 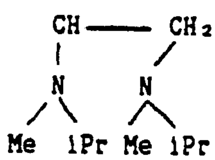 | " | N | 1 |
| 91 | $CH_3$ | 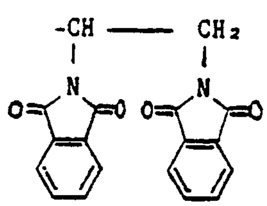 | " | N | 1 |

| Nr. | R1 | R2* | R4 | X | n |
|---|---|---|---|---|---|
| 92 | $CH_3$ | 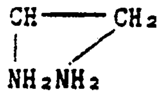 | 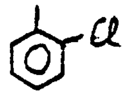 | N | 1 |
| 93 | $CH_3$ | 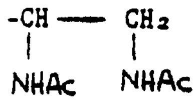 | " | N | 1 |
| 94 | $CH_3$ | 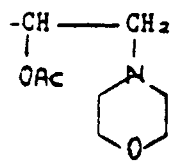 | " | N | 1 |
| 95 | $CH_3$ | 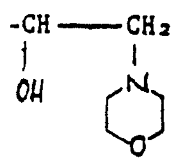 | " | N | 1 |
| 96 | $CH_3$ | 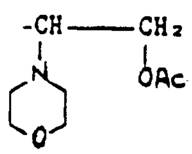 | " | N | 1 |
| 97 | $CH_3$ | 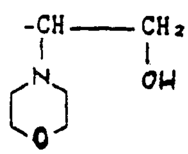 | " | N | 1 |
| 98 | $CH_3$ | 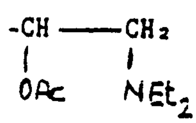 | " | N | 1 |
| 99 | $CH_3$ | 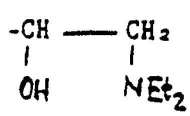 | " | N | 1 |

| Nr. | R1 | R2* | R4 | X | n |
|---|---|---|---|---|---|
| 100 | $CH_3$ | 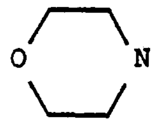 - CO | 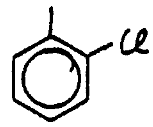 | N | 2 |
| 101 | - | 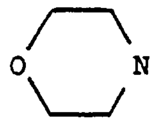 - CO | " | N | 2 |
| 102 | $CH_3$ | 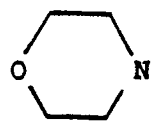 - CO | " | N | O |
| 103 | $CH_3$ | $H_2N$-CO | " | N | O |
| 104 | $CH_3$ | $(CH_3CH_2)_2NCO$ | " | N | 2 |
| 105 | $CH_3$ | 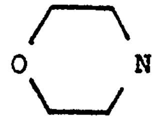 - CO | " | N | 1 |

| Nr. | R1 | R2* | R4 | X | n |
|---|---|---|---|---|---|
| 106 | $CH_3$ | 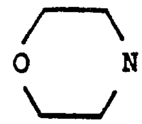 - CO | 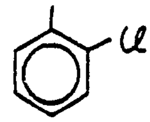 | N | 3 |
| 107 | $CH_3$ | 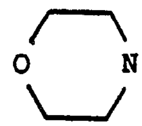 - CO | " | N | 4 |
| 108 | Br | 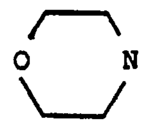 - CO | " | " | 2 |
| 109 | $CH_3O$ | 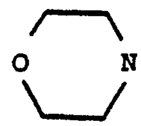 - CO | " | " | 2 |
| 110 | - | 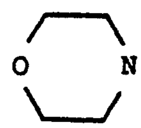 - CO | " | N | 8 |
| 111 | $CH_3$ | $CH_3NH-CO$ | " | N | 2 |

| Nr. | R1 | R2* | R4 | X | n |
|---|---|---|---|---|---|
| 112 | $CH_3$ | isoPrHN-CO | 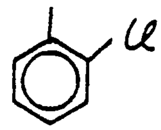 | N | 2 |
| 113 | $CH_3$ | $(CH_3)_2N$-CO | " | N | 2 |
| 114 | $CH_3$ | 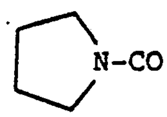 | " | N | 2 |
| 115 | $CH_3$ | 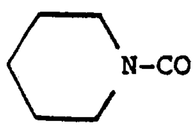 | " | N | 2 |
| 116 | $CH_3$ | 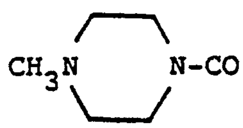 | " | N | 2 |
| 117 | 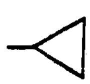 | $(C_2H_5)_2N$-CO | " | N | 2 |
| 118 | H | 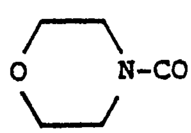 | " | N | 2 |

| Nr. | $R_1$ | $R_2$* | $R_4$ | X | n |
|---|---|---|---|---|---|
| 119 | $CH_3$ | $NH_2$-CO | 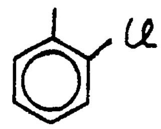 | N | 1 |
| 120 | $CH_3$ | $(HOCH_2CH_2)_2NCO$ | " | N | 2 |
| 121 | $CH_3$ | 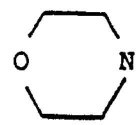 - CO | $C_6H_5$ | N | 2 |
| 122 | $CH_3$ | $HO(CH_2)_2N$-CO | 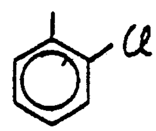 | N | 2 |
| 123 | $ClCH_2$ | 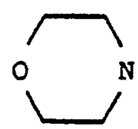 - CO | " | N | 2 |
| 124 | $BrCH_2$ | 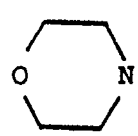 - CO | " | N | 2 |
| 125 | n-$C_3H_7O$ | 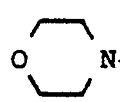-CO | " | N | 2 |

| Nr. | R1 | R2* | R4 | X | n |
|---|---|---|---|---|---|
| 126 | $CH_3$ | 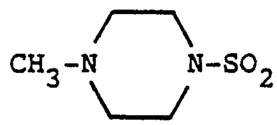 | 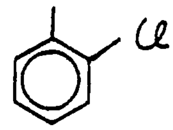 | N | 2 |
| 127 | $CH_3$ | $CH_3-C(=O)-O-$ | " | N | 3 |
| 128 | $CH_3$ | HO | " | N | 3 |
| 129 | $CH_3$ | $CH_3-C(=O)-O$ | " | N | 7 |
| 130 | $CH_3$ | $CH_3-CH(CH_3)-C(=O)-O$ | " | N | 3 |
| 131 | $CH_3$ | $CH_3SO_2-O-$ | " | N | 3 |

| Nr. | $R_1$ | $R_2$* | $R_4$ | X | n |
|---|---|---|---|---|---|
| 132 | $CH_3$ | J | 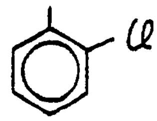 | N | 3 |
| 133 | $CH_3$ | 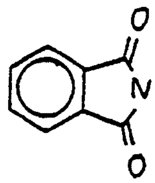 | " | N | 7 |
| 134 | $CH_3$ | $H_2N$ | " | N | 7 |
| 135 | $CH_3$ | 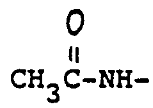 | | N | 7 |
| 136 | $CH_3$ | 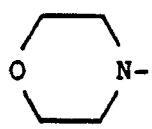 | " | N | 3 |
| 137 | $CH_3$ | HCO- | " | N | 6 |

| Nr. | $R_1$ | $R_2$ * | $R_4$ | X | n |
|---|---|---|---|---|---|
| 138 | $CH_3$ | $CH_3$-COO | 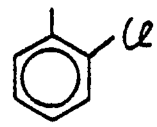 | N | 3 |
| 139 | $CH_3$ | HO- | " | CH | 3 |
| 140 | $CH_3$ | 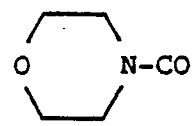 | " | CH | 2 |
| 141 | $CH_3$ | $(C_2H_5)_2NCO$ | " | CH | 2 |
| 142 | H | 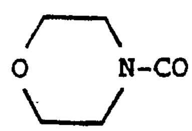 | " | CH | 2 |
| 143 | $CH_3$ | 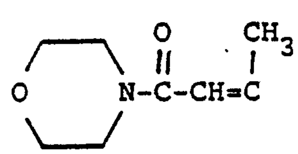 | " | N | O |

| Nr. | $R_1$ | $R_2$* | $R_4$ | X | n |
|---|---|---|---|---|---|
| 144 | $CH_3$ | 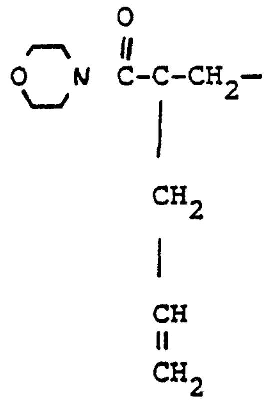 | 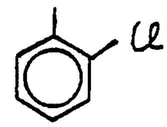 | N | O |
| 145 | $CH_3$ | 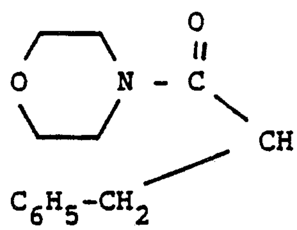 | | N | 1 |
| 146 | $CH_3$ | 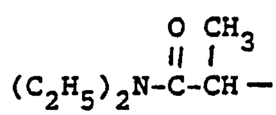 | | N | 1 |
| 147 | $CH_3$ | 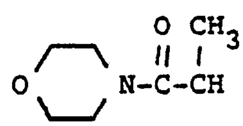 | | N | 1 |

| Nr. | $R_1$ | $R_2$* | $R_4$ | X | n |
|---|---|---|---|---|---|
| 148 | $CH_3$ | 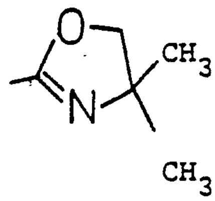 | 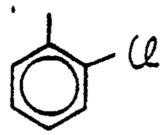 | N | 2 |
| 149 | $CH_3$ | 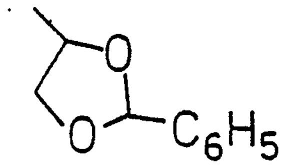 | " | N | 1 |
| 150 | $CH_3$ | 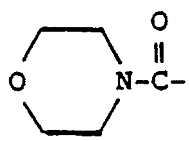 | " | N | 2 |
| 150a | $CH_3$ | 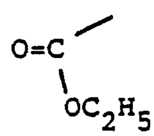 | " | N | 2 |

Bei der in den Beispielen angegebenen Numerierung wurden die folgenden Strukturen zugrunde gelegt:

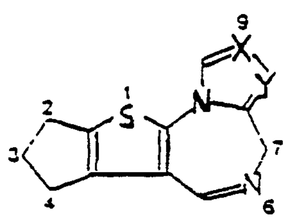

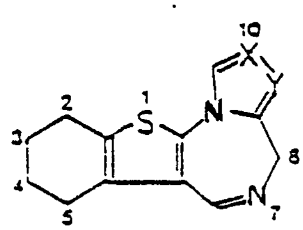

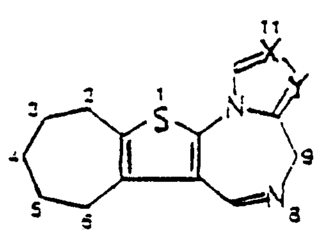

Nach den vorstehend beschriebenen Verfahren werden die folgenden Verbindungen zugänglich:

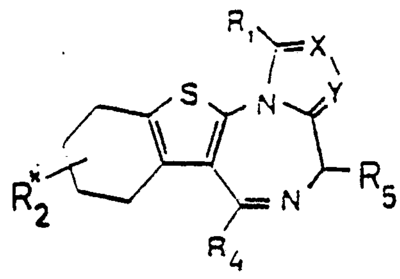

A

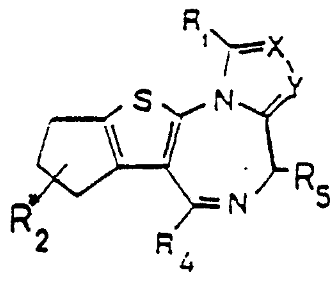

B

soweit nichts anderes angegeben, bedeuten X und Y beide Stickstoff und $R_4$ = o-Chlorphenyl und $R_5$ in der Bedeutung von Methyl-, Hydroxy wie auch Hydroxymethyl

| Bsp. | $R_2^x$ | $R_1$ | Type | Pos. $R_2$ |
|---|---|---|---|---|
| 151 | $-CO-N(C_2H_5)_2$ | $CH_3$ | A | 3 |
| 152 | $-CO-N(C_2H_5)_2$ | $CH_3$ | A | 4 |
| 153 | 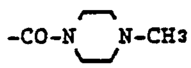 | $CH_3$ | A | 4 |
| 154 | 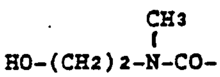 | $CH_3$ | A | 4 |
| 155 | 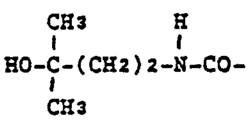 | $CH_3$ | A | 4 |
| 156 | 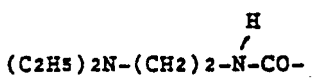 | $CH_3$ | A | 4 |
| 157 | 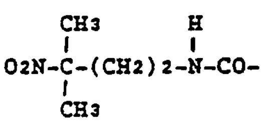 | $CH_3$ | A | 3 |
| 158 | 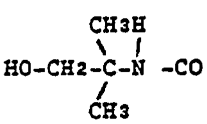 | $CH_3$ | A | 4 |

| Bsp. | $R_2^*$ | $R_1$ | Type | Pos. $R_2$ | |
|---|---|---|---|---|---|
| 159 | 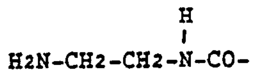 | $CH_3$ | A | 4 | 1 |
| 160 | $H_2N-CO-$ | $CH_3$ | A | 4 | |
| 161 | 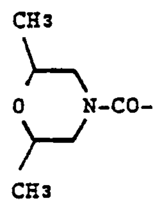 | $CH_3$ | A | 4 | |
| 162 | 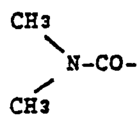 | $CH_3$ | B | 4 | |
| 163 | 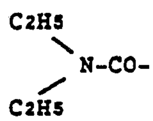 | $CH_3$ | B | 4 | |
| 164 | 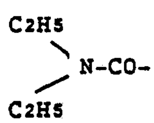 | $CH_3$ | B | 3 | |
| 165 | 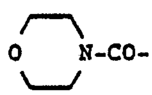 | $-OCH_3$ | B | 3 | |
| 166 | 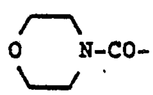 | $-OCH_3$ | B | 4 | |
| 167 | 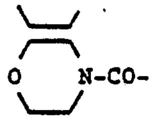 | $-OCH_3$ | A | 4 | |

| Bsp. | $R_2^*$ | $R_1$ | Type | Pos. $R_2$ |
|---|---|---|---|---|
| 168 | 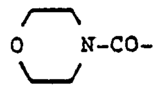 | H | A | 4 |
| 169 | 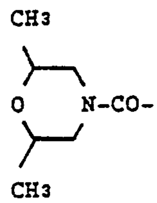 | $-OCH_3$ | B | 3 |
| 170 | 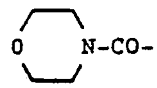 | H | B | 4 |
| 171 | 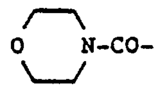 | Br | B | 4 |
| 172 | 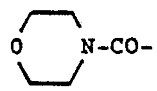 | H | B | 3 |
| 173 | 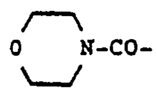 | Br | B | 3 |
| 174 | 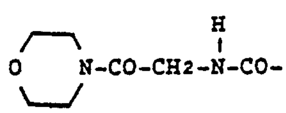 | $CH_3$ | B | 3 |
| 175 | 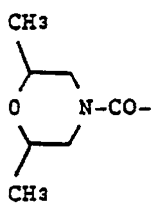 | $CH_3$ | B | 3 |
| 176 | 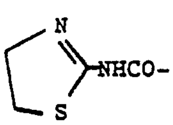 | $CH_3$ | A | 4 |

| Bsp. $R_2$* | $R_1$ | Type | Pos. $R_2$ |
|---|---|---|---|
| 177 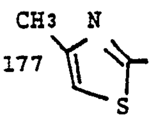 NHCO- | $CH_3$ | A | 4 |
| 178 $-CH_2-O-\underset{\underset{O}{\|}}{C}-CH_3$ | $CH_3$ | A | 4 |
| 179 $-CH_2-$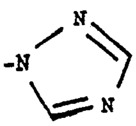 | $CH_3$ | A | 4 |
| | | X=CH | |
| 180 $-CH_2-OH$ | $CH_3$ | A | 4 |
| 181 $-CH_2-OH$ | $CH_3$ | B | 3 |
| 182 $-CH_2-$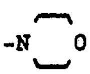 | $CH_3$ | B | 3 |
| 183 $-CH_2-OSO_2-CH_3$ | $CH_3$ | B | 3 |
| 184 $-CH_2-O-\underset{\underset{O}{\|}}{C}-CH_3$ | -H | B | 3 |
| 185 $-CH_2-OSO_2CH_3$ | -H | B | 3 |

| Bsp. | $R_2$* | $R_1$ | Type | Pos. $R_2$ |
|---|---|---|---|---|
| 186 | 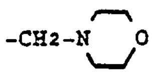 | -H | B | 3 |
| 187 | 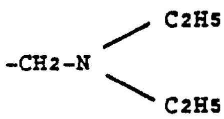 | -H | B | 3 |
| 188 | 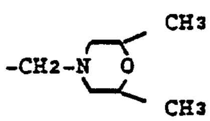 | $-CH_3$ | B | 3 |
| 189 | 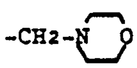 | -Br | B | 3 |
| 190 | 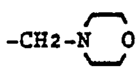 | $-OCH_3$ | B | 3 |
| 191 | 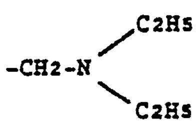 | Br | B | 3 |
| 192 | 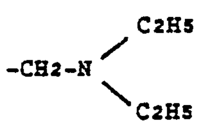 | $OCH_3$ | B | 3 |
| 193 | 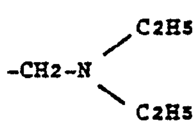 | $CH_3$ | B | 3 |

| Bsp. | $R_2$* | $R_1$ | Type | Pos. $R_2$ |
|---|---|---|---|---|
| 194 | 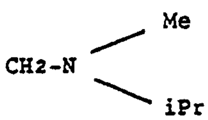 | $CH_3$ | B | 3 |
| 195 | 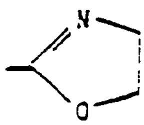 | $CH_3$ | A | 4 |
| 196 | 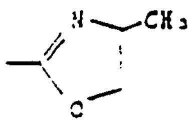 | $CH_3$ | A | 4 |
| 197 | 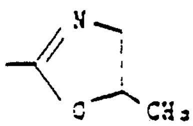 | $CH_3$ | A | 4 |
| 198 | 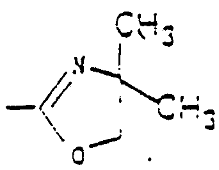 | $CH_3$ | B | 3 |
| 199 | 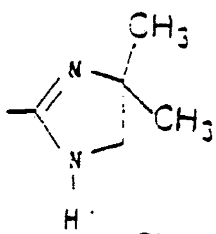 | $CH_3$ | A | 4 |
| 200 | 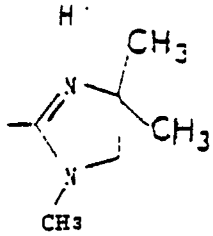 | $CH_3$ | A | 4 |

| Bsp. | $R_2$* | $R_1$ | Type | Pos. $R_2$* |
|---|---|---|---|---|
| 201 | 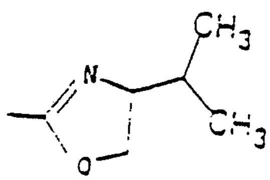 | CH3 | A | 4 |

"Azaverbindungen"

202
3-Acetylaminomethylcarbonyl-6-(2-chlorphenyl)-8,11-dimethyl-2,3,4,5-tetrahydro-8H-pyrido-[4′,3′:4,5]-thieno-[3,2-f][1,2,4]triazolo[4,3-a] [1,4]diazepin

203
3-(N-Morpholinocarbonylmethyl)-6-(2-chlorphenyl)-8,11-dimethyl-2,3,4,5-tetrahydro-8H-pyrido-[4′,3′:4,5]-thieno[3,2-f][1,2,4]triazolo[4,3-a] [1,4]diazepin

204
3-Acetylaminomethylcarbonyl-6-(2-chlorphenyl)-8-hydroxy-11-methyl-2,3,4,5-tetrahydro-8H-pyrido-[4′,3′:4,5]-thieno[3,2-f][1,2,4]triazolo[4,3-a] [1,4]diazepin

205
3-(N-Morpholinocarbonylmethyl)-6-(2-chlorphenyl)-8-hydroxy-11-methyl-2,3,4,5-tetrahydro-8H-pyrido-[4′,3′:4,5]-thieno[3,2-f][1,2,4]triazolo[4,3-a] [1,4]diazepin

| Bsp. | $R_2$* | $R_1$ | Type | Pos. $R_2$* |
|---|---|---|---|---|
| 206 | 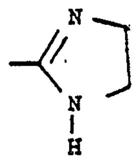 | CH3 | A | 4 |
| 207 | 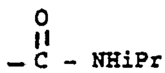 | CH3 | A | 4 |
| 208 | 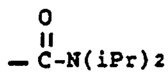 | CH3 | A | 4 |
| 209 | 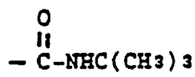 | CH3 | A | 4 |
| 210 | 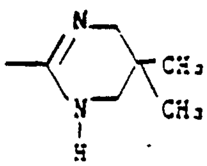 | CH3 | A | 4 |
| 211 | 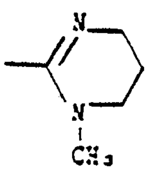 | CH3 | A | 4 |
| 212 | 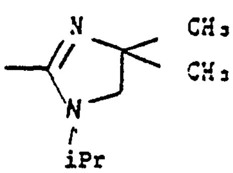 | CH3 | A | 4 |

| Bsp. | $R_2$* | $R_1$ | Type | Pos. $R_2$* |
|---|---|---|---|---|
| 213 | 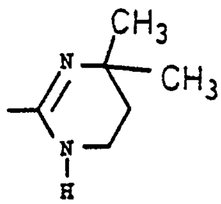 | CH3 | A | 4 |
| 214 | 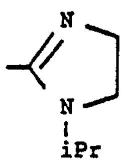 | CH3 | A | 4 |
| 215 | 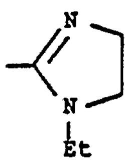 | CH3 | A | 4 |
| 216 | 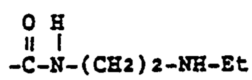 | CH3 | A | |
| 217 | 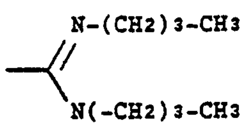 | CH3 | A | 4 |
| 218 | 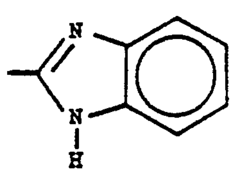 | CH3 | A | 4 |

| Bsp. | $R_2$* | $R_1$ | Type | Pos. $R_2$* |
|---|---|---|---|---|
| 219 | 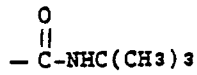 | $CH_3$ | A x=CH | 4 |
| 220 | 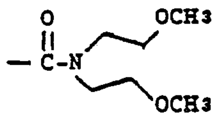 | $CH_3$ | A X=CH | 4 |
| 221 | 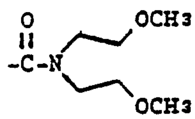 | $CH_3$ | A | 4 |
| 222 | 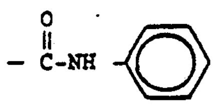 | $CH_3$ | A | 4 |
| 223 | $-CH_2-NH-Et$ | $CH_3$ | B | 3 |
| 224 | 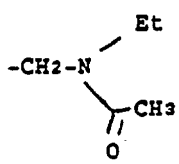 | $CH_3$ | B | 3 |
| 225 | $-CH_2-NH-CH_2-CH_2-NMe_2$ 2HCl | $CH_3$ | B | 3 |
| 226 | 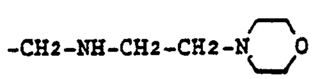 | $CH_3$ | B | 3 |

| Bsp. | $R_2$* | $R_1$ | Type | Pos. $R_2$* |
|---|---|---|---|---|
| 227 | 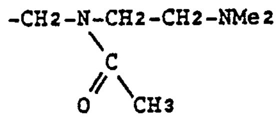 | $CH_3$ | B | 3 |
| 228 | 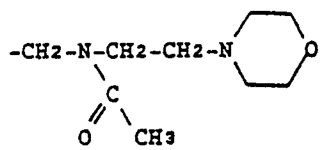 | $CH_3$ | B | 3 |
| 229 | 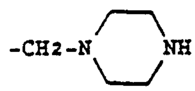 | $CH_3$ | B | 3 |
| 230 | 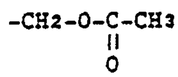 | $CH_3$ | B X=CH | 3 |
| 231 | 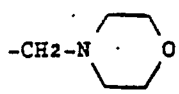 | $CH_3$ | B X=CH | 3 |
| 232 | 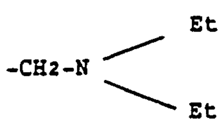 | $CH_3$ | B X=CH | 3 |
| 233 | $-CH_2-NH-(CH_2)_2$ 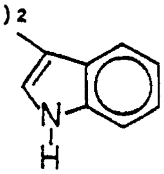 | $CH_3$ | B | 3 |
| 234 | $-CH_2-OCH_3$ | $CH_3$ | B | 3 |

| Bsp. | $R_2$* | $R_1$ | Type | Pos. $R_2$* |
|---|---|---|---|---|
| 235 | -CH2-O 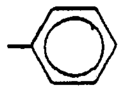 | CH3 | B | 3 |
| 236 | -COOCH3 $R_4$=C6H5 | CH3 | B | 3 |
| 237 | -CH2-OH $R_4$=C6H5 | CH3 | B | 3 |
| 238 | -CH2- 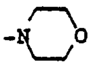 R =C6H5 | CH3 | B | 3 |
| 239 | -CH2-O 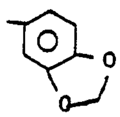 | CH3 | B | 3 |
| 240 | -CH2-O 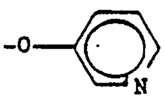 | CH3 | B | 3 |
| 241 | -CH2- 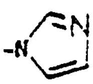 | CH3 | B | 3 |
| 242 | -CH2- 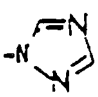 | CH3 | B | 3 |
| 243 | -CH2-S 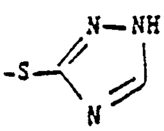 | CH3 | B | 3 |
| 244 | -CH2-Br | CH3 | B | 3 |
| 245 | -CH2-CN | CH3 | B | 3 |
| 246 | -OH | CH3 | A | 3 |

| Bsp. | $R_2$* | $R_1$ | Type | Pos. $R_2$* |
|---|---|---|---|---|
| 247 | 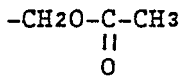 | $CH_3$ | A | 3 |
| 248 | 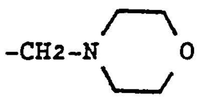 | $CH_3$ | A | 3 |
| 249 | $-CH_2-OH$ | $CH_3$ | A | 3 |
| 250 | $-CON(C_3H_7)_2$ | $CH_3$ | A | 4 |
| 251 | $-CON(CH_2-CH=CH_2)_2$ | $CH_3$ | A X=CH | 4 |
| 252 | $-CON(C_3H_7)_2$ | $CH_3$ | A X=CH | 4 |
| 253 | $-CON(CH_2-CH=CH_2)_2$ | $CH_3$ | A | 4 |
| 254 | 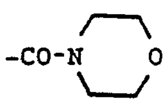 | $CH_2Cl$ | A | 4 |
| 255 | 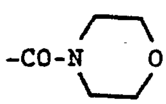 | $CH_2OH$ | A | 4 |
| 256 | $-CO-N(CH_2-CH=CH_2)_2$ | $CH_3$ | B | 3 |

| Bsp. | $R_2$* | $R_1$ | Type | Pos. $R_2$* |
|---|---|---|---|---|
| 257 | $-CON[(CH_2)_7CH_3]_2$ | $CH_3$ | B | 3 |
| 258 | $-CON(C_3H_7)_2$ | $CH_3$ | B | 3 |
| 259 | $-CON(C_3H_7)_2$ | $CH_3$ | B X=CH | 3 |
| 260 | $-CON(CH_2-CH=CH_2)_2$ | $CH_3$ | B X=CH | 3 |
| 261 | -CO-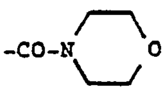 | $CH_3$ | B X=CH | 3 |
| 262 | -CO-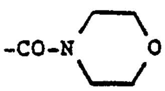 | Br | B X=CH | 3 |
| 263 | -CO-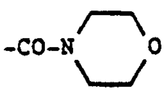 | H | B X= C-$CH_3$ | 3 |
| 264 | -CO-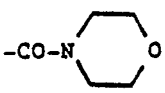 | $CH_3$ | B X=CH | 3 |
| 265 | $-CO-N(H)-(CH_2)_{15}-CH_3$ | $CH_3$ | B | 3 |

| Bsp. | $R_2$* | $R_1$ | Type | Pos. $R_2$* |
|---|---|---|---|---|
| 266 | $-CH_2-COOH$ | $CH_3$ | B | 3 |
| 267 | 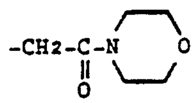 | $CH_3$ | B | 3 |
| 268 | $-CH_2-CH_2-OH$ | $CH_3$ | B | 3 |
| 269 | $-CH_2-CH_2-O-SO_2CH_3$ | $CH_3$ | B | 3 |
| 270 | 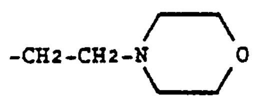 | $CH_3$ | B | 3 |
| 271 | 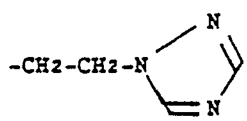 | $CH_3$ | B | 3 |
| 272 | $-CH_2-COOMe$ | $CH_3$ | B | 3 |
| 273 | $-CH_2-NH-n-C_{18}H_{37}$ | $CH_3$ | B | 3 |
| 274 | $-CH_2-O-C-n-C_{17}H_{35}$<br>O | $CH_3$ | B | 3 |
| 275 | $-CH_2-O-n-C_{18}H_{37}$ | $CH_3$ | B | 3 |
| 276 | $CH_2-OH$ | $CH_3$ | B<br>X=CH | 3 |
| 277 | $-CH_2-O-SO_2CH_3$ | $CH_3$ | B<br>X=CH | 3 |
| 278 | 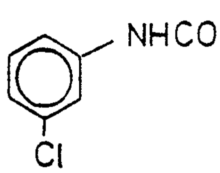 | $CH_3$ | B | 3 |

| Bsp. $R_2$* | $R_1$ | Type | Pos. $R_2$* |
|---|---|---|---|
| 279 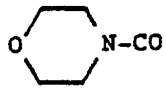 | $CH_3$ | B | 4 |
| 280 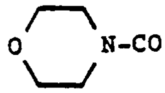 | $CH_3$ | B | 3 |
| 281 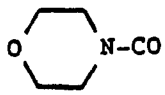 | 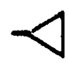 | B | 4 |
| 282 t-BuNHCO | $CH_3$ | B | 4 |
| 283 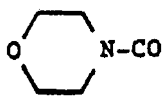 | H | B | 3 |
| 284 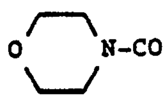 | Br | B | 3 |
| 285 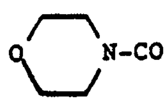 | $OCH_3$ | B | 3 |
| 286 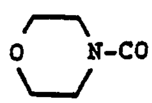 | $CH_3$ | A | 3 |

| Bsp. | $R_2$* | $R_1$ | Type | Pos. $R_2$* |
|---|---|---|---|---|
| 287 | $nC_{16}H_{33}NH-CO$ | $CH_3$ | A | 3 |
| 288 | $(C_8H_{17})N-CO$ | $CH_3$ | A | 3 |
| 289 | $(n-C_3H_7)_2NCO$ | $CH_3$ | A | 3 |
| 290 | 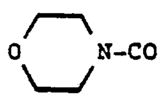 | $CH_3$ | A | 4 |
| 291 | 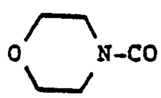 | $CH_3$ | C | 5 |
| 292 | 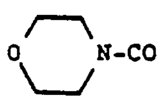 | $CH_3$ | B | 4 |
| 293 | COOH | $CH_3$ | A | 3 |
| 294 | $nC_{16}H_{33}NHCO$ | $CH_3$ | A | 3 |
| 295 | $(C_2H_5)_2NCO$ | $CH_3$ | A | 4 |

| Bsp. | $R_2$* | $R_1$ | Type | Pos. $R_2$* |
|---|---|---|---|---|
| 296 | $(C_2H_5)_2NCO$ | $CH_3$ | B | 4 |
| 297 | 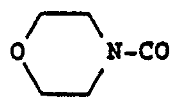 | Br | A<br>$X=C-CH_3$ | 3 |
| 298 | COOH | H | A<br>$X=C-CH_3$ | 3 |
| 299 | COOH | H | A | 3 |
| 300 | =O | $CH_3$ | B | 2 |
| 301 | OH | $CH_3$ | B | 2 |
| 302 | $HOCH_2$- | $CH_3$ | B | 4 |

| Bsp. | $R_2$* | $R_1$ | Type | Pos. $R_2$* |
|---|---|---|---|---|
| 303 | $HOCH_2$– | H | A | 3 |
| 304 | $CH_3COOCH_2$- | $CH_3$ | A | 3 |
| 305 | $CH_3SO_2OCH_2$– | $CH_3$ | B | 4 |
| 306 | 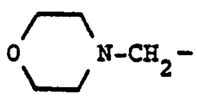 | $CH_3$ | B | 4 |
| 307 | 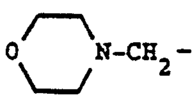 | $CH_3$ | A | 3 |
| 308 | 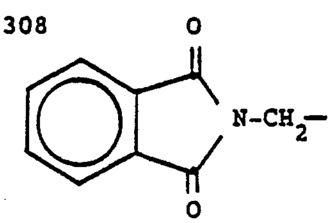 | $CH_3$ | A | 3 |
| 309 | $H_2NCH_2$– | $CH_3$ | A | 3 |

| Bsp. $R_2$* | $R_1$ | Type | Pos. $R_2$* |
|---|---|---|---|
| 310 $H_2NCH_2$ | $CH_3$ | B | 4 |
| 311 $CH_3CONHCH_2$ | $CH_3$ | A | 3 |
| 312 $C_2H_5OCO$ | $CH_3$ | B | 4 |
| 313 $CH_3OCO$ | $CH_3$ | A X=CH | A |
| 314 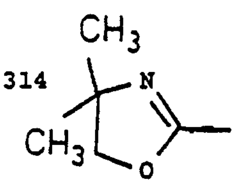 | H | B | 4 |
| 315 CN | $CH_3$ | B | 4 |
| 316 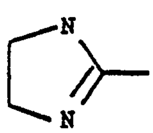 | $CH_3$ | B | 4 |

| Bsp. $R_2$* | | $R_1$ | Type | Pos. $R_2$* |
|---|---|---|---|---|
| 317 | 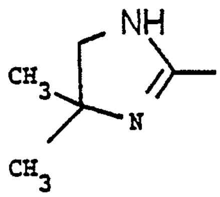 | $CH_3$ | B | 4 |
| 318 | 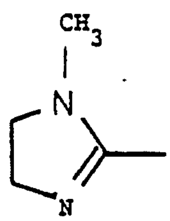 | $CH_3$ | B | 4 |
| 319 | $H_2N$ | $CH_3$ | B | 4 |
| 320 | $CH_3OCONH-$ | $CH_3$ | B | 4 |

Beispiel 321

3-Thioacetyl-6-(2-chlorphenyl)-8,11-dimethyl-2,3,4,5-tetrahydro-8H-pyrido-[4', 3': 4,5][3,2-f][1,2,4]-triazolo-[4,3-a][1,4]diazepin

Beispiel 322

6-(2-Chlorphenyl)-8,11-dimethyl-2,3,4-tetrahydro-8-H-pyrido[4', 3': 4,5][3,2-f][1,2,4]-triazolo[4,3-a][1,4]-diazepin

Beispiel 323

3-(Carboethoxymethyl)-6-(2-chlorphenyl)-8,11-dimethyl-2,3,4,5-tetrahydro-8-H-pyrido[4',3': 4,5]thieno[3,2-f]-[1,2,4]-triazolo[4,3-a][1,4]-diazepin

Beispiel 324

3-Thioacetyl-6-(2-chlorphenyl)-8-hydroxy-11-methyl-2,3,4,5-tetrahydro-8H-pyrido-[4′,3′:4,5]thieno[3,2-f]-[1,2,4]-triazolo[4,3-a][1,4]dioazepin

Beispiel 325

6-(2-Chlorphenyl)-8-hydroxy-11-methyl-2,3,4,5-tetrahydro-8-H-pyrido-[4′,3′:4,5]thieno[3,2-f]1,2,4]triazolo[4,3-a]diazepin

Beispiel 326

3-(Carboethoxymethyl)-6-(2-chlorphenyl)-8-hydroxy-11-methyl-2,3,4,5-tetrahydro-8-H-pyrido[4′,3′:4,5]thieno-[3,2-f][1,2,4]triazolo[4,3-a][1,4]diazepin

Beispiel 327

3-Thioacetyl-6-(2-chlorphenyl)-8-hydroxymethyl-11-methyl-2,3,4,5-tetrahydro-8H-pyrido-[4′,3′:4,5]thieno[3,2-f][1,2,4]triazolo[4,3-a][1,4]diazepin

Beispiel 328

6-(2-Chlorphenyl)-8-hydroxymethyl-11-methyl-2,3,4,5-tetrahydro-8-H-pyrido-[4′,3′:4,5]thieno[3,2-f][1,2,4]-triazolo[4,3-a][1,4]diazepin

Beispiel 329

3-(Carbethoxymethyl)-6-(2-chlorphenyl)-8-hydroxymethyl-11-methyl-2,3,4,5-tetrahydro-8-H-pyrido[4′,3′:4,5]-thieno[3,2-f][1,2,4]triazolo[4,3-a][1,4]diazepin

Beispiel 330

3-Acetylaminomethylcarbonyl-6-(2-chlorphenyl)-8-hydroxymethyl-11-methyl-2,3,4,5-tetrahydro-8H-pyrido-[4′,3′:4,5]thieno[3,2-f][1,2,4]triazolo[4,3-a][1,4]diazepin

Beispiel 331

3-(N-Morpholinocarbonylmethyl)-6-(2-chlorphenyl)-8-hydroxymethyl-methyl-2,3,4,5-tetrahydro-8H-pyrido-[4′,3′:4,5]-thieno[3,2-f][1,2,4]triazolo[4,3-a][1,4]diazepin

Tabletten

1. Die Tablette enthält folgende Bestandteile:

| | |
|---|---|
| Wirkstoff gemäß Formel Ia/Ib | 0,020 Teile |
| Stearinsäure | 0,010 " |
| Dextrose | 1,890 " |
| gesamt | 1,920 Teile |

Herstellung:

Die Stoffe werden in bekannter Weise zusammengemischt und die Mischung zu Tabletten verpreßt, von denen jede 1,92 g wiegt und 20 mg Wirkstoff enthält.

2. Salbe

Die Salbe setzt sich aus folgenden Bestandteilen zusammen:

| | |
|---|---|
| Wirkstoff gemäß Formel Ia/Ib | 50 mg |
| Neribas Salbe (Handelsware Scherax) | ad 10 g |

Herstellung:

Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

| 3. Creme | |
|---|---|
| Zusammensetzung: | |
| Wirkstoff gemäß Formel Ia/Ib | 50 mg |
| Neribas Salbe (Handelsware Scherax) | ad 10 mg |

Herstellung

Der Wirkstoff wird mit 0,5 g Cremegrundlage verrieben und die restliche Grundlage in Teilmengen zu 1,0 g nach und nach mit Pistill eingearbeitet. Man erhält eine 0,5%ige Creme. Die Verteilung des Wirkstoffes in der Grundlage wird optisch unter dem Mikroskop kontrolliert.

| 4. Ampullenlösung | |
|---|---|
| Zusammensetzung: | |
| Wirkstoff gemäß Formel Ia/Ib | 1,0 mg |
| Natriumchlorid | 45,0 mg |
| Aqua pro inj. | ad 5,0 ml |

Herstellung:

Der Wirkstoff wird bei Eigen-pH oder gegebenenfalls bei pH 5,5 bis 6,5 in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 10 mg Wirkstoff.

| 5. Suppositorien | |
|---|---|
| Jedes Zäpfchen enthält: | |
| Wirkstoff gemäß Formel Ia/Ib | 1,0 Teile |
| Kakaobutter (Fp.36-37° C) | 1200,0 Teile |
| Carnaubawachs | 5,0 Teile |

Herstellung

Kakaobutter und Carnaubawachs werden zusammengeschmolzen. Bei 45° C gibt man den Wirkstoff hinzu und rührt, bis eine komplette Dispersion entstanden ist.

Die Mischung wird in Formen entsprechender Größe gegossen und die Zäpfchen zweckmäßig verpackt.

| 6. Inhalationslösungen | | |
|---|---|---|
| Zusammensetzung: | | |
| (a) | Wirkstoff gemäß Formel Ia/Ib<br>Na-EDTA<br>Benzalkoniumchlorid<br>Natriumchlorid<br>destilliertes Wasser | 500 mg<br>50 mg<br>25 mg<br>880 mg<br>ad 100 ml |

Herstellung:

96 % der Wassermenge werden vorgelegt, darin nacheinander Na-EDTA, Benzalkoniumchlorid, Natriumchlorid und Wirkstoff klar gelöst und mit dem restlichen Wasser aufgefüllt. Die Lösung wird in 20 ml-Tropfflaschen abgefüllt. Eine Dosis (20 Tropfen, 1 ml) enthält 5 mg Wirkstoff.

| | | |
|---|---|---|
| (b) | Wirkstoff gemäß Formel Ia/Ib<br>Natriumchlorid<br>destilliertes Wasser | 500 mg<br>820 mg<br>ad 100 ml |

Herstellung

96 % der Wassermenge werden vorgelegt, darin nacheinander der Wirkstoff und Natriumchlorid gelöst, mit dem restlichen Wasser aufgefüllt und die Lösung in Eindosenbehälter (4 ml) abgefüllt. Die Lösung enthält 20 mg Wirkstoff.

**Ansprüche**

1. Neue Hetrazepine der allgemeinen Formel Ia, Ib

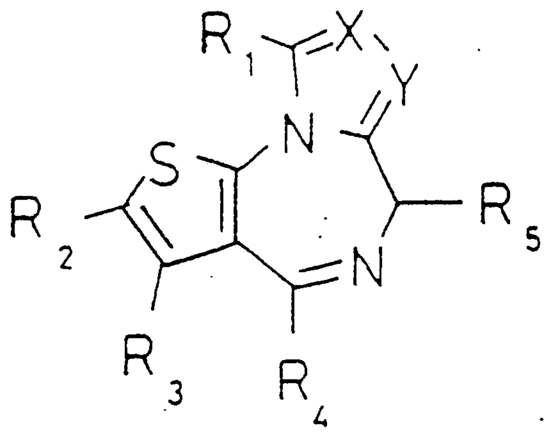

Ia

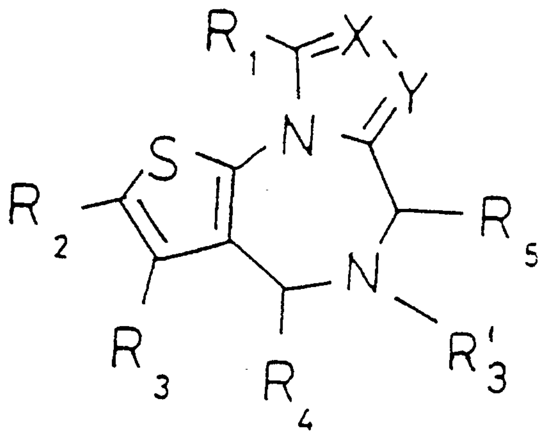

Ib

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, eine Cyclopropyl-, eine Cyclobutyl-, Cyclopentylgruppe, eine verzweigte oder unverzweigte Alkoxygruppe mit 1 bis 4 Kohlenstoffatomen, bevorzugt Methoxy, Halogen, bevorzugt Chlor oder Brom;

$R_2$ den Rest $R_a$-$Z_n$-

worin

für n > O

$R_a$ Halogen, Hydroxy, Alkenyl,

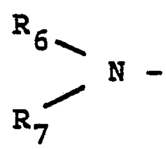

,

wobei $R_6$ und $R_7$, die gleich oder verschieden sein können, Wasserstoff, eine verzweigte oder unverzweigte Alkylgruppe mit 1 -18 Kohlenstoffatomen, bevorzugt 1 -6 Kohlenstoffatomen, eine verzweigte oder unverzweigte Alkenylgruppe mit 3 bis 18 Kohlenstoffatomen, bevorzugt 3 - 6 Kohlenstoffatomen, eine verzweigte oder unverzweigte Alkinylgruppe mit 3 bis 18 Kohlenstoffatomen, bevorzugt 3 bis 6 Kohlenstoffatomen, die gegebenenfalls durch Halogen, Hydroxy, Phenyl, substituiertes Phenyl, oder durch einen C-verknüpften Heterocyclus substituiert sein können, wobei die Kohlenstoffketten durch Stickstoff (auch NH), Sauerstoff oder Schwefel unterbrochen sein können, eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine Aminogruppe, die gegebenenfalls ein- oder zweifach durch eine verzweigte oder unverzweigte Alkylgruppe mit 1 bis 6 Kohlenstoffatomen substituiert sein ann, wobei die Alkylgruppe wiederum durch Halogen oder Hydroxy substituiert sein kann,

eine gegebenenfalls substituierte Arylcarbonylgruppe, bevorzugt Phenylcarbonyl oder Tolylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen, einen gegebenenfalls substituierten $C_3$-$C_7$-Cycloalkylrest, einen gegebenenfalls substituierten $C_5$-$C_7$-Cycloalkenylrest,

$R_6$ oder $R_7$ ein gegebenenfalls ein- oder mehrfach durch verzweigtes oder unverzweigtes Alkyl mit 1 bis 4

Kohlenstoffatomen substituierter, gesättigter oder ungesättigter über ein Kohlenstoffatom gebundener 5-, 6- oder 7-gliedriger heterocyclischer Ring,

oder

$R_6$ und $R_7$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_a$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatomen substituiert;

eine verzweigte oder unverzweigte Alkylsulfonyloxygruppe mit 1 bis 4 Kohlenstoffatomen;

eine Arylcarbonyloxygruppe, bevorzugt Phenylcarbonyloxy oder Tolylcarbonyloxy -gegebenenfalls ein- oder mehrfach durch verzweigte oder unverzweigte Alkyl- und/oder Alkoxygruppen mit 1 bis 4 Kohlenstoffatomen substituiert;

Ra eine verzweigte oder unverzweigte Alkylcarbonyloxygruppe mit 1 bis 12, bevorzugt 1 bis 8, Kohlenstoffatomen, wobei die Alkylkette durch Stickstoff, Sauerstoff oder Schwefel unterbrochen sein kann;

Ra 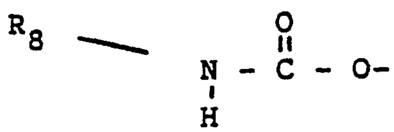 , 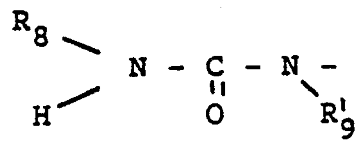

wobei $R_8$ eine verzweigte oder unverzweigte $C_1$-$C_{10}$-, bevorzugt $C_1$-$C_4$-Alkyl-, $C_3$-$C_{10}$-, bevorzugt $C_3$-$C_4$-Alkenyl- oder $C_3$-$C_{10}$-, bevorzugt $C_3$-$C_4$-Alkinylgruppe, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Arylgruppe, bevorzugt Phenyl, und $R_9'$ Wasserstoff oder eine verzweigte oder unverzweigte Alkylgruppe mit 1 bis 4 Kohlenstoffatomen;

Ra 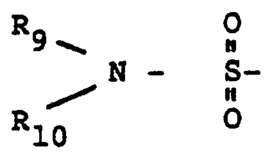

wobei $R_9$ und $R_{10}$, die gleich oder verschieden sein können, eine verzweigte oder unverzweigte Alkylgruppe mit 1 bis 18 Kohlenstoffatomen, bevorzugt 1 bis 6 einen gegebenenfalls substituierten $C_3$-$C_7$-Cycloalkylrest, einen gegebenenfalls substituierten $C_5$ - $C_7$-Cycloalkenylrest, $R_9$ oder $R_{10}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,

oder $R_9$ und $R_{10}$ zusammen mit dem Stickstoffatom einen gegebenenfalls ein- oder mehrfach durch verzweigte oder unverzweigte Alkylgruppen mit 1 bis 4 Kohlenstoffatomen substituierten gesättigten oder ungesättigten 5-, 6- oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatomen, bevorzugt Methyl, substituiert sein kann;

$R_a$ eine verzweigte oder unverzweigte Alkoxygruppe mit 1 bis 10, bevorzugt 1 bis 4 Kohlenstoffatomen; eine Aryloxygruppe, bevorzugt Phenyloxy oder substituiertes Phenyloxy;

$R_a$ einen C-verknüpften 5 bis 7-gliedrigen nicht aromatischen Heterocyclus;

$R_a$ einen Imidorest, einen Benzimidazoylrest, ein Imid;

für n größer gleich O

Ra -CH=0, COOH, Cyano, Wasserstoff,

einen Rest der allgemeinen Formel

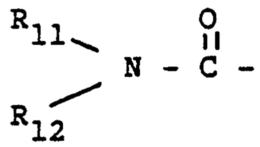

,

worin $R_{11}$ und $R_{12}$, die gleich oder verschieden sein können, Wasserstoff, Phenyl, substituiertes Phenyl, eine verzweigte oder unverzweigte Alkylgruppe mit 1 bis 18 Kohlenstoffatomen, bevorzugt 1 bis 6 Kohlenstoffatomen, eine verzweigte oder unverzweigte Alkenylgruppe mit 3 bis 18, bevorzugt 3 bis 6 Kohlenstoffatomen oder eine verzweigte oder unverzweigte Alkinylgruppe mit 3 bis 18, bevorzugt 3 bis 6 Kohlenstoffatomen, wobei die Alkyl-, Alkenyl- oder Alkinylgruppe gegebenenfalls durch Halogen, Hydroxy, Nitro, Amino, substituiertes Amino oder

im Fall von $R_{11}$ = Wasserstoff oder Alkyl und Y = C-$R_1$ oder Y Stickstoff und X C-Alkyl, $R_{12}$ durch eine Esterfunktion oder ein Säureamid der allgemeinen Formel

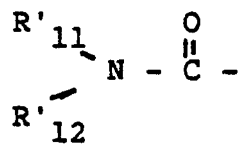

worin $R'_{11}$ und $R'_{12}$ dieselbe Bedeutung wie $R_{11}$ und $R_{12}$ - jedoch mit Ausnahme eines Säureamids, haben - substituiert sein kann,

$R_{11}$ oder $R_{12}$ einen gegebenenfalls substituierten $C_3$ bis $C_7$-Cycloalkylrest, einen gegebenenfalls substituierten $C_5$ bis $C_7$-Cycloalkenylrest,

oder

$R_{11}$ oder $R_{12}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,

$R_{11}$ und $R_{12}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6-oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann,

$R_a$ einen Rest der allgemeinen Formel

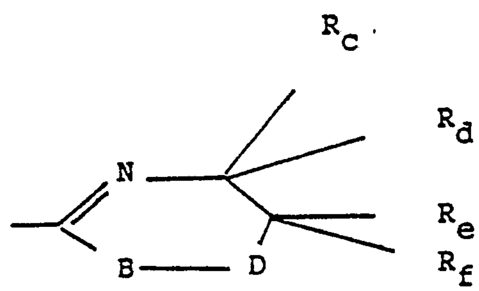

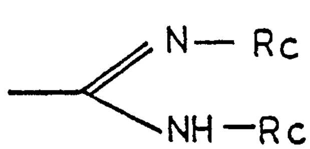

worin

B Sauerstoff, NH oder $NC_1$-$C_6$-Alkyl,

D den Rest $(CR_gR_h)_n$, wobei n 0 bis 3 sein kann,

$R_c$ Wasserstoff, gegebenenfalls durch eine Hydroxy- oder Aminogruppe substituiertes Alkyl mit 1 bis 6 Kohlenstoffatomen, $C_1$ bis $C_4$ Alkoxycarbonyl, Dialkylaminocarbonyl,

$R_d$, $R_e$, $R_f$, $R_g$, $R_h$ Wasserstoff, gegebenenfalls durch eine Hydroxy- oder Aminogruppe substituiertes Alkyl mit 1 bis 6 Kohlenstoffatomen, oder Phenyl;

$R_a$ Aryl, bevorzugt Phenyl oder substituiertes Phenyl, Naphthyl, substituiertes Naphthyl;

$R_3$ Wasserstoff, Phenyl, substituiertes Phenyl oder eine verzweigte oder unverzweigte Alkylgruppe mit 1 bis 4 Kohlenstoffatomen, bevorzugt Methyl;

Z eine verzweigte oder unverzweigte Alkyl-, Alkenyl-, oder Alkinylgruppe mit n Kohlenstoffatomen, wobei Z gegebenenfalls zusätzlich durch Aryl oder zusätzlich durch $R_2$ bevorzugt Ra substituiert sein kann;

n eine der Zahlen 0, 1, 2, 3, 4, 5, 6, 7, 8, 9 oder 10;

oder

$R_2$ und $R_3$ zusammen einen Rest der allgemeinen Formel

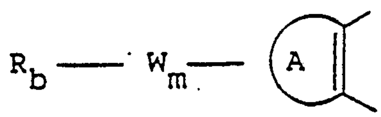

worin

A einen ankondensierten einfach ungesättigten 5-, 6-oder 7- gliedriger Ring, wobei im Fall von m = 0 und $R_b$ = Wasserstoff ein Kohlenstoffatom durch C=0 ersetzt werden kann,

oder A einen ankondensierten Ring der Formel

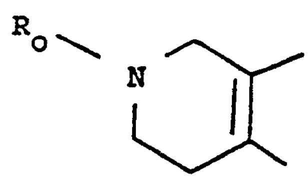

bedeutet,

wobei $R^0$ eine verzweigte oder unverzweigte Alkylgruppe mit 1 bis 18, bevorzugt mit 1 bis 4, Kohlenstoffatomen, eine Alkylcarbonyl- oder Alkylthiocarbonylgruppe mit 1 bis 18 bevorzugt 1 bis 4, Kohlenstoffatomen in der Alkylkette oder eine Arylcarbonyl- oder Arylthiocarbonylgruppe eine Alkoxycarbonylalkylgruppe mit bis zu 18, bevorzugt bis zu 8 Kohlenstoffatomen, besonders bevorzugt bis zu 4, eine Alkylcarbonylalkylgruppe mit bis zu 18, bevorzugt bis zu 8 Kohlenstoffatomen, besonders bevorzugt bis zu 4, eine Alkylcarbonylaminoalkylcarbonylgruppe mit bis zu 18, bevorzugt bis 10 Kohlenstoffatomen oder eine Aminocarbonylalkylgruppe mit bis zu 18, bevorzugt bis 4 Kohlenstoffatomen in der Alkylkette oder Wasserstoff,

Ro einen Rest der allgemeinen Formel

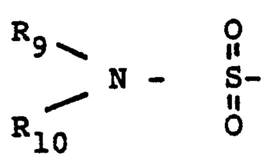

wobei $R_9$ und $R_{10}$, die gleich oder verschieden sein können, eine verzweigte oder unverzweigte Alkylgruppe mit 1 bis 18 Kohlenstoffatomen, bevorzugt 1 bis 6, einen gegebenenfalls substituierten $C_3$-$C_7$-Cycloalkylrest, einen gegebenenfalls substituierten $C_5$ - $C_7$-Cycloalkenylrest,

$R_9$ oder $R_{10}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,

oder $R_9$ und $R_{10}$ zusammen mit dem Stickstoffatom einen gegebenenfalls ein- oder mehrfach durch verzweigte oder unverzweigte Alkylgruppen mit 1 bis 4 Kohlenstoffatomen substituierten gesättigten oder ungesättigten 5-, 6- oder 7-Ring, der als weitere Heteroatome Stickstoff, Sauerstoff oder Schwefel enthalten

kann, wobei jedes weitere Stickstoffatom durch eine Alkylgruppe mit 1 bis 4 Kohlenstoffatomen, bevorzugt Methyl, substituiert sein kann;

W eine verzweigte oder unverzweigte Alkyl-, Alkenyl-, Alkinylgruppe mit m Kohlenstoffatomen;

m 0, 1, 2, 3, 4, 5 oder 6;

$R_b$ Wasserstoff, Hydroxy, Amino, Formyl, Carboxy, Cyano, verzweigtes oder unverzweigtes Alkyloxycarbonyl mit 1 bis 18, bevorzugt 1 bis 8, Kohlenstoffatomen, wobei die Alkylkette gegebenenfalls durch Hydroxy, Amino, Nitro oder Halogen substituiert sein kann, eine gegebenenfalls substituierte Aryloxycarbonylgruppe bevorzugt Phenyloxycarbonyl;

$R_b$ einen Rest der allgemeinen Formel

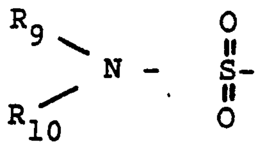

worin $R_9$ und $R_{10}$ wie zuvor definiert sind;

$R_b$ einen Rest der allgemeinen Formel

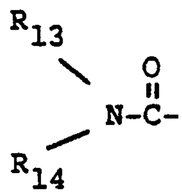

,

worin $R_{13}$ und $R_{14}$, die gleich oder verschieden sein können, Wasserstoff, Phenyl, subst. Phenyl, eine verzweigte oder unverzweigte Alkylgruppe mit 1 bis 18 Kohlenstoffatomen, bevorzugt mit 1 bis 6, besonders bevorzugt 1 bis 4, unverzweigte Alkenyl- mit 3 bis 18 Kohlenstoffatomen, bevorzugt 3 - 6 Kohlenstoffatomen, eine verzweigte oder unverzweigte Alkinylgruppe mit 3 bis 18 Kohlenstoffatomen, bevorzugt 3 bis 6 Kohlenstoffatomen, wobei die Alkyl-, Alkenyl- oder Alkinylgruppe gegebenenalls durch Halogen, Hydroxy, Nitro, Amino, substituiertes Amino, $C_1$ bis $C_6$ Alkoxy, bevorzugt Methoxy oder im Fall von $R_{13}$ = Wasserstoff oder Alkyl, durch eine Esterfunktion oder durch ein Säureamid der allgemeinen Formel

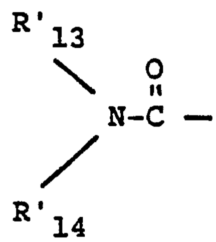

worin $R'_{13}$ und $R'_{14}$ dieselbe Bedeutung wie $R_{13}$ und $R_{14}$ jedoch mit Ausnahme eines Säureamids haben können, substituiert sein kann,

$R_{13}$ oder $R_{14}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring oder ein gegebenenfalls substituierter $C_3$ bis $C_7$ Cycloalkylrest, ein gegebenenfalls substituierter $C_5$ bis $C_7$ Cycloalkenylrest oder

$R_{13}$ und $R_{14}$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4

Kohlenstoffatomen, bevorzugt Methyl, substituiert sein kann;
$R_b$ einen Rest der allgemeinen Formel

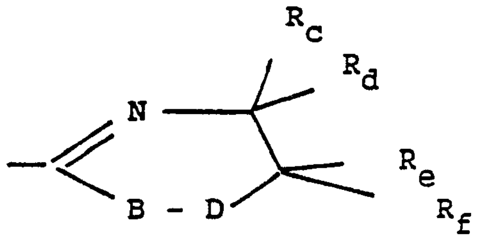

,

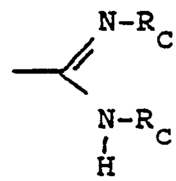

worin
B Sauerstoff, Schwefel, NH oder N-$C_1$-$C_6$-Alkyl
D den Rest $(CR_gR_h)_n$, wobei n 0 bis 3 sein kann,
$R_c$ Wasserstoff, gegebenenfalls durch eine Hydroxy- oder Aminogruppe substituiertes Alkyl mit 1 bis 6 Kohlenstoffatomen, $C_1$ bis $C_4$ Alkoxycarbonyl, $C_1$ bis $C_4$ Dialkylaminocarbonyl,
$R_d$, $R_e$, $R_f$, $R_g$, $R_h$ Wasserstoff, gegebenenfalls durch eine Hydroxy oder Aminogruppe substituiertes Alkyl mit 1 bis 6 Kohlenstoffatomen oder Phenyl;

$R_b$

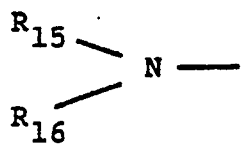

,

$R_{15}$ = Wasserstoff, $R_{16}$ = Wasserstoff, Alkylcarbonyl oder Alkoxycarbonyl mit 1 bis 18, bevorzugt 1 bis 6 Kohlenstoffatomen, Aminocarbonyl, Alkylaminocarbonyl, Dialkylaminocarbonyl mit 1 bis 18 bevorzugt 1 bis 6 Kohlenstoffatomen in der Alkylkette;
$R_b$ Wasserstoff, Aryl, bevorzugt Phenyl, substituiertes Phenyl, Naphthyl, substituiertes Naphthyl;
$R_b$ Halogen,

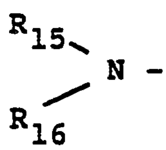

,

wobei $R_{15}$ und $R_{16}$, die gleich oder verschieden sein können, Wasserstoff, eine verzweigte oder unverzweigte Alkylgruppe mit 1 - 18 Kohlenstoffatomen, bevorzugt 1 bis 6 Kohlenstoffatomen, eine verzweigte oder unverzweigte Alkenylgruppe mit 3 bis 18, bevorzugt 3 bis 6 Kohlenstoffatomen, eine verzweigte oder unverzweigte Alkinylgruppe mit 3 bis 18, bevorzugt 3 bis 6 Kohlenstoffatomen, wobei die Alkyl-, Alkenyl- oder Alkinylgruppe gegebenenfalls durch Halogen, Hydroxy oder einen C-verknüpften Heterocyclus substituiert sein kann, wobei die Kohlenstoffkette der Alkyl-, Alkenyl- oder Alkinylgruppe durch Stickstoff (auch NH), Sauerstoff oder Schwefel unterbrochen sein kann,
eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann, eine gegebenenfalls substituierte Arylcarbonylgruppe, bevorzugt Phenylcarbonyl, eine gegebenenfalls substituierte Arylsulfonylgruppe, bevorzugt Phenylsulfonyl oder Tolylsulfonyl, eine Alkylsulfonylgruppe mit 1 bis 4 Kohlenstoffatomen,
$R_{15}$ oder $R_{16}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoff verknüpfter 5-, 6-, oder 7-gliedriger heterocyclischer Ring, ein gegebenenfalls substituierter $C_3$ bis $C_7$-Cycloalkylrest, ein

gegebenenfalls substituierter $C_5$ bis $C_7$ Cycloalkenylrest,
oder
$R_{15}$ und $R_{16}$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_b$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatomen substituiert;

$R_b$ eine verzweigte oder unverzweigte Alkylsulfonyloxygruppe mit 1 bis 4 Kohlenstoffatomen;

$R_b$ eine Arylcarbonyloxygruppe, bevorzugt Tolylcarbonyloxy oder Phenylcarbonyloxy, gegebenenfalls ein- oder mehrfach durch verzweigte oder unverzweigte Alkyl- und/oder Alkoxygruppen mit 1 bis 4 Kohlenstoffatomen substituiert;

$R_b$ eine verzweigte oder unverzweigte Alkylcarbonyloxygruppe mit 1 bis 18, bevorzugt 1 bis 8, Kohlenstoffatomen, wobei die Alkylkette durch Stickstoff, Sauerstoff oder Schwefel unterbrochen sein kann;

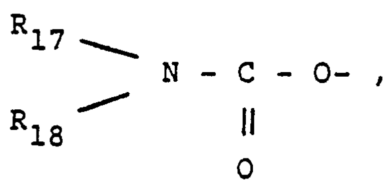 , 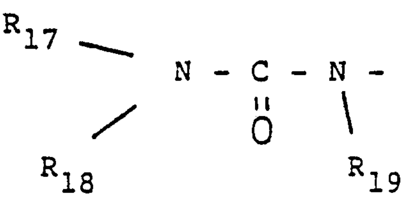

wobei $R_{17}$ Wasserstoff und $R_{18}$ eine Alkyl-, Alkenyl- oder Alkinylgruppe mit 1 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Arylgruppe,
$R_{19}$ Wasserstoff oder eine verzweigte oder unverzweigte Alkylgruppe mit 1 bis 4 Kohlenstoffatomen;

$R_b$ einen Imidorest; einen Benzimidazolylrest, ein Imid;

$R_b$ einen verzweigten oder unverzweigten Alkyloxy bzw. Alkylthiorest mit 1 bis 18, bevorzugt 1 bis 4 Kohlenstoffatomen, Aryloxy, bevorzugt einen gegebenenfalls substituierten Phenyloxy- oder Phenylthiorest, einen über Sauerstoff oder Schwefel verknüpften, gesättigten oder ungestättigten 5- oder 6-gliedrigen heterocyclischen Ring;

$R_3'$ Wasserstoff, $C_1$ bis $C_4$-Alkyl, $C_1$ bis $C_4$-Acyl;

$R_4$ Phenyl, wobei der Phenylring ein oder mehrfach, bevorzugt in 2-Stellung, durch Methyl, bevorzugt Halogen, bevorzugt Brom, besonders bevorzugt Chlor, Nitro und/oder Trifluormethyl substituiert sein kann, oder Pyridyl;

$R_5$ Hydroxy, verzweigtes oder unverzweigtes Alkyl mit 1 bis 6, bevorzugt 1 bis 4 Kohlenstoffatomen, besonders bevorzugt Methyl, gegebenenfalls ist die Alkylgruppe durch Hydroxy, Halogen, bevorzugt Fluor, Alkylsulfonyloxy, bevorzugt Methylsulfonyloxy,

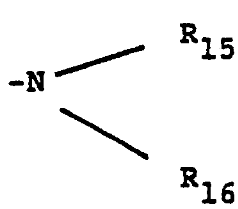

worin $R_{15}$ und $R_{16}$ die zuvor genannte Bedeutung aufweisen können, oder

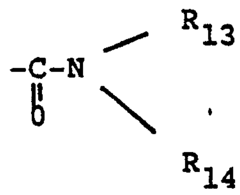

worin $R_{13}$ und $R_{14}$ die zuvor genannte Bedeutng aufweisen können, substituiert;
$R_5$ Alkylcarbonyloxy mit 1 bis 4 Kohlenstoffatomen in den verzweigten oder unverzweigten Alkylkette, Alkoxycarbonyl mit 1 bis 4 Kohlenstoffatomen in der verzweigten oder unverzweigten Alkylkette, Carboxyalkyl mit 1 bis 4 Kohlenstoffatomen in der verzweigten oder unverzweigten Alkylkette, Alkoxycarbonylalkyl mit 1 bis 4 Kohlenstoffatomen in den verzweigten oder unverzweigten Alkylresten,
X/Y unabhängig voneinander C-$R_1$, oder N, aber nicht beide C-$R_1$, mit $R_1$ bevorzugt Wasserstoff oder Methyl,
oder Y die Gruppe C-COOR′, wobei R′ Alkyl oder Wasserstoff bedeutet und X Stickstoff bedeutet;
gegebenenfalls in Form ihrer Racemate, ihrer Enantiomere, ihrer Diastereomere und ihrer Gemische sowie gegebenenfalls ihre physiologisch unbedenklichen Säureadditionssalze.

2. Verbindungen der allgemeinen Formel Ia gemäß Anspruch 1, worin $R_2$ = $R_aZ_n$, $R_3$ = Wasserstoff, Z eine unverzweigte Alkylgruppe,
$R_1$ Ethyl, Methoxy, Ethoxy oder Halogen, besonders bevorzugt Chlor oder Brom; besonders bevorzugt Methyl;
$R_a$ Chlor, Brom, Jod, Hydroxy, Phenyl, p-Isobutylphenyl,

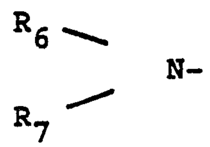

worin $R_6$ und $R_7$, die gleich oder verschieden sein können, Wasserstoff, eine verzweigte oder unverzweigte Alkylgruppe mit 1 bis 6, besonders bevorzugt 1 bis 4, Kohlenstoffatomen, wobei die Kohlenstoffkette durch Stickstoff unterbrochen sein kann, eine verzweigte oder unverzweigte Alkylcarbonylgruppe mit 1 bis 4 Kohlenstoffatomen, gegebenenfalls substituiert durch eine Dimethylaminogruppe, eine Phenylcarbonylgruppe, im Fall von $R_6$ = Wasserstoff eine gegebenenfalls durch Acylamino, besonders bevorzugt Acetylamino, Amino, Alkylamino- oder Dialkylamino substituierte Phenylsulfonylgruppe,
oder
$R_6$ und $R_7$ bilden zusammen mit dem Stickstoffatom einen Piperidin-, Pyrrolidin-, N′-Methylpiperazin-, einen gegebenenfalls dimethylsubstituierten Morpholinring, einen Pyrrol-, Pyrazol-, Imidazol-oder Triazolring,
$R_a$ -CH=O,
$R_a$ ein gegebenenfalls durch Methyl ein- oder mehrfach substituiertes $^2$-Imidazolin, -Oxazolin, -Thiazolin, eine Tolylsulfonyloxygruppe, eine Methylsulfonyloxygruppe,
$R_a$ eine Phenylcarbonyloxygruppe, eine Alkylcarbonyloxygruppe mit 1 bis 5 Kohlenstoffatomen

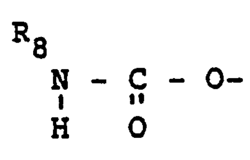 , 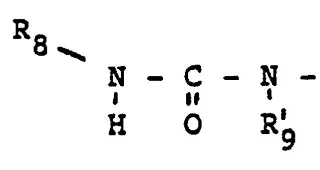

wobei $R_8$ eine verzweigte oder unverzweigte Alkylgruppe mit 1 bis 4 Kohlenstoffatomen, $R_9'$ Wasserstoff oder eine verzweigte oder unverzweigte Alkylgruppe mit 1 bis 4 Kohlenstoffatomen;

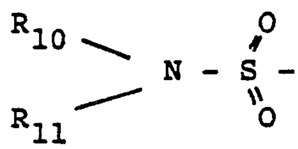

wobei $R_{10}$ und $R_{11}$ die gleich oder verschieden sein können, eine Methyl-, Ethyl-, Propyl- oder Isopropylgruppe oder $R_{10}$ und $R_{11}$ zusammen mit dem Stickstoffatom einen N′-Methylpiperazin- oder Morpholinring;

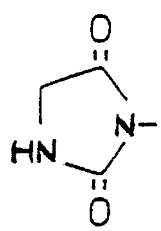

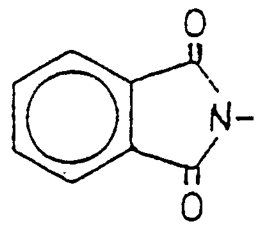

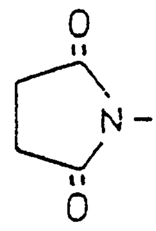

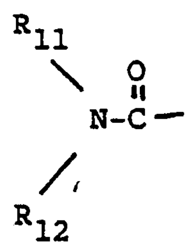

worin $R_{11}$ und $R_{12}$, die gleich oder verschieden sein können, Wasserstoff, eine verzweigte oder unverzweigte Alkylgruppe oder Alkenylgruppe mit 1 bis 6, 8 oder 16 Kohlenstoffatomen, die gegebenenfalls durch Halogen, Hydroxy, Methoxy, Nitro, Amino, Alkylamino oder Dialkylamino mit 1 bis 4 Kohlenstoffatomen in der Alkylkette, oder im Fall von $R_{12}$ = Wasserstoff oder Alkyl durch Morpholinylcarbonyl oder Diethylaminocarbonyl substituiert sein kann,
im Fall von $R_{11}$ = Wasserstoff oder Methyl,
$R_{12}$ ein Thiazolin- oder Thiazolrest, der gegebenenfalls durch eine verzweigte oder unverzweigte Alkylgruppe mit 1 bis 4 C-Atomen substituiert sein kann,
oder
$R_{11}$ und $R_{12}$ zusammen mit dem Stickstoffatomen einen Morpholino- oder Piperazinorest bilden, der gegebenenfalls ein- oder mehrfach durch Methyl substituiert sein kann;
$R_4$ Phenyl, wobei der Phenylring, bevorzugt in 2-Stellung durch Halogen, bevorzugt Chlor, substituiert sein kann;
$R_5$ Hydroxy, Methyl, Hydroxymethyl oder Trifluormethyl
X,Y unabhängig voneinander C-$R_1$ oder N, aber nicht gleichzeitig beide C-$R_1$, ($R_1$ auch Wasserstoff), oder Y die Gruppe C-COOR*, mit R* = Alkyl oder Wasserstoff und X = Stickstoff;
n eine der Zahlen 1, 2, 3, 4, 5, 6, 7, 8, 9 oder 10 bedeuten können sowie gegebenenfalls ihre physiologisch unbedenklichen Säureadditionssalze und ihre optisch aktiven Verbindungen.

3) Verbindungen der allgemeinen Formel Ia, gemäß Anspruch 1 worin $R_2$ und $R_3$ einen Rest der allgemeinen Formel

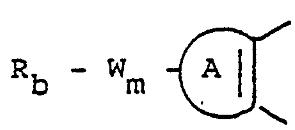

bilden, sind solche worin A ein ankondensierter einfach ungesättigter 5- oder 6-gliedriger Ring bedeutet,

wobei im Fall von m = o und $R_2$ = Wasserstoff in einem 6-gliedrigen Ring ein Kohlenstoffatom durch CO in 2-, 3- oder 4-Stellung des Hetrazepins, ersetzt werden kann, oder A einen ankondensierten Ring der Formel

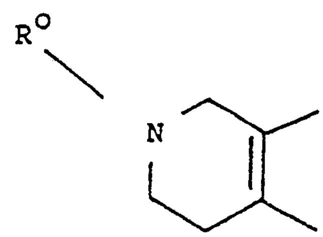

worin $R_0$ Acetylaminoacetyl, Acetyl, Thioacetyl, Ethoxycarbonylmethyl, Methoxycarbonylmethyl, Morpholinylcarbonylmethyl, Diethylaminocarbonylmethyl;

W eine unverzweigte Alkylgruppe mit m Kohlenstoffatomen

m 0, 1, 2, 3 oder 4;

X/Y unabhängig voneinander C-$R_1$ oder N, bevorzugt beide N oder X C-$R_1$ und Y N, aber nicht beide C-$R_1$,

oder

Y C-COOR′, wobei R′ Wasserstoff oder Niederalkyl und X Stickstoff bedeutet;

$R_1$ Wasserstoff, Hydroxymethyl, Chlormethyl, Cyclopropyl, Ethyl, Methoxy, Ethoxy, Chlor oder Brom, bevorzugt Methyl;

$R_b$ Hydroxy, Amino, Carboxy, Cyano, Brom, Phenyl, p-Isobutylphenyl, Alkyloxycarbonyl mit 1 bis 6 Kohlenstoffatomen, bevorzugt 1 bis 2 Kohlenstoffatomen,

einen Rest der allgemeinen Formel

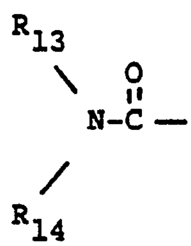

worin $R_{13}$ und $R_{14}$, die gleich oder verschieden sein können, Wasserstoff, eine verzweigte oder unverzweigte Alkylgruppe oder Alkenylgruppe mit 1 bis 6, 8 oder 16 Kohlenstoffatomen, die gegebenenfalls durch Halogen, Hydroxy, Methoxy, Nitro, Amino, Alkylamino oder Dialkylamino mit 1 bis 4 Kohlenstoffatomen in der Alkylkette, oder im Fall von $R_{14}$ = Wasserstoff oder Alkyl durch Morpholinylcarbonyl oder Diethylaminocarbonyl substituiert sein kann,

im Fall von $R_{13}$ = Wasserstoff oder Methyl,

$R_{14}$ ein Thiazolin- oder Thiazolrest, der gegebenenfalls durch eine verzweigte oder unverzweigte Alkylgruppe mit 1 bis 4 C-Atomen substituiert sein kann,

oder

$R_{13}$ und $R_{14}$ zusammen mit dem Stickstoffatomen einen Morpholino- oder Piperazinorest bilden, der gegebenenfalls ein- oder mehrfach durch Methyl substituiert sein kann;

$R_b$ ein C-verknüpfter $\Delta^2$-Imidazolin-, -Thiazolin-, -Oxazolin-, oder Tetrahydropyrimidin-Rest, der gegebenenfalls ein- oder mehrfach durch Alkyl mit 1 bis 4 Kohlenstoffatomen substituiert sein kann;

$R_b$ im Fall von m = O Wasserstoff, wenn A eine Carbonylfunktion oder Stickstoff als Ringglied enthält;

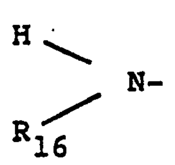

$R_{16}$ eine Alkyloxycarbonylgruppe mit 1 bis 4 Kohlenstoffatomen;
im Fall von m > O
$R_b$ eine Alkylcarbonyloxygruppe mit 1 bis 3 Kohlenstoffatomen;
$R_b$ eine Alkylsulfonyloxygruppe mit 1 bis 2 Kohlenstoffatomen;

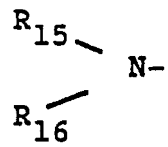

wobei $R_{15}$ und $R_{16}$, die gleich oder verschieden sein können, Wasserstoff, eine verzweigte oder unverzweigte Alkylgruppe mit 1 bis 6 Kohlenstoffatomen, gegebenenfalls durch Dialkylamino mit 1 bis 4 Kohlenstoffatomen, bevorzugt Methyl oder Ethyl, Morpholino oder N-Alkylpiperazino oder ein Indolrest substituiert, eine Alkylcarbonylgruppe mit 1 bis 4 Kohlenstoffatomen,
oder
$R_{15}$ und $R_{16}$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Pyrazolorest, Pyrrolorest, ein Imidorest,
$R_b$ eine verzweigte oder unverzweigte Alkylsulfonyloxygruppe mit 1 bis 4 Kohlenstoffatomen, bevorzugt Methylsulfonyloxy, eine verzweigte oder unverzweigte Alkylcarbonyloxygruppe mit 1 bis 8 Kohlenstoffatomen, bevorzugt 1 bis 4 Kohlenstoffatomen;
$R_b$ Phenyloxy, 3,4-Methylendioxyphenoxy einen Pyridinyloxyrest, einen Alkyloxy- oder Alkylthiorest mit 1 bis 4 Kohlenstoffatomen;
$R_4$ Phenyl oder o-Chlorphenyl;
$R_5$ Hydroxy, Methyl, Hydroxymethyl oder Trifluormethyl bedeuten können, sowie gegebenenfalls ihre physiologisch unbedenklichen Säureadditionssalze und ihre optisch aktiven Verbindungen.

4. Verbindungen der allgemeinen Formel Ia gemäß Anspruch 1, worin A ein ankondensierter, bevorzugt in 3- oder 4-Stellung des Hetrazepins substituierter, einfach ungesättigter 5- oder 6-gliedriger Ring,
W eine unverzweigte Alkylgruppe mit m Kohlenstoffatomen
m 0, 1 oder 2; X/Y beide N, oder X C-H oder $C-CH_3$ und Y N,
$R_1$ Wasserstoff, Cyclopropyl, Methoxy, Brom, bevorzugt Methyl;
$R_b$ Hydroxy, Amino, Carboxy, Cyano, Methoxycarbonyl, Ethoxycarbonyl, p-Isobutylphenyl,
einen Rest der allgemeinen Formel

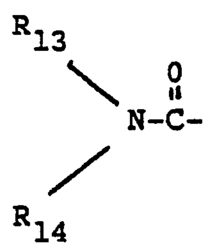

worin $R_{13}$ und $R_{14}$, die gleich oder verschieden sein können, Wasserstoff, Propenyl, Phenyl, eine verzweigte oder unverzweigte Alkylgruppe mit 1 bis 6, 8 oder 16 Kohlenstoffatomen, die gegebenenfalls durch Halogen, Hydroxy, Nitro, Amino, Ethylamino oder Diethylamino, Methoxy, oder im Fall von $R_{14}$ = Wasserstoff oder Alkyl durch Morpholinylcarbonyl oder Diethylaminocarbonyl substituiert sein kann,
im Fall von $R_{13}$ = Wasserstoff oder Methyl,
$R_{14}$ ein Thiazolin- oder Thiazolrest, der gegebenenfalls durch Methyl substituiert sein kann,
oder
$R_{13}$ und $R_{14}$ zusammen mit dem Stickstoffatom einen Morpholino- oder Piperazinorest bilden, der gegebenenfalls ein- oder mehrfach durch Methyl substituiert sein kann;
$R_b$ ein C-verknüpfter $\Delta^2$-Imidazolin-, -Thiazolin-Oxazolin-Rest, der gegebenenfalls ein- oder mehrfach durch Methyl, Ethyl und/oder iso-Propyl substituiert sein kann, ein Tetrahydro-Pyrimidinring, gegebenenfalls ein- oder mehrfach durch Methyl substituiert, ein Benzimidazolrest, ein Indolrest,

$R_2$ im Fall von m = 0 Wasserstoff, wenn A eine Carbonylfunktion oder Stickstoff als Ringglied enthält, oder Methoxycarbonylamino;
im Fall von m > 0 $R_b$ eine Acetoxygruppe, eine Methansulfonyloxygruppe,

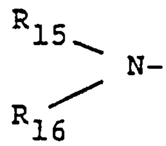

wobei $R_{15}$ und $R_{16}$, die gleich oder verschieden sein können, Wasserstoff, eine verzweigte oder unverzweigte Alkylgruppe mit 1 bis 4 Kohlenstoffatomen, die durch Diethylamino oder Morpholino, substituiert sein kann, eine Acetylgruppe
oder
$R_{15}$ und $R_{16}$ zusammen mit dem Stickstoffatom einen Morpholino- oder Piperazinorest bilden, der gegebenenfalls ein- oder mehrfach durch Methyl substituiert sein kann, ein Triazolorest, ein Imidazolorest, ein Phthalimid,
$R_b$ eine verzweigte oder unverzweigte Alkylsulfonyloxygruppe mit 1 bis 4 Kohlenstoffatomen, bevorzugt Methylsulfonyloxy, eine verzweigte oder unverzweigte Alkylcarbonyloxygruppe mit 1 bis 8 Kohlenstoffatomen, bevorzugt 1 bis 4 Kohlenstoffatomen;
$R_b$ ein Phenyloxyrest, ein Pyridyloxyrest, 3,4-Methylendioxyphenoxy, eine 1,2,4-Triazol-3-yl-thiogruppe, Methoxy;
$R_4$ Phenyl, bevorzugt o-Chlorphenyl;
$R_5$ Hydroxy, Hydroxymethyl oder Methyl bedeuten können, sowie gegebenenfalls ihre physiologisch unbedenklichen Säureadditionssalze, gegebenenfalls ihre optisch aktiven Verbindungen.

5. (-) Enantiomere der allgemeinen Formel Ia gemäß Anspruch 1 oder 2, worin $R_2$ = $R_a$ - $Z_n$ und X und Y beide Stickstoff bedeuten.

6. (-)-Enantiomere der allgemeinen Formel Ia gemäß Anspruch 1, 3 oder 4, worin $R_2$ zusammen mit $R_3$ einen ankondensierten Rest der allgemeinen Formel

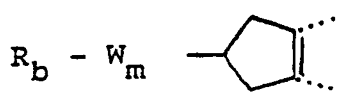

bilden und X und Y beide Stickstoff bedeuten.

7. Pharmazeutische Zubereitung enthaltend eine Verbindung der allgemeinen Formel Ia gemäß einem der Ansprüche 1 bis 6 sowie übliche pharmakologisch unbedenkliche Hilfs- und Trägerstoffe.

8. Verwendung einer Verbindung der allgemeinen Formel I gemäß einem der Ansprüche 1 bis 6 als Arzneimittel mit PAF-antagonistischer Wirkung

9. Verfahren zur Herstellung von Verbindungen der allgemeinen Formel Ia, dadurch gekennzeichnet, daß man ausgehend von einer Verbindung der allgemeinen Formel Ia worin $R_5$ = Wasserstoff bedeutet

a) zur Herstellung von Verbindungen der allgemeinen Formel Ia mit $R_5$ = Alkylcarbonyloxy, das entsprechende N-Oxid der allgemeinen Formel Ia im Sinne einer Polonowskyreaktion mit dem entsprechenden Säureanhydrid umsetzt,

b) zur Herstellung von Verbindungen der allgemeinen Formel Ia mit $R_5$ = Hydroxy, eine Verbindung der allgemeinen Formel Ia mit $R_5$ = Alkylcarbonyloxy verseift,

c) zur Herstellung von Verbindungen der allgemeinen Formel Ia mit $R_5$ = Alkyloxycarbonylalkyl eine Verbindung der allgemeinen Formel I mir $R_5$ = Wasserstoff deprotoniert und anschließend mit einem Alkenylcarbonsäurealkylester mit $\gamma$-ständiger Doppelbindung umsetzt, gegebenenfalls anschließend den erhaltenen Ester nach an sich bekannten Methoden über die Carbonsäure in das Amid überführt, oder den Ester zum Alkohol reduziert, oder den Alkohol in das entsprechende Amin umwandelt;

d) zur Herstellung von Verbindungen der allgemeinen Formel Ia mit $R_5$ = Alkoxycarbonyl eine Verbindung der allgemeinen Formel Ia mit $R_5$ = Wasserstoff in Gegenwart einer Base mit Dialkylcarbonaten umsetzt.

10. Verfahren zur Herstellung von Verbindungen der allgemeinen Formel I gemäß Anspruch 1, worin $R_1$, $R_2$, $R_3$, $R_4$ wie zuvor definiert, mit der Maßgabe, daß in $R_2$ Ra und Rb Wasserstoff, COOR′ (R′ = Alkyl

oder Wasserstoff), Alkylcarbonyloxy, einen Ether- oder Thioetherrest, ein Säureamid oder ein Amin und $R_5$ einen Alkyl oder substituierten Alkylrest bedeuten, dadurch gekennzeichnet, daß man eine entsprechende Verbindung der allgemeinen Formel

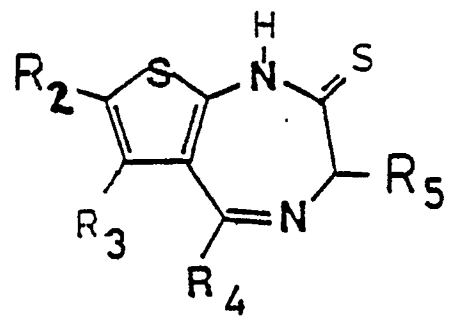

A) für den Fall, daß X und Y Stickstoff bedeutet

a) mit einem Säureanhydrazid der allgemeinen Formel

$R_1$-CONHNH$_2$

umsetzt,

b) oder aber mit Hydrazin in eine Verbindung der allgemeinen Formel

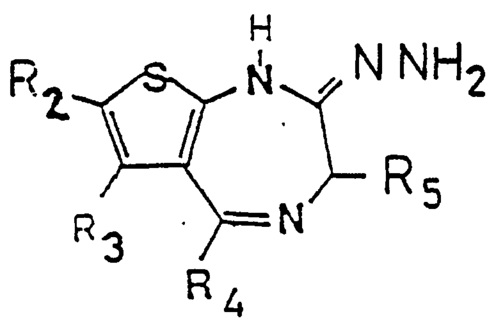

überführt und anschließend mit einem Säurehalogenid der allgemeinen Formel

$R_1$-CO-Hal

oder mit einem Orthoester der allgemeinen Formel

$R_1$ - C (OR)$_3$

worin R′ eine niedere Alkylgruppe bedeutet, umsetzt, oder

B) für den Fall daß X C-H, C-Alkyl und Y Stickstoff

a) mit einem Aminoalkin der allgemeinen Formel

$R_{11}$ - C≡C - $CH_2$-$NH_2$

worin $R_{11}$ Wasserstoff oder eine niedere Alkylgruppe bedeutet

oder aber

b) mit einem α-Aminoaldehyd-alkylacetal oder α-Aminoketon-alkylketal der allgemeinen Formel

$H_2NCH_2$-$CR_1(OR')_2$

worin $R_1$ Wasserstoff oder eine Alkylgruppe mit 1 bis 4 Kohlenstoffatomen und R′ eine niedere Alkylgruppe bedeutet, umsetzt

C) für den Fall, daß X Stickstoff und Y C-H man eine Verbindung der allgemeinen Formel

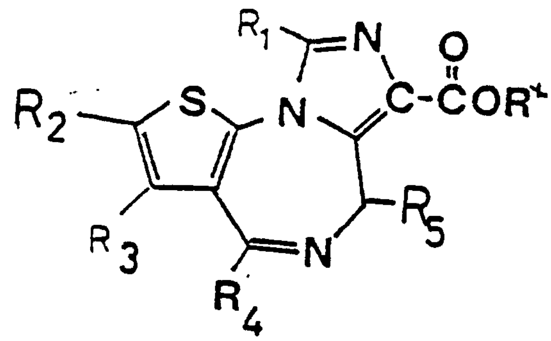

R′ = niederes Alkyl decarboxyliert.

11. Verfahren zur Herstellung von Verbindungen der allgemeinen Formel I gemäß Anspruch 1, dadurch gekennzeichnet, daß man Verbindungen der allgemeinen Formel Ia, worin $R_1$ Wasserstoff, Alkyl, Cycloalkyl, $R_2$, $R_3$, $R_4$, $R_5$, X, Y wie zuvor definiert mit der Maßgabe, daß in $R_2$ $R_a$ oder $R_b$ die Bedeutung eines Alkylcarbonsäureesters aufweist wie folgt umsetzt

1) Verbindungen der allgemeinen Formel Ia mit
$R_a$, $R_b$ = COOH
erhält man durch Verseifen von Verbindungen der Formel Ia

2) Verbindungen der allgemeinen Formel Ia mit
$R_a$ = $R_{11}R_{12}NCO$,
$R_b$ = $R_{13}R_{14}NCO$
erhält man durch Umsetzung von Ia mit
$R_a$, $R_b$ = COOH
mit einem Amin
gegebenenfalls in Gegenwart von Sulfonyldimidazol, Carbonyldiimidazol oder Dicyclohexylcarbodiimid;

3) Verbindungen der allgemeinen Formel Ia mit
$R_a$, $R_b$ = OH
erhält man durch selektive Reduktion von Ia, z.B. mit Lithiumalanat oder Natriumborhydrid, bzw. für m = O, n = O durch Reduktion der entsprechenden Carbonylverbindung;

4) Verbindungen der allgemeinen Formel Ia mit
$R_a$ = $R_8$NH-COO- $R_8$-CO-$NR_9'$,
$R_b$ = $R_{17}R_{18}NCOO$, $R_{17}R_{18}CONR_{19}$
erhält man durch Reaktion des Alkohols oder Amins, z.B. hergestellt nach 3) mit einem Isocyanat der allgemeinen Formel
$R_8N=C=O$; $R_{18}$ $N=C=O$

5) Verbindungen der allgemeinen Formel Ia mit $R_a$, $R_b$ = Alkyl- bzw. Arylcarbonyloxy erhält man durch Reaktion des Alkohols, z. B. hergestellt nach 3) mit einem Säureäquivalent, z.B. einem Säurehalogenid, einer Carbonsäure der Formel
R-COOH,
worin R ein Arylrest oder ein verzweigter oder ein unverzweigter Alkylrest mit 1 bis 8 Kohlenstoffatomen, wobei die Kohlenstofkette durch Stickstoff, Sauerstoff oder Schwefel unterbrochen sein kann; bedeutet;

6) Verbindungen der allgemeinen Formel Ia mit $R_a$, $R_b$ = -Alkylsulfonyloxy oder Arylsulfonyloxy erhält man durch Reaktion des Alkohols, z.B. hergestellt nach 3), mit einem Alkyl- oder Arylsulfonylhalogenid unter Zusatz eines säurebindenden Mittels;

7) Verbindungen der allgemeinen Formel Ia mit
$R_a$ = $NR_6R_7$; $R_b$ = $NR_{15}R_{16}$
oder ein Imidorest
erhält man durch Reaktion des Mesylats mit einem Amin der Formel
$HNR_6R_7$; $HNR_{15}R_{16}$
oder einem Imid, gegebenenfalls in Form ihrer Alkalimetallsalze;

8) Verbindungen der allgemeinen Formel Ia mit
$R_a$, $R_b$ = $NH_2$
erhält man durch Spaltung des Phthalimids oder durch Curtius Abbau des entsprechenden Säureazids, bzw. durch Hydrolyse der entsprechenden Isocyanate, oder durch Reduktion des entsprechenden Azids;

9) Verbindungen der allgemeinen Formel Ia mit

$R_a$ = $NR_6R_7$ $R_b$ = $NH_{15}R_{16}$
wobei einer der Reste ein Alkyl- oder Arylcarbonyl bedeutet, erhält man durch Reaktion des Amins, z.B hergestellt nach 8), mit einem Carbonsäurequivalent abgeleitet von einer Carbonsäure der Formel
$R_{11}COOH$, $R_{13}$ COOH
10) Verbindungen der allgemeinen Formel Ia mit $R_a$, $R_b$ = Formyl
erhält man durch Oxidation des Alkohols unter Verkürzung der Kette von n auf n-1;
11) Verbindungen der allgemeinen Formel Ia worin
$R_a$, $R_b$ ein Rest der allgemeinen Formel

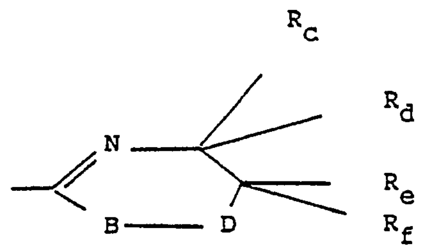

bedeutet,
erhält man durch Reaktion einer Verbindung der allgemeinen Formel Ia mit
$R_a$, $R_b$ = COOH
mit einem bisfunktionalisiertem Amin der allgemeinen Formel

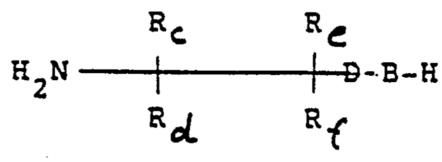

worin $R_c$, $R_d$, $R_e$, $R_f$, D und B die zuvor genannte Bedeutung aufweist,
in Gegenwart von Triphenylphosphin, $CCl_4$ und einer Base;
11a) Verbindungen der allgemeinen Formel Ia, worin $R_2$ ein gegebenenfalls substituierter Thiazolinrest ist, erhält man aus den entsprechenden Oxazolinen, (z.B. hergestellt nach 11) durch Schwefelung mit Phosphorpentasulfid oder Lawesson-Reagenz.
12) Verbindungen der allgemeinen Formel IA, worin
$R_a$, $R_b$ ein Amidin der allgemeinen Formel

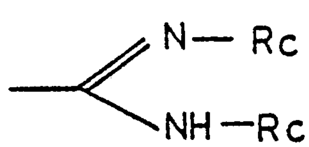

bedeutet,
erhält man analog 11) durch Umsetzung mit einem primären Amin der Formel
$H_2NR_c$
13) Verbindungen der allgemeinen Formel Iamit
$R_a$, $R_b$ = CN
erhält man durch Reaktion von Verbindungen der allgemeinen Formel Ia, worin
$R_a$, $R_b$ = $CONH_2$
bedeutet, mit Phosphoroxychlorid;
14) Verbindungen der allgemeinen Formel Ia worin $R_a$ ode $R_b$ ein gegebenenfalls durch verzweigte oder unverzweigte Alkylgruppe substituiertes 2-Imidazolin ist,
erhält man durch Reaktion von Verbindungen der allgemeinen Formel Ia mit

$R_a$, $R_b$ = CN

a) mit ethanolischer HCl,

b) Umsetzen des entstandenen Imidoethylesters mit einem gegebenenfalls durch verzweigte oder unverzweigte Alkylgruppen substituiertes Ethylendiamin,

c) gegebenenfalls erfolgt die Alkylierung der freien N-H Funktion mit bekannten Alkylierungsmitteln;

15) Verbindungen der allgemeinen Formel Ia, worin $R_a$, $R_b$ = Alkyl- oder Aryloxycarbonyl bedeutet, erhält man durch Umsetzung von einem Säurequivalent von I ($R_a$, $R_b$ = COOH) mit den entsprechenden Alkoholen;

16) Verbindungen der allgemeinen Formel Ia mit $R_a$, $R_b$ = Halogen, erhält man durch Reaktion des Tosylats, z.B. hergestellt nach 6), mit einem Halogenierungsmittel;

17) Verbindungen der allgemeinen Formel Ia, worin $R_a$ oder $R_b$ einen Ether, bzw. Thioether, bedeutet, erhält man aus den entsprechenden Mesylaten, z.B. hergestellt nach 6) ($R_a$, $R_b$ = $CH_3SO_3$), durch Umsetzung mit den entsprechenden Alkoholen oder Mercaptanen als Solvens oder ihrer Alkalimetallslaze in einem inerten organischen Lösungsmittel;

18) Verbindungen der allgemeinen Formel Ia mit A gleich

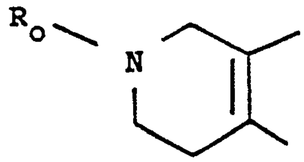

und $R^{\circ}$ gleich Alkyl, substituiertes Alkyl, Alkylcarbonyl, substituiertes Aalkylcarbonyl oder Arylcarbonyl erhält man durch Alkylierung bzw. Acylierung von Verbindungen der allgemeinen Formel IA mit $R^{\circ}$ = Wasserstoff,

die entsprechenden Thiocarbonylverbindungen durch Umsetzung der Carbonylverbindung mit einem Schwefelreagenz, z.B. Phosphorpentasulfid;

anschließend gewünschtenfalls die erhaltenen Verbindungen Ia

mit $R_1$ = Wasserstoff

in Gegenwart einer Base mit Chlor oder Brom zu einer Verbindung der allgemeinen Formel Ia mit $R_1$ = Chlor oder Brom umsetzt;

anschließend gewünschtenfalls die Halogenverbindung in eine Verbindung der allgemeinen Formel Ia mit $R_1$ = Alkoxy mit 1 bis 4 Kohlenstoffatomen durch Reaktion mit dem entsprechenden Alkoholat überführt;

anschließend gewünschtenfalls eine Verbindung der allgemeinen Formel Ia selektiv reduziert und gewünschtenfalls die erhaltene Verbindung Ib worin $R_3'$ Wasserstoff bedeutet, alkyliert oder acyliert und gegebenenfals in ihre pharmakologisch unbedenklichen Säureadditionssalze überführt und anschließend gegebenenfalls in ihre optisch aktiven Verbindungen trennt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 89120420.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE - A1 - 3 724 031 (BOEHRINGER INGELHEIM KG) * Ansprüche 1,2,5,8-11 * -- | 1,7-11 | C 07 D 495/14<br>A 61 K 31/55<br>//(C 07 D 495/14<br>C 07 D 233:00<br>C 07 D 333:00<br>C 07 D 243:00) |
| A | DE - A1 - 3 701 344 (BOEHRINGER INGELHEIM KG) * Ansprüche 1,4,5; Seite 24, Zeilen 12-17 * -- | 1,7-11 | |
| A | CHEMICAL ABSTRACTS, Band 106, Nr. 24, 15. Juni 1987, Columbus, Ohio, USA YOSHITOMI PHARMACEUTICAL INDUSTRIES "Thienodiazepine derivatives as blood platelet-activating factor entagonists" Seite 355, Spalte 1, Zusammenfassung-Nr. 201 745c & Jpn. Kokai Tokkyo Koho JP 62 22 718 (87 22 718) ---- | 1,7-11 | |

RECHERCHIERTE SACHGEBIETE (Int. Cl.5)

C 07 D 495/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7,9-11

Unvollständig recherchierte Patentansprüche: -

Nicht recherchierte Patentansprüche: 8 -

Grund für die Beschränkung der Recherche: Verfahren zur therapeutischen Behandlung des menschlichen oder tierischen Körpers; Art. 52(4) EPÜ

| Recherchenort | Abschlußdatum der Recherche | Prüfer |
|---|---|---|
| WIEN | 19-12-1989 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82